(19) Europäisches Patentamt
European Patent Office
Office européen des brevets

(11) Numéro de publication: **0 188 396 B1**

# (12) FASCICULE DE BREVET EUROPEEN

(45) Date de publication du fascicule du brevet: **30.08.89**

(51) Int. Cl.⁴: **C07J 1/00**, C07J 43/00, C07J 31/00, C07J 41/00, A61K 31/565, A61K 31/58

(21) Numéro de dépôt: **86400057.5**

(22) Date de dépôt: **13.01.86**

(54) **Nouveaux stéroides substitués en position 10, leur procédé et les intermédiaires de préparation, leur application comme médicaments, les compositions pharmaceutiques les contenant.**

(30) Priorité: **14.01.85 FR 8500434**

(43) Date de publication de la demande: **23.07.86 Bulletin 86/30**

(45) Mention de la délivrance du brevet: **30.08.89 Bulletin 89/35**

(84) Etats contractants désignés: **AT BE CH DE FR GB IT LI LU NL SE**

(56) Documents cités:
**WO-A-83/03099**
**FR-A-1 380 411**

**STEROIDS, vol. 39, no. 3, mars 1982, pages 325-344, Holden Day, San Francisco, US; P.A. MARCOTTE et al.: "Synthesis and evaluation of 10beta-substituted 4-estrene-3,17-diones as inhibitors of human placental microsomal aromatase"**

(73) Titulaire: **ROUSSEL-UCLAF, 35, boulevard des Invalides, F-75007 Paris(FR)**

(72) Inventeur: **Teutsch, Jean-Georges, Résidence Lavoisier - Bât. 3 3, rue Lavoisier, F-93500 Pantin(FR)**
Inventeur: **Costerousse, Germain, 10, rue des Réservoirs, F-94410 Saint-Maurice(FR)**
Inventeur: **Torelli, Vesperto, 5, rue de la Convention, F-94700 Maisons-Alfort(FR)**
Inventeur: **Philibert, Daniel, 16, rue Chevalier, F-94210 La Varenne-Saint-Hilaire(FR)**

(74) Mandataire: **Bourgouin, André et al, Département des Brevets ROUSSEL UCLAF B.P. no 9, F-93230 Romainville(FR)**

**EP 0 188 396 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne de nouveaux stéroïdes substitués en position 10, leur procédé et les intermédiaires de préparation, leur application comme médicaments et les compositions pharamaceutiques les renfermant.

Dans le brevet français FR 1 380 411 sont décrits des produits comportant un radical alkyle en position 10β. Cependant ce brevet ne décrit aucun produit tombant dans la formule générale de la présente demande. De plus le procédé décrit dans le brevet cité est très différent du procédé de la présente demande.

Dans la publication Stéroïde vol 39, nº 3, 1982 p. 325–344 sont décrits des produits pouvant comporter, en position 10β, un radical propargyle ou alkyle. Cependant le procédé d'accès aux produits décrits dans cette publication est également très différent du procédé de la présente demande et aucun produit de cette publication ne tombe dans la formule de la présente demande.

La demande internationale WO 8 303 099 décrit des produits portant un substituant en position 11 et aucun substituant en position 10β. Les produits de la demande citée sont donc très différents des produits de la présente demande.

L'invention a pour objet les produits de formule générale I:

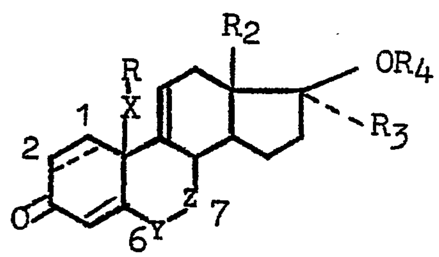

(I)

dans laquelle X et R sont tels que:

– soit X représente un radical méthylène et R représente

ou bien un radical aryle carbocyclique ou hétérocyclique choisi parmi les radicaux phényle ou naphtyle, furyle, thiényle, pyrolyle, oxazolyle, thiazolyle, imidazolyle, pyrazolyle, thiadiazolyle, triazolyle, oxadiazolyle ou tétrazolyle, pyridinyle, pyrazinyle ou pyrimidinyle, chacun de ces radicaux étant éventuellement substitué par un ou plusieurs substituants choisis parmi les radicaux alkyle ayant de 1 à 4 atomes de carbone, alkoxy ayant de 1 à 4 atomes de carbone, alkylthio ayant de 1 à 4 atomes de carbone, alkényle choisis parmi vinyle ou allyle, alkynyle choisis parmi éthynyle, propargyle ou prop-1-ynyle, halogènes, trifluorométhyle, amino, méthylamino ou diaméthylamino, amino protégé choisi parmi les radicaux tritylamino ou bis triméthylsilylamino, hydroxyle, mercapto, carboxyle, méthoxycarbonyle, éthoxycarbonyle ou tert-butoxy carbonyle, carboxyle salifié, carbamoyle, nitro, aminoalkyle, diméthylaminoéthyle, carboxyméthyle ou hydroxyméthyle ou bien un radical vinyle ou éthynyle éventuellement substitué, par un ou plusieurs substituants choisis parmi les radicaux alkyle ayant de 1 à 4 atomes de carbone, alkoxy ayant de 1 à 4 atomes de carbone, alkylthio ayant de 1 à 4 atomes de carbone, alkényle choisis parmi vinyle ou allyle, alkynyle choisis parmi éthynyle, propargyle ou prop-1-ynyle, halogènes, trifluorométhyle, amino, méthylamino ou diméthylamino, amino protégé choisi parmi les radicaux tritylamino ou bis triméthylsilylamino, hydroxyle, mercapto, carboxyle, méthoxycarbonyle, éthoxycarbonyle ou tert-butoxy carbonyle, carboxyle salifié; carbamoyle, nitro, aminoalkyle, diméthylaminoéthyle, carboxyméthyle ou hydroxyméthyle et les radicaux aryles carbocycliques ou hétérocycliques choisis parmi les radicaux phényle ou naphtyle, furyle, thiényle, pyrolyle, oxazolyle, thiazolyle, imidazolyle, pyrazolyle, thiadiazolyle, triazolyle, oxadiazolyle ou tétrazolyle, pyridinyle, pyrazinyle ou pyrimidinyle

– soit X représente un atome de soufre ou une simple liaison et R représente un radical aryle carbocyclique ou hétérocyclique choisi parmi les radicaux phényle ou naphtyle, furyle, thiényle, pyrolyle, oxazolyle, thiazolyle, imidazolyle, pyrazolyle, thiadiazolyle, triazolyle, oxadiazolyle ou tétrazolyle, pyridinyle, pyrazinyle ou pyrimidinyle, chacun de ces radicaux étant éventuellement substitué par un ou plusieurs substituants choisis parmi les radicaux alkyle ayant de 1 à 4 atomes de carbone, alkoxy ayant de 1 à 4 atomes de carbone, alkylthio ayant de 1 à 4 atomes de carbone, alkényle choisi parmi vinyle ou allyle alkynyle choisis parmi éthynyle, propargyle ou prop-1-ynyle, halogènes, trifluorométhyle, amino, méthylamino ou diméthylamino, amino protégé choisi parmi les radicaux tritylamino ou bis triméthylsilylamino, hydroxyle, mercapto, carboxyle, méthoxycarbonyle, éthoxycarbonyle ou tert-butoxy carbonyle, carboxyle salifié; carbamoyle, nitro, aminoalkyle, monoalkylaminoalkyle, diméthylaminoéthyle, carboxyméthyle ou hydroxyméthyle

$R_2$ représente un radical méthyle ou éthyle,

$R_3$ représente un radical alkyle, alkényle ou alkynyle ayant au plus 8 atomes de carbone ou un radical hydroxypropynyle ou chloroéthynyle,

$R_4$ représente un atome d'hydrogène ou un radical acyle, choisi parmi les radicaux acétyle, propionyle ou benzoyle

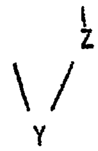

ou bien un groupement

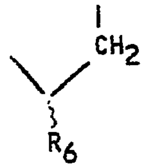

dans lequel $R_6$ représente un atome d'hydrogène ou un radical méthyle en position α et/ou β, ou bien un groupement

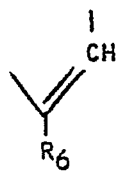

dans lequel $R_6$ représente un atome d'hydrogène ou un radical méthyle, ou bien un groupement

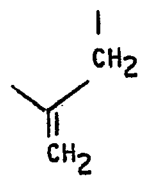

Le trait pointillé en position 1(2) indique la présence éventuelle d'une seconde liaison carbone-carbone.

Parmi les produits de formule générale (I), l'invention a notamment pour objet les produits répondant à la formule générale ($I_1$):

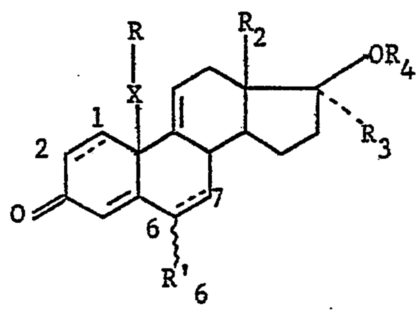

($I_1$)

dans laquelle X, R, $R_2$, $R_3$ et $R_4$ sont définis comme précédemment, $R'_6$ représente un atome d'hydrogène ou un radical méthyle, les traits pointillés en position 1(2) et 6(7) indiquent la présence éventuelle d'une seconde liaison entre les carbones qui les portent, le trait ondulé en position 6 indique que le substituant $R'_6$ lorsqu'il s'agit d'un radical méthyle, peut se trouver dans l'une des positions possibles α ou β lorsque le trait pointillé en position 6(7) n'indique pas la présence d'une seconde liaison.

Parmi les valeurs de R, on peut citer les radicaux aryles carbocycliques, tels que phényle ou naphtyle.

Parmi les radicaux aryles hétérocycliques, on peut citer les radicaux à 5 ou 6 chaînons comportant de 1 à 4 hétéro-atomes choisis parmi les atomes d'azote d'oxygène ou de soufre.

Parmi les radicaux à 5 chaînons, on peut citer les radicaux furyle, thiényle, pyrolyle, oxazolyle, thiazolyle, imidazolyle, pyrazolyle, thiadiazolyle, triazolyle, oxadiazolyle ou tétrazolyle.

Parmi les radicaux à 6 chaînons, on peut citer les radicaux pyridyle, pyrazinyle ou pyrimidinyle.

Les radicaux aryles carbocycliques ou hétérocycliques ainsi que les radicaux vinyle ou éthynyle que peut représenter R peuvent être substitués par un ou plusieurs substituants qui peuvent être choisis dans la liste suivante:

- les radicaux alkyles ayant de 1 à 4 atomes de carbone, tels que méthyle, éthyle, propyle isopropyle, butyle, tert-butyle,
- les radicaux alkoxy ayant de 1 à 4 atomes de carbone, tels que méthoxy, éthoxy, propyloxy, butoxy ou tert-butoxy,
- les radicaux alkylthio ayant de 1 à 4 atomes de carbone, tels que méthylthio, éthylthio, propylthio ou butylthio,
- les radicaux vinyle ou allyle, les radicaux
- éthynyle, propargyle ou prop-1-ynyl,
- les atomes d'halogènes tels que fluor, chlore, brome ouiode,
- le radical trifluorométhyle,
- les radicaux amino, amino protégé, méthylamino ou diméthylamino,
- les radicaux hydroxyles éventuellement protégés, mercapto, carboxyle, carboxyle estérifié ou salifié, carbamoyle ou nitro,
- les radicaux diméthylamino éthyle,
- carboxyméthyle,
- hydroxyméthyle.

Les radicaux vinyle ou éthynyle que peut représenter R peuvent eux-mêmes être substitués par un radical aryle carbocyclique ou hétérocyclique choisi dans la liste précédente.

Les valeurs alkyle, alkényle ou alkynyle que peut représenter $R_3$ sont choisies de préférence dans les listes données ci-dessus. $R_3$ peut particulièrement représenter un radical alkynyle, tel que propynyle.

Le radical acyle que peut représenter $R_4$ est de préférence un reste d'un acide carboxylique, tel que acétyle, propionyle, benzoyle.

L'invention a plus particulièrement pour objet, les produits de formule générale I telle que définie ci-dessus caractérisée en ce que le radical aryle carbocyclique ou hétérocyclique éventuellement substitué que peut représenter R signifie <u>ou bien</u> un radical phényle éventuellement substitué par un radical alkyle ou alkoxy ayant de 1 à 4 atomes de carbone, par un atome d'halogène ou par un radical amino ou dialkylamino, <u>ou bien</u> un radical pyridyle.

La présente invention a également plus particulièrement pour objet, les produits de formule générale I telle que définie ci-dessus caractérisée en ce que le radical vinyle ou éthynyle éventuellement substitué que peut représenter R signifie <u>ou bien</u> un radical vinyle ou méthylvinyle de formule

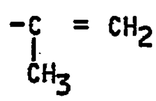

<u>ou bien</u> un radical éthynyle éventuellement substitué par un radical alkyle ayant de 1 à 4 atomes de carbone, par un radical phényle, par un radical carboxy éventuellement estérifié, par un radical hydroxyméthyle ou par un radical amino tritylamino, bis triméthylsilylamino méthylamino ou diméthylamino.

Le radical amino protégé peut être choisi parmi les radicaux tritylamino, bis triméthylsilylamino. Le radical carboxy estérifié peut être un radical méthoxycarbonyle, éthoxycarbonyle ou tert-butoxy carbonyle. Le radical amino monoalkylé ou dialkylé est de préférence un méthylamino ou diméthylamino.

L'invention a plus particulièrement pour objet les produits décrits ci-après dans les exemples et notamment:

– la 10β-benzyl 17β-hydroxy 17α-(prop-1-ynyl) estra 4,9(11-dièn-3-one;
– la 17β-hydroxy 10β-/(4-méthylphényl) méthyl/17α-(prop-1-ynyl) estra 4,9(11)-dièn 3-one;
– la 10β-(2-méthylprop-2-én-1-yl) 17α-(prop-1-ynyl) 17β-hydroxy estra-4,9(11)-dièn 3-one;
– la 10β-/(4-méthoxyphényl) thio/ 17α-(prop-1-ynyl) 17β-hydroxy estra 4,9(11)-dièn 3-one;
– la 10β-/(2-fluorophényl) méthyl/17α-(prop-1-ynyl) 17β-hydroxy estra 4,9(11)-dièn 3-one.

L'invention a également pour objet un procédé de préparation des produits de formule I telle que définie ci-dessus caractérisé en ce que l'on traite un produit de formule II:

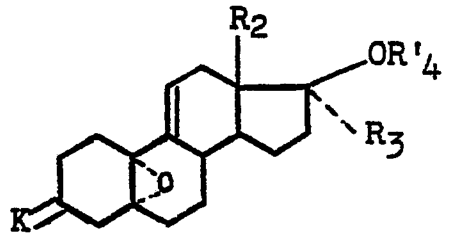

(II)

dans laquelle $R_2$ et $R_3$ ont la signification indiquée ci-dessus, K représente un groupement protecteur de la fonction cétone et $R'_4$ représente un atome d'hydrogène ou un groupement protecteur du radical hydroxyle,

- soit par un lithien de formule R-X-Li,
- soit par un magnésien de formule R-X'-Mg-Hal, formules dans lesquelles R et X ont les significations indiquées ci-dessus, X' représente un radical méthylène ou une simple liaison et Hal représente un atome d'halogène, pour obtenir un produit de formule III :

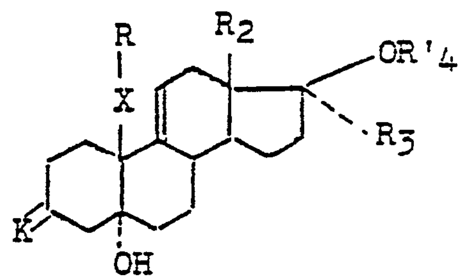

(III)

que l'on traite par un acide pour obtenir les produits de formule IV

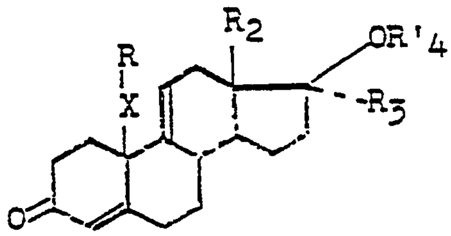

(IV)

que, si nécessaire, l'on traite par un réactif de clivage du groupement $R'_4$ lorsque celui-ci représente un groupement protecteur du radical hydroxyle différent d'un radical acyle, pour obtenir un produit de formule $I_A$:

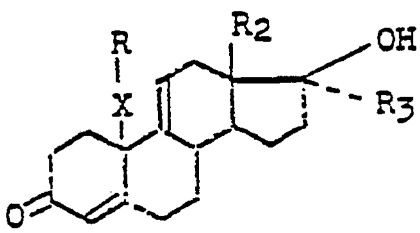

($I_A$)

et que si désiré l'on traite un produit de formule IV par un orthoformiate d'alkyle, puis par un réactif de déshydrogénation et enfin, si nécessaire, par un réactif de clivage du groupement $R'_4$ lorsque celui-ci représente un groupement protecteur du radical hydroxyle différent d'un radical acyle, pour obtenir un produit de formule $I_B$:

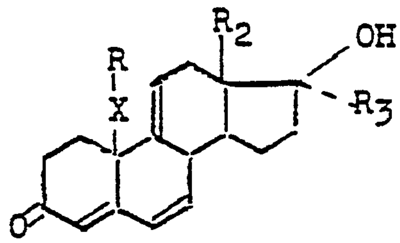

$(I_B)$

et que si désiré l'on traite un produit de formule IV par un réactif susceptible d'introduire un radical méthylène, en position 6, pour obtenir un produit de formule V:

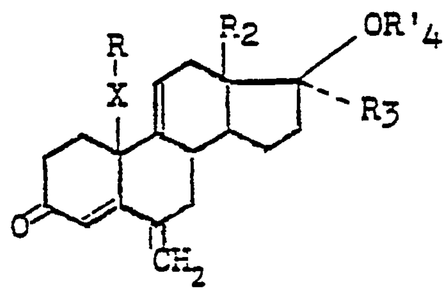

(V)

que, si nécessaire, l'on traite par un réactif de clivage du groupement R'4 lorsque celui-ci représente un groupement protecteur du radical hydroxyle différent d'un radical acyle, pour obtenir un produit de formule ($I_C$):

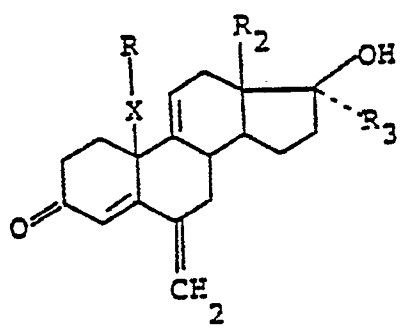

$(I_C)$

ou produit de formule (V) que:

- soit l'on traite par un réactif d'hydrogénation et éventuellement d'épimérisation, puis par un réactif de clivage du groupement R'4 lorsque celui-ci représente un groupement protecteur du radical hydroxyle différent d'un radical acyle, pour obtenir un produit de formule ($I_D$) :

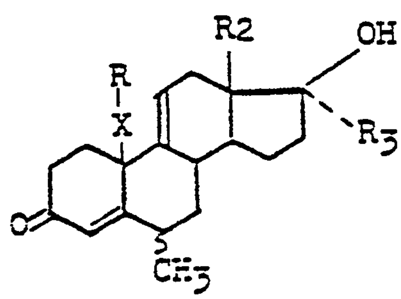

$(I_D)$

- soit l'on traite par un agent d'isomérisation pour obtenir après traitement par un réactif de clivage du groupement R'4, lorsque celui-ci représente un groupement protecteur du radical hydroxyle un produit de formule $I_E$:

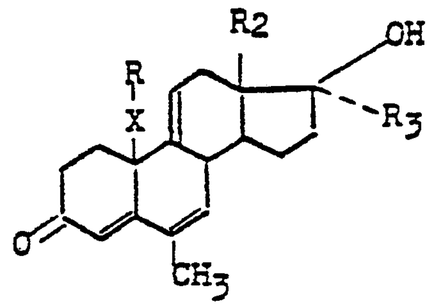

($I_E$)

et que si désiré l'on traite les produits de formules ($I_A$), ($I_B$), ($I_C$), ($I_D$) et ($I_E$ précédentes par un réactif de déshydrogénation ou par un microorganisme susceptible de déshydrogéner, pour obtenir les produits correspondants comportant une insaturation en position 1(2) et produits de formules ($I_A$), ($I_B$), ($I_C$), ($I_D$) et ($I_E$) et les produits corres-pondants comportant une insaturation en position 1(2) que si désiré l'on traite par un réactif d'acylation.

Le groupement protecteur de la fonction cétone que représente K peut être un cétal, un thiocétal, une oxime ou une alkoxime. On utilise de préférence un cétal tel que le bis méthoxy ou un cétal cyclique tel que l'éthylènedioxy. Les groupements sont éliminés en milieu acide. Le lithien de formule R-X-Li est préparé de préférence in situ, par action du butyl lithium sur le produit de formule R-XH. La réaction est effectuée de préférence à basse température dans un solvant ou un mélange de solvants organiques anhydres tels que le tétrahydrofuranne ou l'éther éthylique.

L'action du magnésien est effectuée dans les conditions usuelles. Le magnésien peut être préparé in situ par action de l'halogénure correspondant sur du magnésium métal.

On peut opérer avec le chlorure ou le bromure de l'organomagnésien, la réaction est effectuée dans un solvant organique anhydre tel que le tétrahydrofuranne ou l'éther éthylique.

L'acide que l'on utilise de préférence pour déshydrater la molécule est l'acide chlorhydrique aqueux. On peut opérer dans un solvant miscible à l'eau tel que l'éthanol.

Le groupement protecteur du radical hydroxyle, en position 17, que peut représenter R'$_4$, peut être choisi dans la liste des groupements usuels. On peut citer, par exemple, les groupements tétrahydropyrannyle ou tert butyle ou un radical acyle tel qu'acétyle chloroacétyle ou trifluoracétyle. L'élimination des groupements non acylés peut être effectuée selon les méthodes usuelles. On peut opérer en milieu acide de préférence. Le traitement acide requis pour transformer les produits de formule III en produits de formule IV peut d'ailleurs conduire à une élimination simultanée du radical R'$_4$.

L'orthoformiate d'alkyle, que l'on utilise pour transformer les produits de formule IV en produits de formule $I_B$, est de préférence l'orthoformiate d'éthyle. On opère alors en présence d'un acide tel que l'acide paratoluène sulfonique. On obtient intermédiairement les produits de formule:

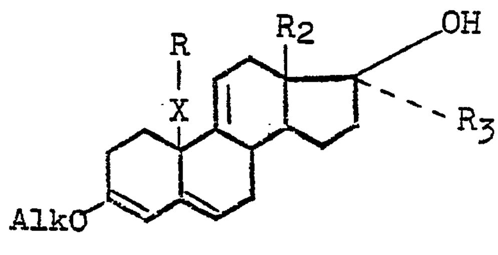

C'est sur ces produits intermédiaires que l'on fait agir un réactif de déshydrogénation qui peut être le chloranile ou la D.D.Q (2,3-dichloro 5,6-dicyano benzoquinone).

La préparation du produit de formule (V) ou ($I_C$), à partir du produit de formule IV, peut être effectuée selon les modes opératoires décrits dans les publications Liebigs Ann Chem 1983 p. 712-713 ou Synthesis 1982 p. 34-40.

Dans un mode préférentiel d'exécution du procédé, on traite le produit de formule IV par du chlorure de phosphoryle, en présence de diméthylacétal ou de diétylacétal de formaldéhyde et d'acétate de sodium. On opère dans un solvant tel que le chloroforme.

L'hydrogénation et l'épimérisation éventuelle du produit de formule (V) en produit de formule ($I_D$) est réalisée dans les conditions usuelles. On peut, par exemple, opérer avec de l'hydrogène gazeux en présence d'un catalyseur.

L'isomérisation des produits de formule (V) en produits de formule ($I_E$) peut être réalisée selon la méthode décrite dans Tétrahedron 20, 597 (1964) ou 21, 1619 (1965).

Cette méthode utilise le palladium sur charbon en présence de cyclohexène.

On peut également indiquer que le passage des produits de formule IV aux produits de formule ($I_E$) peut être effectué directement sans isolement intermédiaire du produit de formule V par action, sur le produit IV de chlorure de phosphoryle et d'acétate de méthoxyméthyle en présence d'acétate de sodium dans le chloroforme (Ann. Chem. 1983 p.712).

Le réactif de déshydrogénation que l'on fait agir sur les produits de formules ($I_A$), ($I_B$), ($I_C$), ($I_D$) et ($I_E$) est de préférence l'anhydride séléninique. On peut cependant également utiliser un dérivé de la benzoquinone tel que le chloranile ou la DDQ. On peut, enfin, utiliser un microorganisme tel que la bactérie "Arthrobacter simplex". On opère alors en milieu aqueux tamponné. L'acylation éventuelle des produits finals est effectué selon les méthodes usuelles. On peut aussi utiliser un anhydride ou un halogénure d'acyle.

La présente invention a également pour objet un procédé de préparation des produits de formule générale I telle que définie ci-dessus caractérisé en ce que l'on traite un produit de formule (VI) :

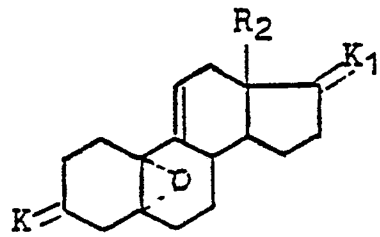

(VI)

dans laquelle $R_2$ a la signification indiquée ci-dessus, K représente un groupement protecteur de la fonction cétone et $K_1$ représente une fonction cétone éventuellement protégée :

- soit par un lithien de formule R-X-Li,
- soit par un magnésien de formule R-X'-Mg-Hal, formules dans lesquelles R et X ont les significations indiquées ci-dessus, X' représente un radical méthylène ou une simple liaison et Hal représente un atome d'halogène, pour obtenir un produit de formule (VII) :

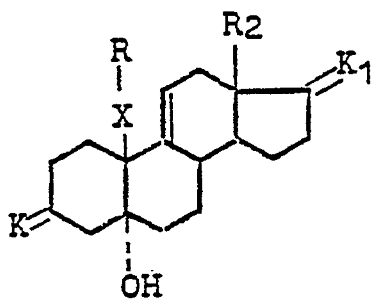

(VII)

produit que l'on traite :
- soit par un réactif de clivage du radical $K_1$ lorsque celui-ci représente un groupement protecteur du radical ceto en position 17, puis par un magnésien de formule $R_3$-Mg-$Hal_1$ ou par un lithien de formule $R_3$-Li, formules dans lesquelles $R_3$ a la signification indiquée ci-dessus et $Hal_1$ représente un atome d'halogène, pour obtenir un produit de formule ($III_A$) :

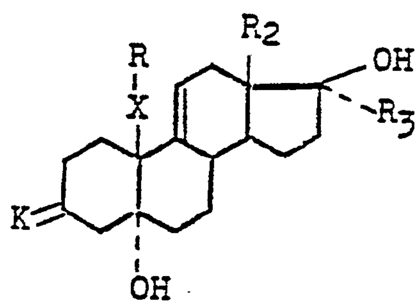

($III_A$)

que l'on traite par un acide pour obtenir un produit de formule ($I_A$) telle que définie ci-dessus, produit que l'on soumet éventuellement à une réaction de protection du radical hydroxyle en position 17β et traite le produit de formules ($I_A$) ou (IV) ainsi obtenus, selon le procédé décrit ci-dessus, pour obtenir les produits de formules ($I_B$), ($I_C$), ($I_D$) et ($I_E$) et les produits insaturés en 1(2) correspondants ;

- soit l'on traite le produit de formule (VII) par un acide pour obtenir un produit de formule (VIII) :

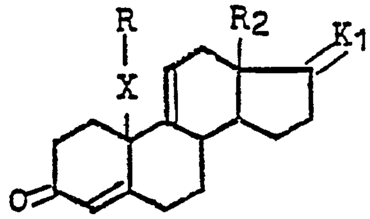

(VIII)

produit de formule (VIII) que l'on traite,
ou bien par un orthoformiate d'alkyle, puis par un réactif de déshydrogénation, pour obtenir un produit de formule (IX) :

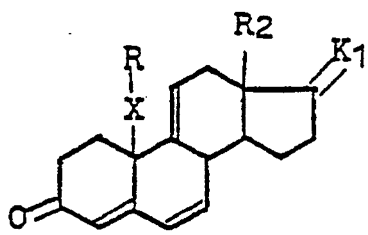

(IX)

ou bienpar un réactif susceptible d'introduire un radical méthylène, en position 6, pour obtenir un produit de formule (X):

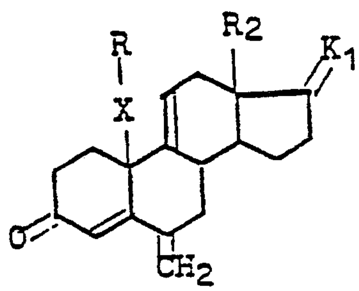

(X)

et produit de formule (X) que l'on traite ou par un réactif d'hydrogénation et éventuellement d'épimérisation, pour obtenir un produit de formule (XI) :

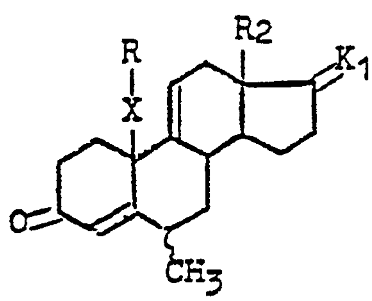

(XI)

ou par un agent d'isomérisation, pour obtenir un produit de formule (XII):

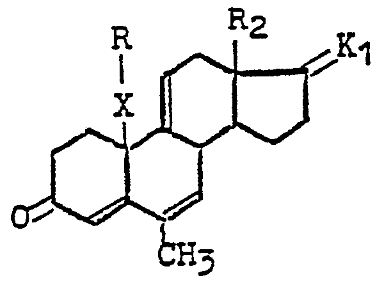

(XII)

et produits de formules (VIII), (IX), (X), (XI) et (XII) que l'on traite, éventuellement, par un réactif de clivage du groupement $K_1$ lorsque celui-ci représente un groupement protecteur de la fonction cétone et par un agent de blocage de la fonction cétone en position 3, puis par un magnésien de formule $R_3$-Mg-$Hal_1$ dans laquelle $R_3$ a la signification indiquée ci-dessus et $Hal_1$ représente un atome d'halogène pour obtenir respectivement, après déblocage de la fonction cétone en position 3, les produits de formules ($I_A$), ($I_B$), ($I_C$), ($I_D$) et ($I_E$) tels que définis ci-dessus, que l'on peut transformer selon le procédé décrit ci-dessus en produits insaturés en position 1(2) correspondants et que l'on peut acyler.

Parmi les valeurs de $K_1$, on peut citer les valeurs citées précédemment pour le radical K. On peut également utiliser de préférence des produits dans lesquels $K_1$ représente le radical

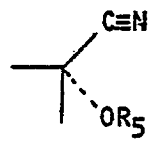

dans lequel $R_5$ représente un atome d'hydrogène, un radical triméthylsilyle ou un radical acyle.

L'action du lithien de formule R-X-Li ou du magnésien de formule R-X'-Mg-Hal sur le produit de formule VI est effectuée dans les mêmes conditions que celles décrites ci-dessus pour l'action sur le produit de formule (II).

Il en est de même des autres réactions effectuées sur le produit de formules (VII) et (VIII). La réaction de protection éventuelle, en position 17, du produit de formule ($I_A$) est effectuée dans les conditions usuelles. On peut, par exemple, utiliser un halogénure de tertbutyle, un anhydride d'acyle ou le dihydrofuranne.

La réaction de clivage éventuel du groupement $K_1$, qui peut protéger la fonction cétone en position 17, est effectuée dans les conditions utuselles, on opère ainsi de préférence par hydrolyse acide.

La réaction éventuelle de blocage de la fonction cétone, en position 3, est également effectuée dans les conditions usuelles. On utilise de préférence un des radicaux cétals décrits précédemment.

Les produits de formule (I) sont des produits particulièrement intéressants du point de vue pharmacologique; ils possèdent en particulier une remarquable activité antiglucocortiocoïde comme le montrent les résultats des tests exposés ci-après.

Les produits de formule (I) peuvent donc être utilisés comme médicaments pour lutter principalement contre les effets secondaires des glucocorticoïdes; ils permettent de lutter également contre les troubles dus à une hypersécrétion de glucocorticoïdes et notamment contre le vieillisement en général et plus particulièrement contre l'hypertension, le glaucome, l'athérosclérose, l'ostéoporose, le diabète, l'obésité ainsi que l'immunodépression et l'insomnie.

Certains produits de formule I peuvent également présenter des propriétés glucocorticoïdes et peuvent ainsi être employées dans le traitement des réactions inflammatoires; ils peuvent être intéressants pour le traitement des réactions inflammatoires locales comme par exemple, les oedèmes, les dermatoses, les prurits, les diverses formes d'eczéma et les érythèmes solaires ou dans le traitement des manifestations inflammatoires d'origine rhumatismale ou arthritique

L'invention a donc pour objet, à titre de médicament, les produits de formule I pharmaceutiquement acceptables.

L'invention a plus spécialement pour objet, à titre de médicaments:

A) les produits de formule générale I telle que définie ci-dessus, caractérisée en ce que le radical aryle carbocyclique ou hétéroocyclique éventuellement substitué que peut représenter R signifie <u>ou bien</u> un radical phényle éventuellement substitué par un radical alkyle ou alkoxy ayant de 1 à 4 atomes de carbone, par un atome d'halogène ou par un radical amino ou dialkylamino, <u>ou bien</u> un radical pyridyle;

B) les produits de formule générale I telle que définie ci-dessus, caractérisée en ce que le radical vinyle ou éthynyle éventuellement substitué que peut représenter R signifie <u>ou bien</u> un radical vinyle ou méthylvinyle de formule

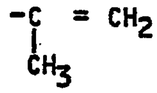

ou bien un radical éthynyle éventuellement substitué par un radical alkyle ayant de 1 à 4 atomes de carbone, par un radical phényle, par un radical carboxy éventuellement estérifié, par un radical hydroxyméthyle ou par un radical amino éventuellement protégé ou alkylé.

L'invention a particulièrement pour objet, à titre de médicament, les produits décrits ci-après dans les exemples et parmi ceux-ci les produits suivants:

- la 10β-benzyl 17β-hydroxy 17α-(prop-1-ynyl) estra 4,9(11)-dièn-3-one;
- la 17β-hydroxy 10β-/(4-méthylphényl) méthyl/17α-(prop-1-ynyl) estra 4,9(11)-dièn 3-one;
- la 10β-(2-méthylprop-2-én-1-yl) 17α-(prop-1-ynyl) 17β-hydroxy estra 4,9(11)-dièn 3-one;
- la 10β-/(4-méthoxyphényl) thio/ 17α-(prop-1-ynyl) 17β-hydroxy estra 4,9(11)-dièn 3-one;
- la 10β-/(2-fluorophényl) méthyl/ 17α-(prop-1-ynyl) 17β-hydroxy estra 4,9(11)-dièn-3-one;

La posologie utile varie en fonction de l'affection à traiter et de la voie d'administration; elle peut varier par exemple de 10 mg à 1 g par jour chez l'adulte par voie orale.

Les nouveaux produits de formule I, tels que définis ci-dessus peuvent être employés pour préparer des compositions pharmaceutiques renfermant, à titre de principe actif, l'un au moins desdits produits.

Les produits de formule I sont utilisés par voie digestive, parentérale ou locale. Ils peuvent être prescrits sous forme de comprimés simples ou dragéifiés, de gélules, de granulés, de suppositoires, de préparations injectables, de pommades, de crèmes, de gels, de collyres, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les cors gras d'origine animale ou végétale, les dérivés paraffiniques, les glycols, les divers agents mouillants, dispersants ou émulsifiants, les conservateurs.

L'invention a donc pour objet les compositions pharmaceutiques renfermant comme principe actif au moins un produit de formule (I).

L'invention a également pour objet, à titre de produits industriels nouveaux, les produits de formule générale (A):

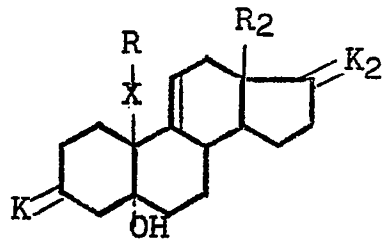

dans laquelle X, R et $R_2$ ont la signification indiquée ci-dessus, K représente un groupement protecteur de la fonction cétone choisi parmi les groupements cétal, thiocétal, oxime ou alkyloxime et $K_2$ représente:

- soit une fonction cétone éventuellement protégée, par un groupement cétal, thiocétal, oxime ou alkyloxime,
- soit un radical

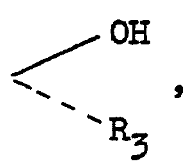

dans lequel $R_3$ a la signification précédente et dans lequel l'hydroxyle est éventuellement protégé par un groupement choisi parmi les groupements tétrahydropyrannyle, tert-butyle, acétyle, chloroacétyle ou trifluoroacétyle.

Les produits de départ de formules II ou VI sont connus ou peuvent être préparés selon le procédé décrit dans les brevets français 2 423 486, 2 522 328 et européen 0 057 115.

Les exemples suivants illustrent l'invention sans toutefois la limiter.

Exemple 1: 10β-benzyl 17β-hydroxy 17α-(prop-1-ynyl) estra 4,9(11) dièn-3-one.

Stade A: 10β-benzyl 3,3-éthylène bis oxy 17α-(prop-1-ynyl) estra 9(11) en 5α, 17β-diol.

a. Préparation du chlorure de benzylmagnésium.

On introduit en 25 minutes, en maintenant la température entre 30 et 35°C, une solution de 42,6 g d'α-chlorotoluène dans 220 $cm^3$ de tétrahydrofuranne, dans un mélange de 8,2 g de magnésium, dans 39 $cm^3$ de tétrahydrofuranne. On amorce le magnésien, après introduction des premiers centimètres cubes, par ajout de 2 $cm^3$ d'une solution éthérée du magnésien de 1,2-dibromoéthane. On laisse ensuite revenir à 20°C sous agitation et azote, et obtient une solution titrant 0,5 M/l.

b. On introduit, goutte à goutte, en maintenant la température à + 5°/+ 8°C une solution de 3,7 g de 3,3-éthylène bisoxy 5α, 10α-époxy 17-(prop-1-ynyl) estra 9(11) èn 17β-ol dans 20 $cm^3$ de tétrahydrofuranne, dans une solution de 60 $cm^3$ de chlorure de benzyl magnésium dans le tétrahydrofuranne préparée comme indiqué ci-dessus en a et refroidie préalablement à 0°C. On agite le mélange réactionnel pendant 30 minutes à 0°C, puis laisse remonter la température à 20°C et maintient 3 heures sous agitation. On verse alors dans un mélange de 370 $cm^3$ d'eau et de glace et 37 g de chlorure d'ammonium. On agite 15 minutes, puis décante la phase aqueuse et l'extrait à l'éther éthylique. On lave la phase organique à l'eau, la sèche, puis la concentre à sec sous pression réduite. On obtient 8,1 g de produit brut que l'on chromatographie sur silice ou éluant par un mélange chlorure de méthylène-acétone 9–1 avec 1‰ de triéthylamine. On obtient 2,435 g de produit attendu, puis 1,032 g de l'isomère 10α-benzyl.

Un échantillon analytique de l'isomère 10β recherché est obtenu par dissolution de 440 mg au reflux dans 7 $cm^3$ d'éther isopropylique et 2 $cm^3$ de chlorure de méthylène. On filtre la solution à chaud et concentre à petit volume. On garde 3 heures au repos à 20°C, puis essore et lave avec deux fois 1 cm3 d'éther isopropylique. On sèche le produit et obtient ainsi 342 mg de produit pur. F = 200°C.
$/\alpha/_D$ = -4,45° ± 2° (c= 0,5% dans le chloroforme).

| | | |
|---|---|---|
| Analyse: | $C_{30}H_{38}O_4$ = 462,635 | |
| Calculé: | C % : 77,89 | H % : 8,28 |
| Trouvé: | 77,9 | 8,3 |

Stade B : 10β-benzyl-17β-hydroxy 17α-(prop-1-ynyl) estra 4,9(11) dièn-3-one.

On chauffe au reflux pendant 2 heures sous azote un mélange de 2,07 g de produit obtenu au stade A, 6,2 cm3 d'éthanol à 95° et 2,07 g de résine sulfonique.

On filtre la résine et l'empâte par de l'éthanol à 95°.

La solution est concentrée à sec sous pression réduite. Le produit obtenu (1,97 g) est chromatographié sur silice (éluant : chlorure de méthylène-acétone 9-1). On recueille 1,24 g de 10β-benzyl 5α, 17β-dihydroxy 17α-(prop-1-ynyl) estra 9(11) èn 3-one.

On porte à 40°C une solution de 1,08 g de ce produit dans 10,8 cm3 d'éthanol, puis ajoute en une seule fois 5,4 cm3 d'acide chlorhydrique 2 N. On chauffe à 60°C sous agitation et azote pendant 6 heures 30mn. On refroidit à 20°C et ajoute 15 cm3 de solution aqueuse saturée de carbonate acide de sodium.

On ajoute ensuite 15 cm3 de chlorure de méthylène, décante la phase aqueuse et l'extrait du chlorure de méthylène. On lave la phase organique à l'eau, sèche, concentre à sec sous pression réduite, et obtient 1,04 g de produit que l'on chromatographie sur silice (éluant chlorure de méthylène-acétone 95-5). On recueille 932 mg de produit attendu. Ce produit est recristallisé par dissolution au reflux dans un mélange de 7 cm3 d'éther isopropylique et 8 cm3 de chlorure de méthylène. On filtre à chaud, concentre à volume réduit. On garde 3 heures au repos à 20°C, essore et lave avec avec 0,7 cm3 d'éther isopropylique. On obtient 902 mg de produit attendu. F = 217°C.
$/\alpha/_D$ = +16,5° ± 2°C (c = 0,7 % dans le chloroforme).

Spectre UV (éthanol)

| | | |
|---|---|---|
| infl. 215 nm | $E^1_1$ = 365 | |
| max. 244 nm | $E^1_1$ = 332 | ε = 13300 |
| infl. 311 nm | $E^1_1$ = 2 | |

Exemple 2 : 17β-hydroxy 10β-/(2-méthylphényl) méthyl/17α-(prop-1-ynyl) estra 4,9(11) dièn-3-one.

Stade A : 3,3-bis méthoxy 10β-/(2-méthylphényl) méthyl/ 17α-(prop-1-ynyl) estra 9(11) èn 5α, 17β-diol.

a/. Magnésien de l'α-chloro orthoxylène.

On ajoute sous azote et agitation 20 cm3 d'éther éthylique et 0,2 cm3 d'α-chloro orthoxylène sur 5,5 g de magnésium en tournure, on ajoute ensuite, goutte à goutte, en une heure, une solution de 32 cm3 d'α-chloroorthoxylène dans 250 cm3 d'éther, de manière à maintenir la température à 35°C. On agite 1 heure et obtient une solution titrant 0,5 M/l.

b/. On ajoute en 20 minutes une solution dans 30 cm3 de tétrahydrofuranne de 10 g de produit comprenant 50% de 3,3 bis méthoxy 5α, 10α-époxy 17α-(prop-1-ynyl) estra 9(11) en 17β-ol, 30% de 3,3 bis-méthoxy 5β, 10β-époxy 17α-(prop-1-ynyl) estra 9(11) èn 17β-ol et 20 % de 3,3-bis méthoxy 17α-(prop-1-ynyl) estra 5(10) 9(11) dièn 17β-ol dans 215 cm3 de magnésium préparé comme précédemment, en maintenant la température à 20°C.

On laisse 2 heures sous agitation, puis verse sous agitation dans une solution de 100 g de chlorure d'ammonium dans 1 l d'eau. On agite vivement pendant 15 minutes, décante, extrait la phase aqueuse à l'éther. On lave les phases organiques à l'eau, sèche, filtre et concentre à sec sous pression réduite. On obtient 24 g de produit que l'on purifie sur silice (éluant : chlorure de méthylène, acétone 95-5, tréthylamine 1‰).

On obtient 2,3 g de produit attendu Rf = 0,5.

| | | |
|---|---|---|
| Analyse: | $C_{31}H_{42}O_4$ = 478,68 | |
| Calculé: | C % : 77,78 | H % : 8,84 |
| Trouvé: | 77,7 | 9,1 |

Stade B : 17β-hydroxy 10β-/(2/méthylphényl) méthyl/17α-(prop-1-ynyl) estra 4,9(11) dièn-3-one.

On chauffe à 40°C une solution de 2,5 g de produit obtenu au stade A dans 25 cm3 d'éthanol à 96° et ajoute en une fois 9,6 cm3 d'acide chlorhydrique 2 N. On chauffe au reflux pendant 45 minutes. Le produit attendu cristallise. On refroidit à température ambiante, filtre le produit, le lave à l'éther isopropylique et obtient 1,6 g de produit attendu que l'on dissout à chaud dans un mélange de 20 cm3 d'éther isopropylique et 15 cm3 de chlorure de méthylène. On filtre les impuretés et laisse cristalliser à température ambiante.

On filtre, lave, sèche et obtient 1,4 g de produit attendu.

F = 220°C, Rf = 0,5 (chlorure de méthylène : 95, acétone : 5).

| | | |
|---|---|---|
| Analyse: | $C_{29}H_{34}O_2$ = 414,56 | |
| Calculé: | C % : 84,02 | H % : 8,26 |
| Trouvé: | 84,2 | 8,4 |

Exemple 3 : 10β-/(2-chlorophényl) méthyl/ 17β-hydroxy 17α-(prop-1-ynyl) estra-4,9(11)-dièn-3-one.

Stade A : 3,3 bis méthoxy 10β-/(2-chlorophényl)méthyl/ 17α-(prop-1-ynyl) estra 9(11) èn 5α, 17β-diol.

a/. Magnésien du chlorure d'orthochloro benzyle.

On amorce la réaction en ajoutant 0,2 cm3 de 1,2-dibromoéthane à un mélange de 2,05 g de magnésium en tournure, 7 cm3 d'éther et 0,2 cm3 de chlorure d'orthochlorobenzyle. On ajoute alors, goutte à goutte, en 45 minutes et sans dépasser 33°-35°C, une solution de 10,5 g de chlorure d'orthochlorobenzyle dans 61 cm3 d'éther. On obtient une solution titrant 0,9 M/l.

b/. On refroidit à 0°C, 54 cm3 de magnésien préparé comme précédemment et ajoute en 25 minutes une solution de 6,05 g de 3,3-bis méthoxy 5α, 10α-époxy 17α-(prop-1-ynyl) estra 9(11) èn 17β-ol contenant de l'époxyde 5β, 10β, dans 33 cm3 de tétrahydrofuranne. On laisse une heure à 0°/-5°C, puis amène à température ambiante.

On verse sur 400 cm3 d'eau glacée contenant 40 g de chlorure d'ammonium, agite pendant 15 minutes, décante, extrait à l'éther, lave à l'eau, sèche, essore, puis amène à sec. On obtient 12 g de produit que l'on purifie par passage sur silice, en éluant avec un mélange de chlorure de méthylène acétone (95:5). On obtient 3,4 g de produit attendu Rf = 0,55.

Stade B : 10β-/(2-chlorophényl) méthyl) 17β-hydroxy 17α-(prop-1-ynyl) estra 4,9(11) dièn-3-one.

On chauffe à 40°C une solution de 2,7 g de produit obtenu au stade A dans 27 cm3 d'éthanol à 96% et ajoute en une seule fois 10,8 cm3 d'acide chlorhydrique 2 N. On chauffe au reflux pendant 45 minutes, puis refroidit à température ambiante. On filtre le produit cristallisé, le lave avec un mélange comprenant 30 cm3 d'alcool et 10 cm3 d'eau. On sèche et obtient 1,8 g de produit attendu. Ce produit est dissous à chaud dans un mélange de 40 cm3 d'éther isopropylique et 20 cm3 de chlorure de méthylène. On essore à chaud, puis laisse cristalliser à température ambiante pendant 3 heures. On filtre, lave à l'éther isopropylique, puis sèche. On obtient 1,65 g de produit attendu. F = 201°C.
/α/D = +36° ±2°(c =0,6 % dans le chloroforme).

**Spectre UV (éthanol)**

| | | |
|---|---|---|
| infl. 218 nm | $E^1_1$ = 402 | ε = 18800 |
| max. 243 nm | $E^1_1$ = 302 | ε = 14100 |
| infl. 275 nm | $E^1_1$ = 40 | |

Exemple 4 : 17β-hydroxy 10β-/(4-méthylphényl) méthyl/ 17α-(prop-1-ynyl) estra 4,9(11) dièn-3-one.

Stade A : 3,3-bis méthoxy 10β-/(4-méthylphényl) méthyl/ 17α-(prop-1-ynyl) estra 9(11) èn 5α,17β-diol.

a) magnésien du chlorure de paraméthyl benzyle.

On amorce la réaction en ajoutant 0,2 cm3 de 1,2-dibromoéthane dans un mélange de 4,1 g de magnésium en tournures, 14 cm3 d'éther et 0,2 cm3 de chlorure de paraméthyl benzyle. La température monte jusqu'à 30-35°C et on ajoute alors, goutte à goutte, en une heure, 23 cm3 de chlorure de paraméthyl benzyle dans 180 cm3 d'éther. On laisse ensuite 1 heure sous agitation à température ambiante et obtient une solution titrant 0,5 M/l.

b) On ajoute alors, goutte à goutte, en 20-25 minutes, une solution de 10 g de 3,3 bis méthoxy 5α, 10α-époxy 17α-(prop-1-ynyl) estra 9(11) èn 17β-ol contenant de l'époxyde 5β, 10β, dans 50 cm3 de tétrahydrofuranne dans 215 cm3 de magnésien préparé comme ci-dessus.

On laisse une heure et demie sous agitation à température ambiante, puis verse dans un litre d'eau contenant 100 g de chlorure d'ammonium.

On agite pendant 15 minutes, décante la phase organique, puis extrait à l'éther.

On lave à l'eau les phases organiques, sèche, filtre, puis porte à sec sous pression réduite.

On obtient 25 g de produit brut que l'on chromatographie sur silice, en éluant avec un mélange chlorure de méthylène-acétone (95-5) avec 1‰ de triéthylamine. On obtient 5,4 g de produit attendu Rf = 0,4. F = 170°C

| | | |
|---|---|---|
| Analyse: | $C_{31}H_{42}O_4$ = 478,68 | |
| Calculé: | C % : 77,78 | H % : 8,84 |
| Trouvé: | 77,9 | 8,9 |

Stade B : 17β-hydroxy 10β-/(4-méthylphényl) méthyl/ 17α-(prop-1-ynyl) estra 4,9(11) dièn-3-one.

On chauffe à 40°C une solution de 4,6 g de produit obtenu au stade A dans 46 cm3 d'éthanol à 96%, puis ajoute en une seule fois 19 cm3 d'acide chlorhydrique 2 N. On chauffe au reflux pendant 45 minutes, puis refroidit à température ambiante. On filtre le produit cristallisé, le lave avec le minimum d'un mélange éthanol-eau (45:20). On sèche sous pression réduite et obtient 3 g de produit attendu que l'on purifie par recristallisation à chaud dans 100 cm3 d'un mélange d'éther isopropylique et de chlorure de méthylène (1-1). On filtre les impuretés, concentre aux trois quarts, puis laisse cristalliser à température ambiante pendant 3 heures. On obtient finalement 2,6 g de produit attendu. F = 220°C. Rf = 0,5 (chlorure de méthylène-acétone (95:5) triéthylamine 1‰.
/α/D = +18° ± 1° = (c = 1% chloroforme).

**Spectre UV (éthanol)**

| | | |
|---|---|---|
| max. 222 nm | $E^1_1$ = 425 | ε = 17600 |
| max. 240 nm | $E^1_1$ = 302 | ε = 12500 |

Exemple 5 : 10β-(2-propényl) 17α-(prop-1-ynyl) 17β-hydroxy estra 4,9(11)-dièn-3-one.

Stade A : 3,3-bis diméthoxy 10β-(2 propényl) 17α-(prop-1-ynyl) estra-9(11) èn 5α,17β-diol.

a/. Magnésium du chlorure d'allyle.

Dans un mélange de 8,51 g de magnésium en tournure et de 85 cm3 d'éther éthylique, on ajoute quelques centimètres cubes d'une solution de 31,4 cm3 de chlorure d'allyle dans 170 cm3 d'éther éthylique. Lorsque la réaction est amorcée, on introduit le reste de la solution en 45 minutes à +5°C, agite ensuite pendant 1 heure et 30 minutes à 20°C. On obtient une solution de magnésien titrant 0,9 M/l.

b/. 3,3-bis méthoxy 10β-(2 propényl) 17α-(prop-1-ynyl) estra-9(11)-èn 5α, 17β-diol.
A 118 cm3 de la solution de magnésien préparée comme au stade a/, on ajoute goutte à goutte, sous agitation, en 30 minutes, une solution de 10 g de produit comprenant deux tiers de 3,3-bis méthoxy 5α, 10α-époxy 17α-(prop-1-ynyl) estra-9(11)-èn 17β-ol et un tiers de l'époxyde 5β, 10β, agite pendant 1 heure à 20°C, abandonne pendant 16 heures à 20°C, verse le mélange réactionnel dans une solution aqueuse de chlorure d'ammonium, extrait à l'éther éthylique, lave la phase organique par une solution aqueuse saturée de chlorure de sodium, sèche, concentre à sec, sous pression réduite, purifie le résidu par chromatographie sur silice, en éluant par un mélange de chlorure de méthylène et d'acétone (95-5) à 1‰ de triéthylamine et obtient 5,5 g de produit attendu Rf = 0,45.

Stade B : 10β-(2-propényl) 17α-(prop-1-ynyl) 17β-hydroxy estra-4,9(11)-dièn 3-one.

A une solution de 5,5 g de 3,3-bis méthoxy 10 -(2 propényl) 17α-(prop-1-ynyl) estra-9(11)-èn 5α, 17β-diol préparé au stade A, dans 55 cm3 d'éthanol, on ajoute 26,4 cm3 d'acide chlorhydrique 2 N, agite pendant 45 minutes sous atosmosphère d'azote, refroidit à +5°C, amène à pH 7 par addition d'une solution aqueuse 2 N de soude, extrait au chlorure de méthylène, lave la phase chlorométhylénique à l'eau, sèche, concentre à sec sous pression réduite, purifie le résidu par chromatographie sur silice, en éluant par un mélange de chlorure de méthylène et d'acétone (95/5), obtient 3 g de produit brut que l'on purifie par empâtage, puis cristallisation dans l'éther isopropylique et obtient 2,756 g de produit attendu.
F = 138°C, /α/D = +20,5° ±1,5° (c = 0,8%, éthanol).

Spectre UV (éthanol)
max. 242–243 nm $E^1_1 = 429$ ε = 15000

Exemple 6 : 10β-/(4-fluorophényl) méthyl/ 17α-(prop-1-ynyl) 17 -hydroxy estra-4,9(11)-dièn-3-one.

Stade A : 3,3-bis méthoxy 10β-/4-fluorophényl méthyl/ 17α-(prop-1-ynyl) estra-9(11) en 5α,17β-diol.

a/. Magnésien de 4-fluoro chlorotoluène.

A 8,51 g de magnésium en tournure dans 85 cm3 d'éther éthylique, on ajoute, goutte à goutte, en 3 heures et 15 minutes, sans dépasser 33°C, une solution de 23,8 cm3 de 4-fluoro chlorotoluène dans 115 cm3 d'éther éthylique, agite encore pendant 2 heures, en laissant revenir à 20°C et obtient une solution de magnésien titrant 1,1 M/l.

b/. 3,3-bis méthoxy 10β-/(4-fluorophényl) méthyl/ 17α-(prop-1-ynyl) estra-9(11) èn 5α, 17β-diol.
Dans 98 cm3 de la solution de magnésien préparée comme au stade a/, on ajoute à +13°C, en 15 minutes, une solution de 10 g de produit comprenant deux tiers de 3,3-bis méthoxy 5α, 10α-époxy 17α-(prop-1-ynyl) estra-9(11) èn 17β-ol et d'un tiers de l'époxyde 5β, 10β dans 50 cm3 de tétrahydrofuranne, agite pendant 15 heures à 20°C, verse le mélange réactionnel dansune solution aqueuse de chlorure d'ammonium, extrait à l'éther éthylique, concentre à sec par distillation sous pression réduite, chromatographie le résidu sur silice en éluant par un mélange de chlorure deméthylène, d'acétone et de triéthylamine (95/5/0,1) et obtient 5,9 g de produit attendu, Rf = 0,4.

Stade B : 10β-/(4-fluorophényl) méthyl/ 17α-(prop-1-ynyl) 17β-hydroxy estra-4,9(11)-dièn-3-one.

A une solution de 5,9 g de produit obtenu au stade A dans 59 cm3 d'éthanol, on ajoute 24,4 cm3 de solution aqueuse 2 N d'acide chlorhydrique, agite au reflux pendant 1 heure et 15 minutes, refroidit à +5°C, amène à pH 7 par une solution aqueuse 2 N de soude, extrait au chlorure de méthylène, lave à l'eau, sèche, concentre à sec sous pression réduite, chromatographie le résidu sur silice, en éluant par un mélange de chlorure de méthylène et d'acétone (95/5), cristallise dans l'éthanol à deux reprises et obtient 2,165 g de produit attendu. F = 240°C.

/α/D = +8° ±2° (c = 0,5%, éthanol).

| Spectre UV | | |
|---|---|---|
| max. 244 nm | $E^1_1$ = 341 | ε = 14300 |
| infl. 270 nm | $E^1_1$ = 89 | |

Exemple 7 : 10β-(2-méthyl prop-2-én-1-yl) 17 α-(prop-1-ynyl) 17β-hydroxy-estra-4,9(11)-dièn-3-one.

Stade A : 3,3 bis méthoxy-10β-(2-méthyl prop-2-én-1-yl) 17α -(prop-1-ynyl) estr-9(11)-èn 5α,17β-diol.

a/. Magnésien du 1-chloro 2-méthyl prop-2-én.

A 38,9 g de magnésium en tournure dans 200 cm3 de tétrahydrofuranne, on introduit en 4 heures et 15 minutes à +10° C, 26,8 cm3 de 2-chloropropène, agite pendant 2 heures, en laissant revenir à +20°C et obtient un magnésien titrant 0,5 M/l.

b/. 3,3-bis méthoxy-10β-(2-méthyl prop-2-én-1-yl) 17 α-(prop-1-ynyl) est-9(11)-èn 5α, 17β -diol.
A 214 cm3 de magnésien obtenu précédemment, on ajoute, goutte à goutte, en 15 minutes, sous agitation et azote à +10°C, une solution de 10 g de produit comprenant deux tiers de 3,3 bis méthoxy 5α, 10α-époxy 17α-(prop-1-ynyl) estr-9(11) én 17β-ol et un tiers de l'époxyde de 5β, 10β dans 50 cm3 de tétrahydrofuranne, laisse revenir à 20°C et agite pendant 20 heures, verse le mélange réactionnel dans une solution aqueuse de chlorure d'ammonium, extrait à l'éther éthylique, lave avec une solution aqueuse saturée de chlorure de sodium, concentre à sec sous pression réduite, chromatographie le résidu sur silice, en éluant par un mélange de chlorure de méthylène, d'acétone et de triéthylamine (95-5-0,1) et obtient 5,5 g de produit attendu Rf = 0,4.

Stade B :10β-(2-méthyl prop-2-én-1-yl) 17α-(prop-1-ynyl) 17β-hydroxy estra 4,9(11) dièn-3-one.

A une solution de 5,4 g de produit obtenu au stade A dans 54 cm3 d'éthanol, on ajoute sous agitation et azote 25 cm3 de solution aqueuse 2 N d'acide chlorhydrique, agite pendant 45 minutes au reflux, refroidit à +5°C, amène à pH 7 par addition d'une solution aqueuse 2 N de soude, extrait au chlorure de méthylène, lave la phase chlorométhylénique à l'eau, sèche, concentre à sec sous pression réduite, chromatographie le résidu sur silice, en éluant avec un mélange de chlorure de méthylène et d'acétone (95/5), cristallise dans l'éther isopropylique à deux reprises et obtient 1,92 g de produit attendu. Rf = 0,35. F = 133°C.
/α/D = -19° ± 2° (c = 0,5 %, éthanol)

| Analyse: | $C_{25}H_{32}O_2$ = 364,51 | |
|---|---|---|
| Calculé: | C % : 82,37 | H % : 8,85 |
| Trouvé: | 82,1 | 9,0 |

Exemple 8 : 10β-(prop-2-ynyl) 17α- (prop-1-ynyl) 17β-hydroxy estra-4,9(11)-dièn-3-one.

Stade A : 3,3-bis méthoxy 10β-(prop-2-ynyl) 17α-(prop-1-ynyl) estra-9((11)-én 5α, 17β-diol.

a/. Magnésien du 1 bromo prop-2-yne.

En opérant de façon analogue à celles décrites dans les exemples précédents, on obtient une solution de magnésien attendu titrant 1,55 M/l.

b/. 3,3-bis méthoxy 10β-prop-2-ynyl 17α -(prop-1-ynyl) estra-9(11)-en 5α,17β-diol.
On ajoute en 20 minutes, sous agitation et azote à +5°C, une solution de 10 g de produit comprenant deux tiers de 3,3-bis méthoxy 5α,10α-époxy 17α-(prop-1-ynyl) estr-9,11-én 17β-ol et un tiers de d'époxyde 5β,10β dans 50 cm3 de tétrahydrofuranne à 69 cm3 de solution de magnésien préparé comme au stade a/, agite pendant 2 heures et 40 minutes en laissant revenir à 20° C, verse sous agitation dans une solution aqueuse de chlorure d'ammonium, extrait à l'éther éthylique, lave la phase organique à l'eau saturée de chlorure de sodium, sèche, concentre à sec sous pression réduite, chromatographie sur silice en éluant par un mélange de chlorure de méthylène, d'acétone et de triéthylamine (95-5-0,1) et obtient 3,3 g de produit attendu Rf = 0,4. F = 217°C.

Stade B : 10β-(prop-2-ynyl) 17α-(prop-1-ynyl) 17β-hydroxy estra 4,9(11)-dièn 3-one.

Dans une suspension de 2,8 g de produit obtenu au stade A dans 28 cm3 d'éthanol, on ajoute sous agitation et azote 13,5 cm3 de solution aqueuse 2 N d'acide chlorhydrique, porte au reflux, agite pendant 45 minutes au reflux, refroidit à +5°C, amème à pH 7 par addition d'une solution aqueuse 2 N de soude, extrait au chlorure de méthylène, lave à l'eau, concentre à sec sous pression réduite, chromatographie le résidu sur silice, en éluant par un mélange de chlorure de méthylène et d'acétone (95/5), cristallise dans l'ether isopropolyque à deux reprises et obtient 1,336 g de produit attendu. F = 163°C. Rf = 0,937.
$/\alpha/_D$ = 32° ± 2°(c = 0,6 %, éthanol)

**Spectre UV (éthanol)**
**max. 240 nm $E^1_1$ = 467 ε = 16300**

Exemple 9 : 10β-/(2pyridinyl) thio/ 17α-(prop-1-ynyl) 17β -hydroxy estra-4,9(11)dièn-3-one.

Stade A : 3,3-bis méthoxy 10β-/(2 pyridinyl) thio/ 17α-(prop-1-ynyl) estra-9(11) én 5α,17β-diol.

a/. préparation du dérivé lithien

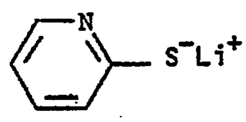

A une solution de 17,78 g de 2 mercapto pyridine dans 75 cm3 de tétrahydrofuranne, on ajoute en 30 minutes à -35°C, sous agitation et azote, 87,5 cm3 de butyllithium en solution 1,6 N dans l'hexane, agite pendant 1 heure, en laissant revenir à 0°C et obtient une suspension du dérivé lithien.

b/. 3,3-bis méthoxy 10β-/(2-pyridinyl) thio/ 17α-(prop-1-ynyl) estra 9(11)-én 5α, 17β-diol.
A la suspension de dérivé lithien obtenue précédemment, on ajoute en 20 minutes à 0°C une solution de 12 g de produit comprenant deux tiers de 3,3-bis méthoxy 5α,10α-époxy 17α-(prop-1-ynyl) estr-9(11)-én-17β-ol et un tiers de l'époxyde 5β,10β dans 60 cm3 de tétrahydrofuranne, agite pendant 2 heures et 30 minutes, en laissant revenir à 20°C, introduit le mélange réactionnel dans une solution aqueuse de chlorure d'ammonium, extrait au chlorure de méthylène, lave à l'eau, sèche, concentre à sec sous pression réduite, chromatographie le résidu sur silice en éluant par un mélange cyclohexane-acétate d'éthyle (1-1) et obtient 9,6 g de produit attendu Rf = 0,37 (produit I).
Spectre I R (chloroforme)
Absorption à 3420 $cm^{-1}$ attribuée au 5-hydroxy ;
absorption à 1573, 1560$cm^{-1}$ du système conjugué ;

absorption à 3603, 2240 $cm^{-1}$ attribuée à 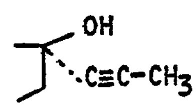

Dans la chromatographie, on isole d'autre part 1,1 g de 3-3-bis méthoxy 10α-/(2-pyridinyl) thio/ 17α-(prop-1-ynyl) estra-9(11)-én 5β, 17β-diol. Rf = 0,27 (produit II)
Spectre I R (chloroforme)
Absorption à 3440 $cm^{-1}$ attribuée au 5-hydroxy.

Stade B : 10β-/(2-pyridinyl) thio/ 17α-(prop-1-ynyl) 17β-hydroxy estra-4,9(11)-dièn-3-one.

A une solution de 7,5 g de produit obtenu au stade A (produit I) dans 150 cm3 d'éthanol, on ajoute 30 cm3 d'une solution aqueuse 2 N d'acide chlorhydrique, agite pendant 20 heures à 40° C, amène à pH 7 par addition d'une solution aqueuse saturée de bicarbonate de sodium, extrait à l'acétate d'éthyle, lave à l'eau, sèche, concentre à sec sous pression réduite, chromatographie le résidu sur silice, en éluant par un mélange chlorure de méthylène et d'acétone (9/1), empâte à l'éther éthylique, cristallise dans l'éthanol et obtient 0,966 g de produit attendu.
F = 192°C. Rf = 0,35.
$/\alpha/_D$ = +174,5° (c = 0,4 %, éthanol)

| | | | | |
|---|---|---|---|---|
| **Analyse:** | **$C_{26}H_{29}NO_2S$ = 419,50** | | | |
| **Calculé:** | **C % : 74,42** | **H % : 6,96** | **N % : 3,33** | **S % : 7,64** |
| **Trouvé:** | **74,2** | **7,0** | **3,3** | **7,5** |

Lors de la chromatographie, on isole d'autre part 0,6 g de 17α-(1-pronynyl) estra 1,3,5(10), 9(11)-tétraèn-3,17β-diol. Rf = 0,55
Spectre I R (chloroforme)
Absorption à 3600$^{cm-1}$ attribuée à OH ;
absorption à 2130$^{cm-1}$ à c≡c ;
absorption à 1630$^{cm-1}$ attribué à Δ 9(11).

<u>Exemple 10 : 10β-/(4 méthoxy phényl) thio/ 17α-(prop-1-ynyl) 17β-hydroxy estra 4,9(11)-dièn-3-one.</u>

<u>Stade A</u> : 3,3-bis méthoxy 10β-/(4-méthoxyphényl) thio/ 17α-(prop-1-ynyl) estra-9(11)-én 5α,17β-diol.

a/. préparation du dérivé lithien

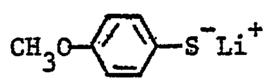

On ajoute à -35°C, en 30 minutes, sous agitation et azote, 73 cm3 de solution 1,6 M/l de butyl lithium dans l'hexane à une solution de 18,8 g de paraméthoxy benzème thiol dans 75 cm3 de tétrahydrofuranne, agite en laissant revenir à 0°C et obtient une solution du dérivé lithien

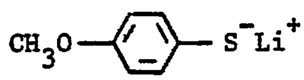

b/. 3,3-bis méthoxy 10β-/(4-méthoxyphényl) thio 17α-(1-propynyl) estra-9(11)èn 5α, 17β-diol.
On ajoute à la solution de dérivé lithien obtenue précédemment, à 0° C, en 30 minutes, 12 g d'une solution de produit comprenant deux tiers de 3,3-bis méthoxy 5α,10α-époxy 17α-(prop-1-ynyl) estra-9(11) én 17β-ol et un tiers de l'époxyde 5β,10β dans 60 cm3 de tétrahydrofuranne, agite pendant 2 heures et 10 minutes, en laissant revenir à 20°C, introduit le mélange réactionnel dans une solution aqueuse de chlorure d'ammonium, extrait à l'éther éthylique, lave à l'eau, concentre à sec sous pression réduite, chromatographie le résidu sur silice, en éluant par le mélange chlorure de méthylène acétone (95/5) et obtient 10,2 g de produit attendu Rf = 0,4 (produit I ).
Dans la chromatographie, on isole d'autre part 3,7 g de 3,3-bis méthoxy 10α-/(4-méthoxyphényl) thio/ 17α-(prop-1-ynyl) estra-9(11)-én 5β, 17β-diol, Rf = 0,3 (produit II).

<u>Stade B</u> : 10β-/(4-méthoxyphényl) thio/ 17α-(prop-1-ynyl) 17β-hydroxy estra-4,9(11) dièn-3-one.

Dans 110 cm3 d'éthanol, on introduit sous agitation et azote 5,5 g deproduit obtenu au stade A (produit I), ajoute 22 cm3 de solution aqueuse 2 N d'acide chlorhydrique et agite 20 heures à 40°C. On refroidit à +5°C, amène à pH 7, par addition d'une solution aqueuse saturée de bicarbonate de sodium, extrait du chlorure de méthylène, lave à l'eau, concentre à sec sous pression réduite, chromatographie le résidu sur silice, en éluant par un mélange de chlorure de méthylène et d'acétone (95/5), empâte à l'éther éthylique, cristallise dans un mélange de 15 cm3 d'éthanol et de 10 cm3 de chlorure de méthylène, élimine le chlorure de méthylène par distillation, glace, essore et sèche sous pression réduite.
On obtient 2,290 g de produit attendu Rf = 0,27, F = 206°C.
$/\alpha/_D$ = +93° (c = 0,6 %, éthanol).

**Spectre UV (éthanol)**

| | | |
|---|---|---|
| max. 234 nm | $E^1_1$ = 532 | ε = 23900 |
| infl. 254 nm | $E^1_1$ = 333 | ε = 14900 |
| infl. 282 nm | $E^1_1$ = 160 | ε = 7200 |

Exemple 11 : 10β -/(4-méthylphényl) méthyl/ 17α-(1-prop-1-ynyl) 17β-hydroxy estra 1, 4, 9(11)-trièn-3-one.

A une solution de 1,21g de 10β-/(4-méthylphényl) méthyl/ 17α-(prop-1-ynyl) 17β-hydroxy estra 1, 4, 9(11)-dièn-3-one obtenue au stade B de l'exemple 4 dans 24 cm3 de tétrahydrofuranne, on ajoute 1,56 g d'anhydride phényl séléninique, porte au reflux sous agitation, maintient le reflux pendant 22 heures, refroidit, introduit le mélange réactionnel, à +5°C, dans une solution aqueuse saturée de bicarbonate de sodium, isole par essorage le précipité formé, obtient 0,354 g de produit attendu, F = 262°C, extrait les liqueurs mères à l'acétate d'éthyle, concentre à sec, chromatographie sur silice, en éluant par un mélange de chlorure de méthylène et d'acétone (95/5) et obtient 0,723 g de produit attendu F = 262°C. On joint les deux fractions du produit attendu obtenues précédemment (1,077 g), cristallise à deux reprises dans un mélange d'éthanol et de chlorure de méthylène et obtient 0,848 g de produit attendu pur F = 262°C, Rf = 0,34.
$/\alpha/_D$ = -60° ± 2,50° (c = 0,5 %, chloroforme).

Spectre UV (éthanol)

| | | |
|---|---|---|
| max. 222 nm | $E^1_1$ = 476 | ε = 19600 |
| max. 241 nm | $E^1_1$ = 330 | ε = 13600 |
| infl. 265 nm | $E^1_1$ = 170 | ε = 7000 |

Exemple 12 : 10β-/(2-fluorophényl) méthyl/ 17α-(prop-1-ynyl) 17β-hydroxy estra-4,9(11)-dièn-3-one.

Stade A : 3,3-bis méthoxy 10β-/(2-fluorophényl) méthyl/ 17α-(prop-1-ynyl) estra-9(11)-én 5α, 17β-diol.

a/. préparation du magnésien du chlorure de 2-fluorobenzyle.

Dans un mélange de 8,2 g de magnésium en tournure dans 30 cm3 d'éther éthylique, on ajoute, sous agitation, 0,5 cm3 de chlorure de 2-fluorobenzyle. La réaction étant amorcée, on ajoute, goutte à goutte, en 1 heure environ, sans dépasser 35°C, une solution de 40 cm3 de chlorure de 2-fluorobenzyle dans 220 cm3 d'éther éthylique, laisse revenir à 20°C en 30 minutes environ, et obtient une solution de magnésien

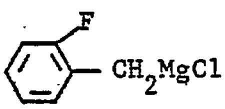

titrant 1,25 M/l.

b/. 3,3-bis méthoxy 10β-/(2-fluoro phényl) méthyl/ 17α-(prop-1-ynyl) estra 9(11) en 5α, 17β-diol.
Dans 90 cm3 de la solution de magnésien précédemment obtenue, on ajoute goutte à goutte, en 25 minutes environ, à 0°C une solution de 10 g de produit comprenant 50 % de 3,3-bis méthoxy 5α, 10α-époxy 17α-(prop-1-ynyl) estr-9(11)-èn 17β-ol, 30 % de l'époxyde 5β, 10β et 20 % de 3,3-bis méthoxy 17α-(prop-1-ynyl) estr-5(10), 9(11)-dièn 17 -ol dans 50 cm3 de tétrahydrofuranne, agite pendant 30 minutes à 0°C, laisse revenir à température ambiante en 1 heure et 30 minutes, introduit le mélange réactionnel dans une solution aqueuse de chlorure d'ammonium, extrait à l'éther éthylique, lave à l'eau, concentre à sec par distillation sous pression réduite, chromatographie le résidu sur silice, en éluant avec un mélange de chlorure de méthylène, d'acétone et de triéthylamine (95/5/0,1) et obtient 2,75 g de produit attendu (produit I), Rf = 0,55.

Spectre UV (éthanol)

| | |
|---|---|
| max. 244 nm | $E^1_1$ = 12 |
| max. 257 nm | $E^1_1$ = 18 |
| max. 263 nm | $E^1_1$ = 24 |
| max. 263 nm | $E^1_1$ = 25 |

Dans la chromatographie, on obtient d'autre part 1,45 g de 3,3-bis méthoxy 10α-/(2-fluorophényl) méthyl/ 17α-(prop-1-ynyl) estra 9(11)-én 5β, 17β-diol (produit II) Rf = 0,40.
Dans la chromatographie, on obtient une troisième fraction constituée par le 3,3-bis méthoxy 10β-/(2-fluorophényl) méthyl/ 17α-(prop-1-ynyl) estra-9-én 5α,17β-diol Rf = 0,45 (produit III).

Stade B : 10β-/(2-fluorophényl) méthyl/ 17α-(prop-1-ynyl) 17β-hydroxy estra 4,9(11)-dièn-3-one.

Dans une solution de 2,6 g de produit (I) obtenu au stade A, dans 26cm3 d'éthanol, on introduit à 40°C, 10,8 cm3 de solution aqueuse 2 N d'acide chlorhydrique, porte au reflux pendant 2 heures, refroidit à 20°C, filtre, lave à l'éther isopropylique, sèche et obtient 1,6 g de produit brut que l'on purifie par chromatographie sur silice, en éluant par un mélange de chlorure de méthylène et d'acétone (95/5) et obtient 1,5 g de produit attendu F = 212°C, Rf = 0,4.
/α/D = +12,5° ±2° (c = 0,5% chloroforme).

| | | | |
|---|---|---|---|
| Analyse: | $C_{28}H_{31}FO_2$ = 418,53 | | |
| Calculé: | C % : 80,35 | H % : 7,46 | F % : 4,53 |
| Trouvé: | 80,2 | 7,6 | 4,6 |

Dans la chromatographie, on obtient d'autre part 0,130 g de 10β-/(2-fluorophényl)méthyl/ 17α-(prop-1-ynyl) estra-9(11)-ène 5α,17β-diol-3 one, F = 240°C, Rf = 0,25.

Exemple 13 : 10β-phénylthio 17α-(prop-1-ynyl) 17β-hydroxy estra-4,9(11)-dièn-3-one.

Stade A : 3,3-bis méthoxy 10β-phénylthio 17α-(prop-1-ynyl) estra 9(11) én 5α,17β-diol.

a/. préparation du dérivé lithien

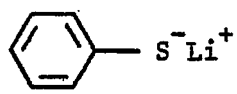

Dans une solution de 15 cm3 du thiophényl, dans 75 cm3 de tétrahydrofuranne, on introduit à -20°C, en 30 Minutes, 63 cm3 de butyllithium en solution dans l'hexane (titre 2,3 M/l), laisse revenir à 20°C en 30 minutes environ et obtient ainsi le dérivé lithien du thiophénol.

b/. 3,3-bis méthoxy 10β-phénylthio 17α-(prop-1-ynyl) estra 9(11) én 5α, 17β-diol.
On ajoute à 20°C, en 30 minutes environ, au dérivé lithien obtenu précédemment, 12 g de produit comprenant 50 % de 3,3-bis méthoxy 5α,10α-époxy 17α-(prop-1-ynyl) estr-9(11)-én 17β-ol, 30 % de l'époxyde 5β, 10β et 20 % de 3,3-bis méthoxy 17α-(prop-1-ynyl) estr -5(10) 9(11)-dièn 17β-ol dans 60cm3 de tétrahydrofuranne, agite pendant 2 heures à température ambiante, introduit le mélange réactionnel dans une solution aqueuse de chlorure d'ammonium, extrait à l'éther éthylique, lave à l'eau, concentre à sec par distillation sous pression réduite, chromatographie le résidu sur silice, en éluant par un mélange de chlorure de méthylène, d'acétone et de triéthylamine (95-5-0,1) et obtient 7,1 g de produit attendu F = 90-95°C, Rf = 0,45 (produit I).

| | | | |
|---|---|---|---|
| Analyse: | $C_{29}H_{38}O_4S$ = 482,688 | | |
| Calculé: | C % : 72,16 | H % : 7,93 | S % : 6,64 |
| Trouvé: | 72,2 | 8,0 | 6,5 |

| | | |
|---|---|---|
| Spectre UV (éthanol) | | |
| infl. 220 nm | $E^1_1$ = 192 | ε = 9300 |
| max. 267 nm | $E^1_1$ = 56 | ε = 2700 |

Dans la chromatographie, on obtient d'autre part 2,25 g de 3,3-bis méthoxy 10α-phénylthio 17α-(prop-1-ynyl) estra 9(11) én 5β, 17β-diol, Rf. = 0,35 (produit II).

Stade B : 10β-phénylthio 17α-(prop-1-ynyl) 17β-(hydroxy) estra-4,9(11)-dièn-3-one.

Dans une solution de 5,6 g de composé (I) obtenu au stade A dans 56 cm3 d'éthanol, on introduit, à 40°C, 23 cm3 de solution aqueuse 2 N d'acide chlorhydrique, porte au reflux pendant 1 heure et 30 minutes, refroidit, verse le mélange réactionnel dans une solution aqueuse saturée de bicarbonate de sodium, extrait au chlorure de méthylène, lave àl'eau, concentre à sec par distillation sous pression réduite, chromatographie le résidu sur silice, en éluant par un mélange de chlorure de méthylène et d'acétone

(95/5) et obtient après empâtage dans l'éther isopropylique 2,6 g deproduit attendu Rf = 0,4, F = 180° C.

| | | | |
|---|---|---|---|
| Analyse: | $C_{27}H_{30}O_2S$ = 418,603 | | |
| Calculé: | C % : 77,47 | H % : 7,22 | S % : 7,65 |
| Trouvé: | 77,3 | 7,3 | 7,5 |

Spectre UV (éthanol)

| | | |
|---|---|---|
| max. 224 nm | $E^1_1$ = 479 | ε = 20000 |
| max. 248 nm | $E^1_1$ = 394 | ε = 16500 |
| infl. 300 nm | $E^1_1$ = 51 | ε = 2100 |

Exemple 14 : 10β-/4(diméthylamino) phényl/ 17α-(prop-1-ynyl) 17β-hydroxy estra-4,9(11)dièn-3-one.

Stade A : 3,3-éthylènedioxy 10β-/4(diméthylamino) phényl/ 5α-hydroxy estra-9(11)-ène-17-one.

On opère comme au stade A de l'exemple 7 du brevet européen EP. 0.057.115.

On introduit en 2 heures 30 minutes, en maintenant la température à 35°C, une solution de 200 g de para bromo diméthylaminophényl dans 950 cm3 de tétrahydrofuranne dans un mélange de 29 g de magnésium en tournure dans 50 cm3 de tétrahydrofuranne. On initie la réaction par chauffage à 50°C.

On refroidit à 5°C un mélange de 25 g de 3,3-éthylénedioxy 5α,10α-époxy estra 9-ène 17-one dans 500 cm3 de tétrahydrofuranne et 757 mg de chlorure cuivreux et introduit, goutte à goutte, en 1 heure 15 minutes, 284 cm3 de la solution de bromure de 4-diméthylamino phénylmagnésium préparé précédemment.

On agite 15 minutes, verse dans un litre de solution aqueuse saturée de chlorure d'ammonium, extrait à l'acétate d'éthyle, lave avec une solution saturée de chlorure d'ammonium, puis avec une solution saturée de chlorure de sodium. On sèche, amène à sec sous pression réduite et obtient 46 g de produit brut.

Ce produit contient le produit principal qui est le 3,3-éthylène dioxy, 5α-hydroxy 11β-(diméthylamino phényl) estra-9(11)-èn-17-one.

Par chromatographie sur silice, on obtient le produit 10β recherché qui possède les Rf suivants :
Rf = 0,28 (éther de pétrole-acétate d'éthyle 5 : 5),
Rf = 0,32 (éther de pétrole-acétone 8 : 2),
Rf = 0,36 (benzène-acétate d'éthyle 8 : 2).

Stade B : 3,3-éthylènedioxy 10β-/4(diméthylamino) phényl/ 17α-(prop-1-ynyl) estra 9(11) én 5α, 17β-diol.

a/. Magnésien du méthyl acétylène

Dans 10cm3 de solution éthérée 1N de bromure d'éthyle magnésium et 10 cm3 de tétrahydrofuranne, on fait barboter pendant 1 heure à +3°C du méthyl acétylène et obtient le magnésien désiré.

b/. 3,3-éthylène dioxy 10β-/(4-diméthylamino) phényl/ 17α-(prop-1-ynyl) estra-9(11) én 5α,17β-diol.

On ajoute, en 5 minutes, à la solution de magnésien ci-dessus 1,13 g de 3 3-éthylène dioxy 10β-/(4-diméthyl amino) phényl/ 5α-hydroxy estra-9(11)-én-17-one préparé comme au stade A en solution dans 10 cm3 de tétrahydrofuranne, laisse revenir à 20°C, agite pendant 2 heures, ajoute au mélange réactionnel une solution aqueuse de chlorure d'ammonium, extrait au chloroforme, lave à l'eau, sèche, concentre à sec par distillation sous pression réduite, chromatographie le résidu sur silice en éluant par un mélange de benzène et d'acétate d'éthyle (7/3) et obtient 0,295 g de produit attendu,Rf = 0,32 (produit I).

Lors de chromatographie, on obtient d'autre part 0,810 g de 3,3-éthylène dioxy 10β-/(4-diméthylamino) phényl/ 17,17-éthylène dioxy estra-9(11)-èn-5α-ol. F = 212° C (produit II).

Stade C : 10β-/4(diméthylamino) phényl 17α-(prop-1-ynyl) 17β-hydroxy estra-4,9(11)-dièn-3-one.

A une solution de 0,295 g de composé (I) obtenu au stade A dans 4 cm3 d'éthanol, on ajoute à 20°C sous barbotage d'argon 0,6 cm3 de solution aqueuse 2N d'acide chlorydrique, agite pendant 2 heures à température ambiante, alcalinise par additionde bicarbonate de sodium, extrait au chlorure de méthylène, lave à l'eau, sèche, concentre à sec par distillation sous pression réduite, chromatographie le résidu sur silice en éluant par un mélange de benzène et d'acétate d'éthyle (9/1) et obtient 0,210 g de produit attendu, Rf = 0,13.

| | | | |
|---|---|---|---|
| Analyse: | $C_{29}H_{35}NO_2$ = 429,58 | | |
| Calculé: | C % : 81,08 | H % : 8,21 | N % : 3,26 |
| Trouvé: | 81,1 | 8,5 | 3,1 |

Spectre UV (éthanol)

| | | |
|---|---|---|
| infl. 246 nm | $E^1_1$ = 510 | |
| max. 259 nm | $E^1_1$ = 576 | ε = 24700 |
| max. 297 nm | $E^1_1$ = 61 | ε = 2600 |

Exemple 15 : 17β-hydroxy 10β-/(4-méthyphényl) méthyl/ 17α-(prop-1-ynyl) estra 4,6,9(11) trièn-3-one.

1/. On agite, pendant une heure, à température ambiante, une suspension de 4 g de 17β-hydroxy 10β-/(4-métylphényl) méthyl/ 17α-(1-propynyl) estra 4,9(11) dién-3-one obtenue à l'exemple 4,80 cm3 d'éthanol, 12 cm3 d'orthoformiate d'éthyle et 16 mg d'acide paratoluène sulfonique, puis ajoute 3 cm3 de triéthylamine. On agite pendant 5 minutes, puis verse dans 100 cm3 d'une solution aqueuse de carbonate acide de sodium. On agite pendant 15 minutes, puis extrait au dichlorométhane.
On amène à pH alcalin par 2 cm3 de triéthylamine, puis sèche et concentre à sec. On obtient 6,5 g d'une huile incolore.

2/. L'huile précédente est ajoutée à une solution de 2,64 g de chroranile dans 100 $cm^3$ d'acétone à 5 % d'eau. Après une heure de réaction, on introduit 100 $cm^3$ de thiosulfate de sodium à 10% et 100 $cm^3$ de solution de carbonate acide de sodium. On agite une heure, puis extrait la phase aqueuse au dichlorométhane. On obtient 4,8 g de produit brut que l'on chromatographie sur silice (éluant : éther de pétrole (Eb. 60-80°C)-acétate d'éthyle 7 : 3). On obtient 3,341 g de mousse blanche que l'on recristallise dans l'acétate d'éthyle. On recueille le produit cristallise, le lave à l'acétate d'éthyle glacé, puis à l'éther de pétrole(Eb 60-80°C), le sèche à 50° C sous pression réduite et obtient 2,142 g de produit attendu, F = 202°C, Rf = 0,17.

| | | |
|---|---|---|
| Analyse: | $C_{29}H_{32}O_2$ = 412,57 | |
| Calculé: | C % : 84,43 | H % : 7,82 |
| Trouvé: | 84,7 | 7,9 |

Exemple 16 : 17β-hydroxy 6-méthylène 10β-/(4-méthylphényl) méthyl/ 17α-propynyl estra 4,9(11) dièn-3-one.

Dans une solution comprenant 3,6 g d'acétate de sodium dans 102 cm3 de chloroforme, on ajoute 102 cm3 de diéthylacétal du formaldéhyde, 13 cm3 d'oxychlorure de phosphore et 3 g de produit obtenu comme à l'exemple 4. On chauffe à 80°C pendant 40 minutes, refroidit dans un bain de glace additionné de méthanol, puis ajoute lentement du carbonate de sodium puis 100 cm3 d'eau. On extrait au chloroforme, concentre sous pression réduite, purifie par chromatographie sur silice (éluant : cyclohexane-acétate d'éthyle 8-2) et recueille 627 mg de produit attendu. F = 188°C après cristallisation dans l'éther.

| | | |
|---|---|---|
| Analyse: | $C_{30}H_{34}O_2$ = 426,60 | |
| Calculé: | C % : 84,47 | H % : 8,03 |
| Trouvé: | 84,2 | 8,1 |

Exemple 17 : 17β-hydroxy 6-méthyl 10β-/(4-méthylphényl) méthyl/ 17α-propynyl estra 4,6,9(11)trièn-3-one.

Stade A : 17β-cyano 17α-hydroxy 10β-/(4-méthylphényl) méthyl/ estra 4,9(11) dièn-3-one.

1) Dans 250 cm3 d'une solution tétrahydrofurannique d'α-chloro paraxylène magnésium (0,75 M) refroidie à +14°C, on ajoute 20 g de 3,3-éthylènedioxy 5α, 10α-époxy 17α-triméthylsilyloxy 17β-cyano estr-9(11)ène préparé comme dans le brevet français 2 082 129 en solution dans 100 cm3 de tétrahydrofuranne. On laisse revenir à température ambiante, puis verse dans une solution aqueuse glacée de chlorure d'ammonium et extrait au chlorure de méthylène. On sèche, évapore le solvant et récupère 37,7 g de produit brut.

2) On dissout le produit brut ci-dessus dans 500 cm3 d'éthanol et 100 cm3 d'acide chlorhydrique 5 N.

On chauffe 1 heure au reflux, glace, filtre, lave le produit cristallisé à l'éthanol et le sèche à 70°C sous pression réduite. On recueille 13,6 g de produit attendu. F = 234°C.

Stade B : 17β-cyano 17-α-hydroxy 6-méthylène 10β-/(4-méthylphényl) méthyl/ estra 4,9(11)dièn-3-one.

On ajoute 8 g de produit obtenu au stade A dans une solution comprenant 9,3 g d'acétate de sodium, 282 cm3 de diéthylacétal du formaldéhyde, 282 cm3 de chloroforme et 34,5 g d'oxychlorure de phosphore. On chauffe 45 minutes à 70°C, refroidit à température ambiante et verse dans une solution aqueuse glacée saturée de bicarbonate de sodium. On agite 45 minutes puis extrait au chloroforme. On concentre à sec, chromatographie le résidu sur silice (éluant : cyclohexane-acétate d'éthyle 6-4), concrète le résidu dans l'éther et isole 2 g de produit attendu. F = 216°C.

Stade C : 17β-cyano 17α-hydroxy 6-méthyl 10β-/(4-méthylphényl) méthyl/ estra 4,6,9(11)trièn-3-one.

On porte au reflux 2,5 g de produit obtenu au stade précédent et 1,5 g de charbon actif à 5% de palladium dans 100 cm3 d'éthanol et introduit en 2 heures 100 cm3 d'éthanol contenant 5% d'alcool benzylique. On refroidit le mélange réactionnel, filtre le catalyseur, évapore les solvants sous pression réduite et purifie le résidu par chromatographie sur silice (éluant : n-hexane-acétate d'éthyle 7-3). On obtient 2,42 g de produit attendu utilisé tel quel pour le stade suivant.

Stade D : 3,3-éthylènedioxy 6-méthyl 10β-/(4-méthylphényl) méthyl/ estra 4,6,9(11)trièn-17-one.

On chauffe 4 heures au reflux 2,4 g de produit obtenu au stade précédent dans 150 cm3 de benzène avec 70 mg d'acide paratoluène sulfonique et 11 cm3 d'éthylène glycol. On concentre en quart du volume et refroidit à température ambiante. On ajoute 10 cm3 de lessive de soude et 50 cm3 d'éthanol, agite 2 heures, dilue avec 100 cm3 d'eau et extrait au chlorure de méthylène. On élimine les solvants, purifie le résidu par chromatographie sur silice (éluant : n-hexane-acétate d'éthyle 7-3 à 1% de triéthylamine. On récupère 1,86 g de produit attendu.

Stade E : 17β-hydroxy 6-méthyl 10β-/(4-méthylphényl) méthyl/ 17α-propynyl estra 4,6,9(11)trièn-3-one.

On ajoute 50 cm3 de tétrahydrofuranne dans 17 cm3 d'une solution hexanique de butyllithium (1M:l), refroidit à -70°C et soumet à un barbotage de méthylacétylène pendant 30 minutes. On maintient sous agitation pendant 30 minutes, ajoute 1,5 g du produit obtenu au stade précédent dans 2 cm3 de tétrahydrofuranne, laisse revenir à température ambiante et laisse 2 heures sous agitation. On verse le milieu réactionnel dans une solution aqueuse glacée de chlorure d'ammonium, extrait à l'acétate d'éthyle, concentre, reprend le résidu dans un mélange de 2 cm3 d'acide chlorhydrique 2 N et 10 cm3 d'éthanol, agite 1 heure 30 minutes et récupère 1,5 g de produit brut que l'on purifie par chromatographie sur silice (éluant : n-hexane-acétate d'éthyle 7-3). On isole 72 mg de produit attendu.
$/\alpha/_D$ = -175° ± 3° (c = 0,5% $CHCl_3$).

Exemple 18 : 17β-hydroxy 10β-/(4-méthylphényl) méthyl/ 17α-(3-hydroxy 1-propynyl) estra 4,9(11) dièn-3-one.

Stade A : 3,3-éthylènedioxy 5α-hydroxy 17β-cyano 17α-triméthylsilyloxy 10β-/(4-méthylphényl) méthyl/ estra 9(11) ène et 3,3-éthylène dioxy 5β-hydroxy 17β-cyano 17α-triméthylsilyloxy 10α-/(4-méthylphényl) méthyl/ estra 9(11) ène sous forme de mélange.

On opère comme au stade A (1) de l'exemple 17, en utilisant au départ 37,4 g de 3,3-éthylène dioxy 5,10-époxy 17α-triméthylsilyloxy 17β-cyano estr-9(11) ène sous forme du mélange d'isomère 5α, 10α et d'isomère 5β, 10β-époxy. On obtient 67 g de produit brut attendu sous forme de mélange d'isomères utilisé tel quel pour le stade suivant.

Stade B : 3,3-éthylènedioxy 5α-hydroxy 10β-/(4-méthylphényl) méthyl/ estra 9(11) ène et 3,3-éthylène dioxy 5β-hydroxy 10α-/(4-méthylphényl) méthyl/ estra 9(11) èn-17-one.

On ajoute 70 cm3 de lessive de soude à une solution de 58,15 g de produit obtenu au stade A dans 2,4 cm3 d'éthanol. On agite 30 minutes, élimine le solvant sous pression réduite à 35-40°C, ajoute de l'eau, extrait au chlorure de méthylène, lave la phase organique à l'eau puis avec une solution aqueuse saturée de chlorure de sodium, sèche et concentre à sec. On isole 44,4 g de produit brut. On isole les 2 isomères par chromatographie sur silice (éluant : éther de pétrole (Eb = 40-70°C)-acétate d'éthyle 7-3 à 1‰ de triéthylamine. Après recristallisation des différentes fractions dans l'éther isopropylique, on recueille 18,6 g d'isomère 5α-hydroxy 10β-/(4-méthylphényl méthyl/ et 3,66 g d'isomère 5β-hydroxy 10α-/(4-méthylphényl) méthyl/.

Stade C : 3,3-éthylènedioxy 5 α,17β-dihydroxy 10β-/(4-méthylphényl) méthyl/ 17α-/3-(2-tétrahydropyranyloxy) 1-propynl/ estr-9(11)-ène et isomère 5β-hydroxy 10α-/(4-méthylphényl) phényl/.

a) Dérivé lithien.
Dans 9,4 cm3 d'une solution éthérée de méthyllithium (1,6 M/l) refroidie à +10°C, on ajoute en 20 minutes, 2,2 g d'éther tétrahydropyrannique de l'alcool propargylique en solution dans 80 cm3 d'éther puis on laisse revenir à température ambiante.

b) On ajoute 874 mg de produit obtenu comme au stade B dans la suspension de dérivé lithien préparée ci-dessus et agite 7 heures à température ambiante. On verse le mélange réactionnel dans une solution aqueuse saturée de chlorure d'ammonium glacée, extrait à l'acétate d'éthyle, lave la phase organique avec une solution aqueuse saturée de chlorure de sodium, sèche et concentre à sec sous pression réduite. On chromatographie le résidu sur silice, éluant : cyclohexane-acétate d'éthyle (6-4) à 1‰ de triéthyl-amine et isole 860 mg de produit attendu sous forme d'isomère 5α-hydroxy 10β-/(4-méthylphényl) méthyl/ ainsi que 80 mg d'isomère 5β-hydroxy 10α-/(4-méthylphényl) méthyl/.

Stade D : 17β-hydroxy 10β-/(4-méthylphényl) méthyl/ 17α-(3-hydroxy 1-propynyl) estra 4,9(11) dièn-3-one.

On chauffe 1 heure à 50°C la solution comprenant 1,7 g de produit sous forme d'isomère 5α-hydroxy 10β-/(4-méthylphényl) méthyl/ obtenu comme au stade précédent, 30 cm3 d'éthanol et 7,5 cm3 d'aide chlorhydrique 5 N. On verse le mélange réactionnel dans de l'eau glacée, alcalinise à l'aide d'ammoniaque concentré, extrait à l'acétate d'éthyle, lave la phase organique à l'eau puis avec une solution aqueuse saturée de chlorure de sodium, sèche et concentre à sec sous pression réduite. On obtient 1,3 g de produit brut que l'on recristallise dans le chlorure de méthylène.
F = 213°C.
/α/$_D$ = +13° ± 1° (c = 1% $CHCl_3$).

| | | | |
|---|---|---|---|
| **Analyse:** | **$C_{29}H_{34}O_3$ = 430,59** | | |
| **Calculé:** | **C % : 79,95** | **H % : 7,87** | **Cl % : 1,17** |
| **Trouvé:** | **80,0** | **7,9** | **1,2** |

Exemple 19 : 21-chloro 17β-hydroxy 10β-/(4-méthylphényl) méthyl/ 19-nor 17α-pregna-4,9(11)-dièn-20-yn-3-one.

Stade A : 3,3-éthylènedioxy 21-chloro 5α,17β-dihydroxy 10β-/(4-méthylphényl) méthyl/ 19-nor 20-yn 17α-pregna-9(11)-ène.

a) Préparation du chloroacétylure de lithium.
Dans 5,5 cm3 d'éther refroidie à 5°C, on ajoute en 3 minutes 3,3 cm3 d'une solution éthérée de phényllithium 0,003M puis en 6 minutes 0,12 cm3 de trans 1,2-dichloroéthylène en maintenant la température inférieure à 10°C. On laisse revenir à température ambiante et agite 40 minutes.

b) A la suspension obtenue précédemment, on ajoute 0,218 g de 3,3-éthylène dioxy 5α-hydroxy 10β-/(4-méthylphényl) méthyl/ estr-9(11)-en-17-one préparé comme au stade B de l'exemple 18 en solution dans 2,2 cm3 de tétrahydrofuranne et agite 16 heures à température ambiante. On ajoute 5,5 cm3 d'une solution aqueuse saturée de chlorure d'ammonium, agite 10 minutes, décante, extrait à l'acétate d'éthyle, lave la phase organique avec une solution aqueuse saturée de chlorure de sodium, sèche et évapore à sec. On chromatographie le résidu sur silice (éluant : éther de pétrole (Eb :40°-70°C)-acétate d'éthyle (7-3) à 1‰ de triéthylamine et isole 0,194 g de produit attendu.

Stade B : 21-chloro 17β-hydroxy 10β-/(4-méthylphényl) méthyl/ 19-nor 17α-pregna-4,9(11)-dièn-20-yn-3-one.

On ajoute 42 cm3 d'acide chlorhydrique au demi dans une solution comprenant 3,5 g de produit préparé comme au stade A et 105 cm3 d'éthanol. On chauffe 4 heures à 55°C, refroidit le milieu réactionnel, ajoute de l'eau puis 20 cm3 d'ammoniaque concentrée, esore, lave à l'eau, sèche et concentre à sec sous pression réduite à 50°C. Onobtient 2,62 g de produit attendu. F = 254°C.
/α/$_D$ = +13° ± 1° (c = 1% $CHCl_3$).

| | | | |
|---|---|---|---|
| Analyse: | $C_{28}H_{31}ClO_2$ = 435,01 | | |
| Calculé: | C % : 77,31 | H % : 7,18 | Cl % : 8,14 |
| Trouvé: | 77,3 | 7,3 | 8,3 |

Etude pharmacologique des produits de l'invention

I/Etude de l'activité des produits de l'invention sur les récepteurs hormonaux:

Récepteur Progestogène de l'utérus de lapine:

Des lapines impubères d'environ 1 kg reçoivent une application cutanée de 25 g d'estradiol. 5 jours après ce traitment, les animaux sont sacrifiés, les utérus sont prélevés, pesés et homogénéisés à 0°C, à l'aide d'un Potter teflon-verre dans une solution tamponnée TS (Tris 10mM, saccharose 0,25M, HCl pH 7,4) 1 g de tissu pour 50 ml de TS). L'homogénat est ensuite ultracentrifugé (105 000 g x 90 mn) à 0°C. Des aliquotes du surnageant ainsi obtenu, sont incubées à 0°C pendant un temps t, avec une concentration constante (T) de Produit R tritié (17,21-diméthyl 19-nor-4,9-pregnadiène-3,20-dione) en présence de concentrations croissantes (0 - 2 500. $10^{-9}$M) soit de R froid, soit de progestérone froide, soit du produit froid à tester. La concentration de R tritié lié (B) est ensuite mesurée dans chaque incubat par la technique d'adsorption au charbon dextran.

Récepteur glucocorticoïde du thymus de rat:

Des rats mâles Sprague Dawley EOPS de 160 à 200 g sont surrénalectomisés. 4 à 8 jours après cette ablation, les animaux sont sacrifiés, et les thymus sont prélevés et homogénéisés à 0°C dans un tampon Tris 10mM, saccharose 0,25 M, dithiotreitol 2mM, HCL pH 7,4, à l'aide d'un Potter polytétrafluoroéthylène-verre (1 g de tissu pour 10 ml de TS). L'homogénat est ensuite ultracentrifugé (105 000 g x 90 mn) à 0°C. Des aliquotes du surnageant ainsi obtenu, sont incubées à 0°C pendant un temps (t) avec une concentration constante (T) de dexaméthasone tritiée en présence de concentrations croissantes (0 - 2 500.$10^{-9}$M) soit de dexaméthasone froide, soit du produit froid à tester. La concentration de la dexaméthasone tritiée liée (B) est ensuite mesurée dans chaque incubat par la technique d'adsorption au charbon dextran.

Calcul de l'affinité relative de liaison:

Le calcul de l'affinité relative de liaison (ARL) est identique pour tous les récepteurs.

On trace les deux courbes suivantes: le pourcentage de l'hormone tritiée liée

$$\frac{B}{T}$$

en fonction du logarithme de la concentration de l'hormone de référence froide et

$$\frac{B}{T}$$

en fonction du logarithme de la concentration du produit froid testé.

On détermine le droite d'équation

$$I_{50} = \left(\frac{B}{T} \max + \frac{B}{T} \min\right)/2.$$

$$\frac{B}{T} \max =$$

Pourcentage de l'hormone tritiée liée pour une incubation de cette hormone tritiée à la concentration (T).

$$\frac{B}{T} \min =$$

Pourcentage de l'hormone tritiée liée pour une incubation de cette hormone tritiée à la concentration (T) en présence d'un grand excès d'hormone froide ($2500.10^{-9}$M).

Les intersections de la droite $I_{50}$ et des courbes permettent d'évaluer les concentrations de l'hormone de référence froide (CH) et du produit froid testé (CX) qui inhibent de 50 % la liaison de l'hormone tritiée sur le récepteur.

L'affinité relative de liaison (ARL) du produit testé est déterminée par l'équation

$$ARL = 100 \frac{(CH)}{(CX)} .$$

Les résultats obtenus sont les suivants :

| Temps d'incubation à 0°C | Progestogène | | Gluco-corticoïde | |
|---|---|---|---|---|
| Produits des exemples | 2 H | 24 H | 4 H | 24 H |
| 1 | 0,1 | 0,1 | 160 | 57 |
| 4 | 0,2 | 0,2 | 125 | 128 |
| 7 | 0,4 | 0,1 | 226 | 112 |
| 10 | 0,9 | 0,5 | 218 | 152 |
| 12 | 0,1 | 0,1 | 136 | 45 |

**CONCLUSION**: Les produits des exemples 1, 4, 7, 10 et 12 présentent une affinité très marquée pour le réception glucocorticoïde ainsi qu'une activité négligeable pour le récepteur progestogène.

Des résultats obtenus, on peut conclure que les produits peuvent présenter des activités agonistes ou antagonistes des glucocorticoïdes, tout en étant dépourvus d'activité progestomimétique ou antiprogestomimétique.

II/. Activité antiglucocorticoïde:

La technique utilisée découle de la méthode décrite par Dausse et Coll dans Molecular Pharmacology 13, 948 - 955 (1977) ("the relationship beetween glucocorticoïd structure and effects upon Thymocytes"), pour des thymocytes de souris.

Des thymocytes de rats surrénalectomisés sont incubés à 37°C pendant 3 heures, dans un milieu nutritif renfermant $5.10^{-8}$M de dexaméthasone en présence ou non d'un produit à étudier à différentes concentrations. On ajoute l'uridine tritiée, et poursuit l'incubation pendant 1 heure. On refroidit les incubats, les traite avec une solution d'acide trichloroacétique à 5 %, les filtre sur papier Whatman GF/A, les lave trois fois à l'aide d'une solution d'acide trichloroacétique à 5 %, les filtre sur papier Whatman GF/A, les lave trois fois à l'aide d'une solution d'acide trichloroacétique à 5 %. On détermine la radioactivité retenue par le filtre.

Les glucocorticoïdes et en particulier la dexaméthasone provoquent une diminution de l'incorporation d'uridine tritiée. Les produits des exemples 1, 4, 7, 10 et 12 s'opposent à cet effect.

| Produit de l'exemple | $5.10^{-8}$ Dexaméthasone + produit à tester à la concentration de | % d'inhibition de l'effet de la Dexaméthasone |
|---|---|---|
| 1 | $10^{-8}$ M | 20 |
| | $10^{-7}$ M | 55 |
| | $10^{-6}$ M | 80 |
| 4 | $10^{-8}$ M | 10 |
| | $10^{-7}$ M | 30 |
| | $10^{-6}$ M | 83 |
| 7 | $10^{-8}$ M | 10 |
| | $10^{-7}$ M | 16 |
| | $10^{-6}$ M | 65 |
| 12 | $10^{-8}$ M | 6 |
| | $10^{-7}$ M | 12 |
| | $10^{-6}$ M | 67 |

**CONCLUSION** :

Les produits étudiés présentent une activité anti-glucocorticoïde très marquée tout en étant dépourvus, le plus souvent, d'activité glucocorticoïde.

Compositions pharmaceutiques:

On a préparé des comprimés répondant à la formule suivante:

– Produit de l'exemple 1 ................................................ 50 mg
Excipient (talc, amidon, stéarate de magnésium)
q.s. pour un comprimé terminé à .................................... 120 mg

**Revendications pour les Etats Contractants: BE, CH, DE, FR, GB, IT, LI, LU, NL, SE**

1. Les produits de formule générale I:

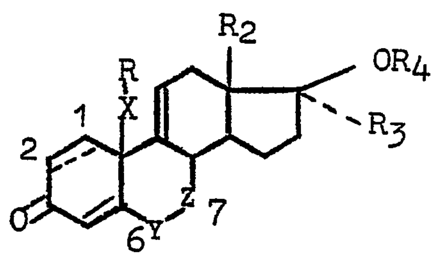

(I)

dans laquelle X et R sont tels que:

– soit X représente un radical méthylène et R représente ou bien un radical aryle carbocyclique ou hétérocyclique choisi parmi les radicaux phényle ou naphtyle, furyle, thiényle, pyrolyle, oxazolyle, thiazolyle, imidazolyle, pyrazolyle, thiadiazolyle, triazolyle, oxadiazolyle ou tétrazolyle, pyridinyle, pyrazinyle ou pyrimidinyle, chacun de ces radicaux étant éventuellement substitué par un ou plusieurs substituants choisis parmi les radicaux alkyle ayant de 1 à 4 atomes de carbone, alkoxy ayant de 1 à 4 atomes de carbone, alkylthio ayant de 1 à 4 atomes de carbone, alkényle choisis parmi vinyle ou allyle, alkynyle choisis parmi éthynyle, propargyle ou prop-1-ynyle, halogènes, trifluorométhyle, amino, méthylamino ou diaméthylamino, amino protégé choisi parmi les radicaux tritylamino ou bis triméthylsilylamino, hydroxyle, mercapto, carboxyle, méthoxycarbonyle, éthoxycarbonyle ou tert-butoxy carbonyle, carboxyle salifié, carbamoyle, nitro, aminoalkyle, diméthylaminoéthyle, carboxyméthyle ou hydroxyméthyle ou bien un radical vinyle ou éthynyle éventuellement substitué, par un ou plusieurs substituants choisis parmi les radicaux alkyle ayant de 1 à 4 atomes de carbone, alkoxy ayant de 1 à 4 atomes de carbone, alkylthio ayant de 1 à 4 atomes de carbone, alkényle choisis parmi vinyle ou allyle, alkynyle

choisis parmi éthynyle, propargyle ou prop-1-ynyle, halogènes, trifluorométhyle, amino, méthylamino ou diméthylamino, amino protégé choisi parmi les radicaux tritylamino ou bis triméthylsilylamino, hydroxyle, mercapto, carboxyle, méthoxycarbonyle, éthoxycarbonyle ou tert-butoxy carbonyle, carboxyle salifié; carbamoyle, nitro, aminoalkyle, diméthylaminoéthyle, carboxyméthyle ou hydroxyméthyle et les radicaux aryles carbocycliques ou hétérocycliques choisis parmi les radicaux phényle ou naphtyle, furyle, thiényle, pyrolyle, oxazolyle, thiazolyle, imidazolyle, pyrazolyle, thiadiazolyle, triazolyle, oxadiazolyle ou tétrazolyle, pyridinyle, pyrazinyle ou pyrimidinyle

– <u>soit</u> X représente un atome de soufre ou une simple liaison et R représente un radical aryle carbocyclique ou hétérocyclique choisi parmi les radicaux phényle ou naphtyle, furyle, thiényle, pyrolyle, oxazolyle, thiazolyle, imidazolyle, pyrazolyle, thiadiazolyle, triazolyle, oxadiazolyle ou tétrazolyle, pyridinyle, pyrazinyle ou pyrimidinyle, chacun de ces radicaux étant éventuellement substitué par un ou plusieurs substituants choisis parmi les radicaux alkyle ayant de 1 à 4 atomes de carbone, alkoxy ayant de 1 à 4 atomes de carbone, alkylthio ayant de 1 à 4 atomes de carbone, alkényle choisis parmi vinyle ou allyle, alkynyle choisis parmi éthynyle, propargyle ou prop-1-ynyle, halogènes, trifluorométhyle, amino, méthylamino ou diméthylamino, amino protégé choisi parmi les radicaux tritylamino ou bis triméthylsilylamino, hydroxyle, mercapto, carboxyle, méthoxycarbonyle, éthoxycarbonyle ou tert-butoxy carbonyle, carboxyle salifié; carbamoyle, nitro, aminoalkyle, monoalkylaminoalkyle, diméthylaminoéthyle, carboxyméthyle ou hydroxyméthyle

$R_2$ représente un radical méthyle ou éthyle,

$R_3$ représente un radical alkyle, alkényle ou alkynyle ayant au plus 8 atomes de carbone ou un radical hydroxypropynyle ou chloréthynyle,

$R_4$ représente un atome d'hydrogène ou un radical acyle, choisi parmi les radicaux acétyle, propionyle ou benzoyle représente:

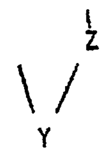

ou bien un groupement

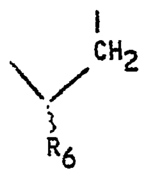

dans lequel $R_6$ représente un atome d'hydrogène ou un radical méthyle en position α et/ou β,
ou bien un groupement

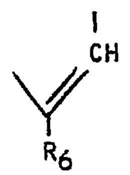

dans lequel $R_6$ représente un atome d'hydrogène ou un radical méthyle,
ou bien un groupement

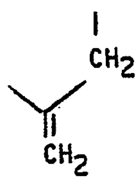

le trait pointillé en position 1(2) indique la présence éventuelle d'une seconde liaison carbone-carbone.

2. Les produits de formule générale I telle que définie à la revendication 1, caractérisée en ce que le radical aryle carbocyclique ou hétérocyclique éventuellement substitué que peut représenter R signifie <u>ou bien</u> un radical phényle éventuellement substitué par un radical alkyle ou alkoxy ayant de 1 à 4 atomes de carbone, par un atome d'halogène ou par un radical amino ou dialkylamino, <u>ou bien</u> un radical pyridyle.

3. Les produits de formule générale I telle que définie à la revendication 1, caractérisée en ce que le radical vinyle ou éthynyle éventuellement substitué que peut représenter R signifie ou bien un radical vinyle ou méthylvinyle de formule:

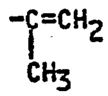

ou bien un radical éthynyle éventuellement substitué par un radical alkyle ayant de 1 à 4 atomes de carbone, par un radical phényle, par un radical carboxy éventuellement estérifié, par un radical hydroxyméthyle ou par un radical amino, tritylamino, bis-triméthylsilylamino, méthylamino ou diméthylamino.

4. Les produits de formule générale I dont les noms suivent:

– la 10β-benzyl 17β-hydroxy 17α-(prop-1-ynyl) estra 4,9(11)-dièn-3-one;
– la 17β-hydroxy 10β-/(4-méthylphényl) méthyl/17α-(prop-1-ynyl) estra 4,9(11)-dièn-3-one;
– la 10β-(2-méthylprop-2-én-1-yl) 17α-(prop-1-ynyl) 17β-hydroxy estra 4,9(11)-dièn-3-one;
– la 10β-/(4-méthoxyphényl) thio/17α-(prop-1-ynyl) 17β-hydroxy estra 4,9(11)-dièn-3-one;
– la 10β-/(2-fluorophényl) méthyl/17α-(prop-1-ynyl) 17β-hydroxy estra 4,9(11)-dièn-3-one;

5. Procédé de préparation des produits de formule I telle que définie à la revendication 1, caractérisé en ce que l'on traite un produit de formule II:

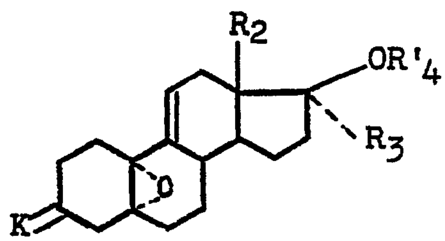

(II)

dans laquelle $R_2$ et $R_3$ ont la signification indiquée à la revendication 1, K représente un groupement protecteur de la fonction cétone et $R'_4$ représente un atome d'hdrogène ou un groupement protecteur du radical hydroxyle,

– soit par un lithien de formule R–X–Li,
– soit par un magnésien de formule R–X'–Mg–Hal, formules dans lesquelles

R et X ont les significations indiquées à la revendication 1,

X' représente un radical méthylène ou une simple liaison et Hal représente un atome d'halogène, pour obtenir un produit de formule (III):

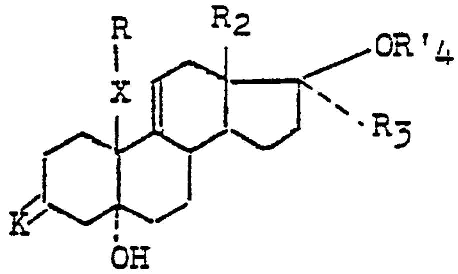

(III)

que l'on traite par un acide pour obtenir les produits de formule (IV):

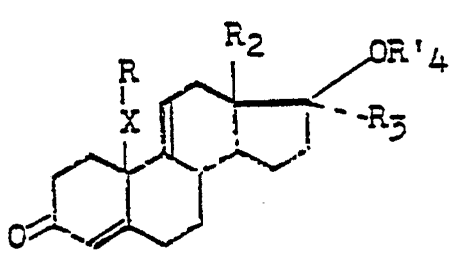

(IV)

que, si nécessaire, l'on traite par un réactif de clivage du groupement $R'_4$ lorsque celui-ci représente un groupement protecteur du radical hydroxyle différent d'un radical acyle, pour obtenir un produit de formule ($I_A$):

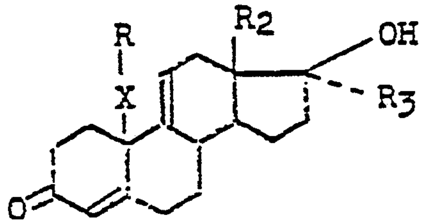

$(I_A)$

et que si désiré l'on traite un produit de formule (IV) par un orthoformiate d'alkyle, puis par un réactif de déshydrogénation et enfin, si nécessaire, par un réactif de clivage du groupement R'4 lorsque celui-ci représente un groupement protecteur du radical hydroxyle différent d'un radical acyle, pour obtenir un produit de formule ($I_B$):

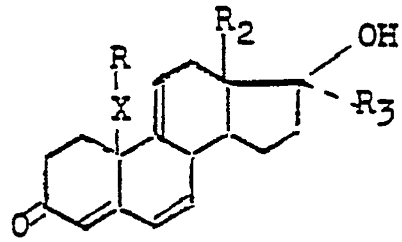

$(I_B)$

et que si désiré l'on traite un produit de formule (IV) par un réactif susceptible d'introduire un radical méthylène, en position 6, pour obtenir un produit de formule (V):

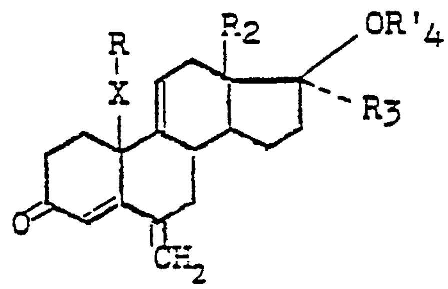

(V)

que, si nécessaire, l'on traite par un réactif de clivage du groupement R'4 lorsque celui-ci représente un groupement protecteur du radical hydroxyle différent d'un radical acyle, pour obtenir un produit de formule ($I_C$):

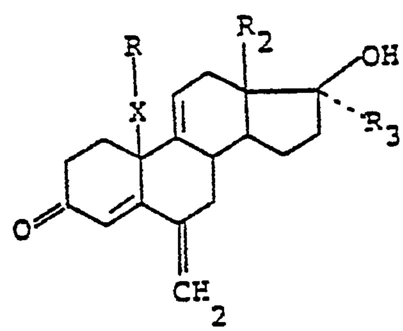

$(I_C)$

ou produit de formule (V) que:

– <u>soit</u> l'on traite par un réactif d'hydrogénation et éventuellement d'épimérisation, puis par un réactif de clivage du groupement R'4, lorsque celui-ci représente un groupement protecteur du radical hydroxyle différent d'un radical acyle, pour obtenir un produit de formule ($I_D$):

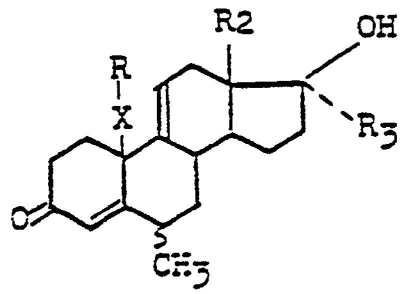

($I_D$)

– soit l'on traite par un agent d'isomérisation pour obtenir après traitement par un réactif de clivage du groupement $R'_4$, lorsque celui-ci représente un groupement protecteur du radical hydroxyle un produit de formule ($I_E$):

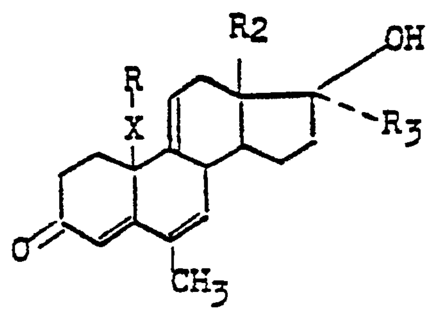

($I_E$)

et que si désiré l'on traite les produits de formules ($I_A$), ($I_B$), ($I_C$), ($I_D$) et ($I_E$) précédentes par un réactif de déshydrogénation ou par un microorganisme susceptible de déshydrogéner, pour obtenir les produits correspondants comportant une insaturation en position 1(2) et produits de formules ($I_A$), ($I_B$), ($I_C$), ($I_D$) et ($I_E$) et les produits correspondants comportant une insaturation en position 1(2) que si désiré l'on traite par un réactif d'acylation.

6. Procédé de préparation des produits de formule générale (I) telle que définie ci-dessus caractérisé en ce que l'on traite un produit de formule (VI):

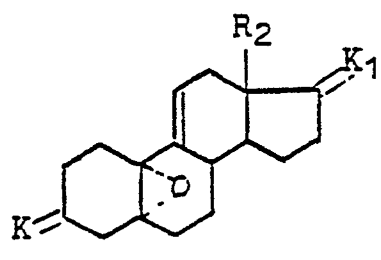

(VI)

dans laquelle $R_2$ a la signification indiquée à la revendication 1, K représente un groupement protecteur de la fonction cétone et $K_1$ représente une fonction cétone éventuellement protégée:

– soit par un lithien de formule R–X–Li,

– soit par un magnésien de formule R–X'–Mg–Hal, formulés dans lesquelles R et X ont les significations indiquées à la revendication 1,

X' représente un radical méthylène ou une simple liaison et Hal représente un atome d'halogène pour obtenir un produit de formule (VII):

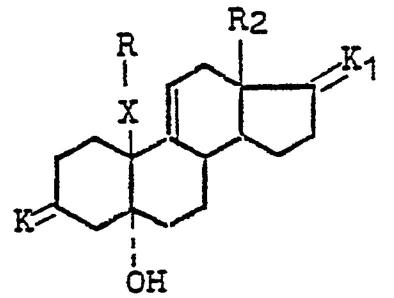

(VII)

produit que l'on traite:

– soit par un réactif de clivage du radical $K_1$ lorsque celui-ci représente un groupement protecteur du radical céto en position 17, puis par un magnésien de formule $R_3$–Mg–$Hal_1$ ou par un lithien de formule $R_3$–Li, formules dans lesquelles $R_3$ a la signification indiquée à la revendication 1, et $Hal_1$ représente un atome d'halogène, pour obtenir un produit de formule ($III_A$):

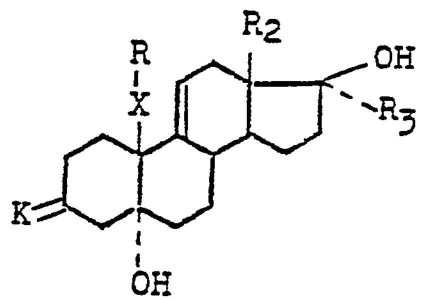

($III_A$)

que l'on traite par un acide pour obtenir un produit de formule ($I_A$) telle que définie à la revendication 5, produit que l'on soumet éventuellement à une réaction de protection du radical hydroxyle en position 17β et traite le produit de formules ($I_A$) ou (IV) ainsi obtenus selon le procédé décrit à la revendication 5, pour obtenir les produits de formules ($I_B$), ($I_C$), ($I_D$) et ($I_E$) et les produits insaturés en 1(2) correspondants,

– soit l'on traite le produit de formule (VII) par un acide pour obtenir un produit de formule (VIII):

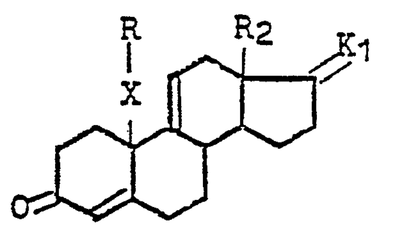

(VIII)

produit de formule (VIII) que l'on traite, ou bien par un orthoformiate d'alkyle, puis par un réactif de déshydrogénation, pour obtenir un produit de formule (IX):

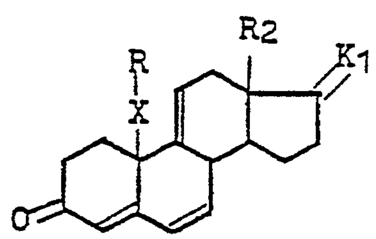

(IX)

ou bien par un réactif susceptible d'introduire un radical méthylène, en position 6, pour obtenir un produit de formule (X):

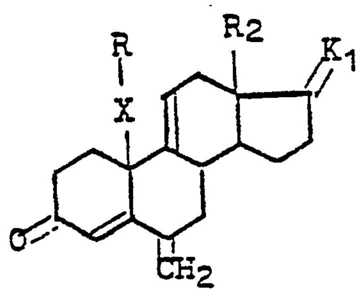

(X)

et produit de formule X que l'on traite ou par un réactif d'hydrogénation et éventuellement d'épimérisation, pour obtenir un produit de formule (XI):

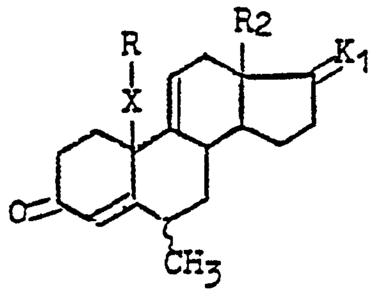

(XI)

ou par un agent d'isomérisation, pour obtenir un produit de formule (XII):

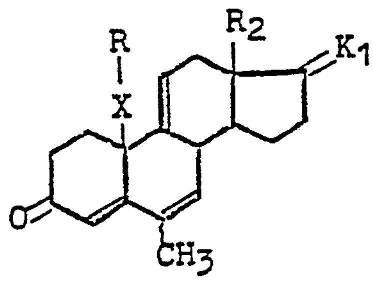

(XII)

et produits de formules (VIII), (IX), (X), (XI) et (XII) que l'on traite, éventuellement, par un réactif de clivage du groupement $K_1$ lorsque celui-ci représente un groupement protecteur de la fonction cétone et par un agent de blocage de la fonction cétone en position 3, puis par un magnésien de formule $R_3$–Mg–$Hal_1$ dans laquelle $R_3$ a la signification indiquée à la revendication 1 et $Hal_1$ représente un atome d'halogène pour obtenir respectivement, après déblocage de la fonction cétone en position 3, les produits de formules ($I_A$), ($I_B$), ($I_C$), ($I_D$) et ($I_E$) tels que définis à la revendication 5, que l'on peut transformer selon le procédé de la revendication 5, en produits insaturés en position 1(2) correspondants et que l'on peut acyler.

7. A titre de médicaments, les produits de formule générale (I) tels que définis à l'une des revendications 1 à 3.

8. A titre de médicaments, les produits de formule générale (I) tels que définis à la revendication 4.

9. Les compositions pharmaceutiques renfermant, comme principe actif, au moins un médicament défini à l'une quelconque des revendications 7 ou 8.

10. A titre de produits industriels nouveaux, les produits de formule générale (A):

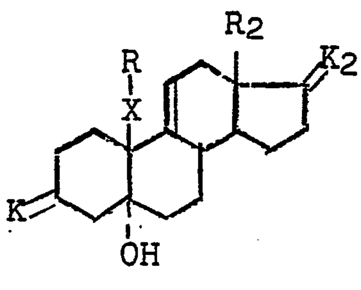

(A)

dans laquelle X, R et $R_2$ ont la signification indiquée à la revendication 1, K représente un groupement protecteur de la fonction cétone choisi parmi les groupements cétal, thiocétal, oxime ou alkyloxime et $K_2$ représente:

– soit une fonction cétone éventuellement protégée par un groupement cétal, thiocétal, oxime ou alkyloxime,

– soit un radical

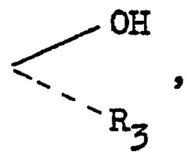

dans lequel $R_3$ a la signification indiquée à revendication 1 et dans lequel l'hydroxyle est éventuellement protégé par un groupement choisi parmi les groupements tétrahydropyrannyle, tert-butyle, acétyle, chloroacétyle ou trifluoroacétyle.

**Revendications pour l'Etat Contractant: AT**

1. Procédé de préparation des produits de formule générale (I):

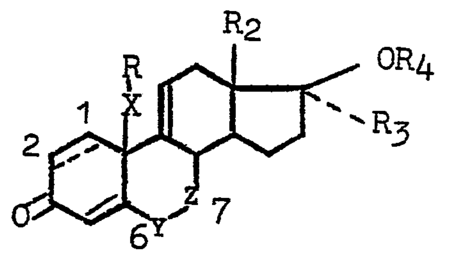

(I)

dans laquelle X et R sont tels que:

– soit X représente un radical méthylène et R représente ou bien un radical aryle carbocyclique ou hétérocyclique choisi parmi les radicaux phényle ou naphtyle, furyle, thiényle, pyrolyle, oxazolyle, thiazolyle, imidazolyle, pyrazolyle, thiadiazolyle, triazolyle, oxadiazolyle ou tétrazolyle, pyridinyle, pyrazinyle ou pyrimidinyle, chacun de ces radicaux étant éventuellement substitué par un ou plusieurs substituants choisis parmi les radicaux alkyle ayant de 1 à 4 atomes de carbone, alkoxy ayant de 1 à 4 atomes de carbone, alkylthio ayant de 1 à 4 atomes de carbone, alkényle choisis parmi vinyle ou allyle, alkynyle choisis parmi éthynyle, propargyle ou prop-1-ynyle, halogènes, trifluorométhyle, amino, méthylamino ou diaméthylamino, amino protégé choisi parmi les radicaux tritylamino ou bis triméthylsilylamino, hydroxyle, mercapto, carboxyle, méthoxycarbonyle, éthoxycarbonyle ou tert-butoxy carbonyle, carboxyle salifié, carbamoyle, nitro, aminoalkyle, diméthylaminoéthyle, carboxyméthyle ou hydroxyméthyle ou bien un radical vinyle ou éthynyle éventuellement substitué, par un ou plusieurs substituants choisis parmi les radicaux alkyle ayant de 1 à 4 atomes de carbone, alkoxy ayant de 1 à 4 atomes de carbone, alkylthio ayant de 1 à 4 atomes de carbone, alkényle choisis parmi vinyle ou allyle, alkynyle choisis parmi éthynyle, propargyle ou prop-1-ynyle, halogènes, trifluorométhyle, amino, méthylamino ou diméthylamino, amino protégé choisi parmi les radicaux tritylamino ou bis triméthylsilylamino, hydroxyle, mercapto, carboxyle, méthoxycarbonyle, éthoxycarbonyle ou tert-butoxy carbonyle, carboxyle salifié; carbamoyle, nitro, aminoalkyle, diméthylaminoéthyle, carboxyméthyle ou hydroxyméthyle et les radicaux aryles carbocycliques ou hétérocycliques choisis parmi les radicaux phényle ou naphtyle, furyle, thiényle, pyrolyle, oxazolyle, thiazolyle, imidazolyle, pyrazolyle, thiadiazolyle, triazolyle, oxadiazolyle ou tétrazolyle, pyridinyle, pyrazinyle ou pyrimidinyle

– soit X représente un atome de soufre ou une simple liaison et R représente un radical aryle carbocyclique ou hétérocyclique choisi parmi les radicaux phényle ou naphtyle, furyle, thiényle, pyrolyle, oxazolyle, thiazolyle, imidazolyle, pyrazolyle, thiadiazolyle, triazolyle, oxadiazolyle ou tétrazolyle, pyridinyle, pyrazinyle ou pyrimidinyle, chacun de ces radicaux étant éventuellement substitué par un ou plusieurs substituants choisis parmi les radicaux alkyle ayant de 1 à 4 atomes de carbone, alkoxy ayant de 1 à 4 atomes de carbone, alkylthio ayant de 1 à 4 atomes de carbone, alkényle choisis parmi vinyle ou allyle, alkynyle choisis parmi éthynyle, propargyle ou prop-1-ynyle, halogènes, trifluorométhyle, amino, méthylamino ou diméthylamino, amino protégé choisi parmi les radicaux tritylamino ou bis triméthylsilylamino, hydroxyle, mercapto, carboxyle, méthoxycarbonyle, éthoxycarbonyle ou tert-butoxy carbonyle, carboxyle salifié; carbamoyle, nitro, aminoalkyle, monoalkylaminoalkyle, diméthylaminoéthyle, carboxyméthyle ou hydroxyméthyle

$R_2$ représente un radical méthyle ou éthyle,

$R_3$ représente un radical alkyle, alkényle ou alkynyle ayant au plus 8 atomes de carbone ou un radical hydroxypropynyle ou chloréthynyle,
$R_4$ représente un atome d'hydrogène ou un radical acyle, choisi parmi les radicaux acétyle, propionyle ou benzoyle

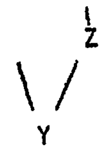

représente:
ou bien un groupement

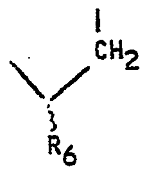

dans lequel $R_6$ représente un atome d'hydrogène ou un radical méthyle en position α et/ou β,
ou bien un groupement

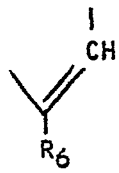

dans lequel $R_6$ représente un atome d'hydrogène ou un radical méthyle,
ou bien un groupement

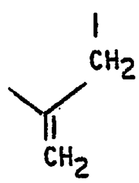

le trait pointillé en position 1(2) indique la présence éventuelle d'une seconde liaison carbone–carbone caractérisé en ce que l'on traite un produit de formule (II):

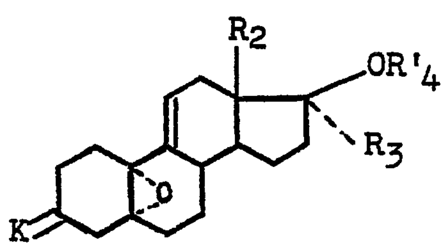

(II)

dans laquelle $R_2$ et $R_3$ ont la signification indiquée précédemment,
K représente un groupement protecteur de la fonction cétone et $R'_4$ représente un atome d'hydrogène ou un groupement protecteur du radical hydroxyle,
– soit par un lithien de formule R–X–Li,
– soit par un magnésien de formule R–X′–Mg–Hal, formules dans lesquelles
R et X ont les significations indiquées précédemment,
X′ représente un radical méthylène ou une simple liaison et Hal représente un atome d'halogène, pour obtenir un produit de formule (III):

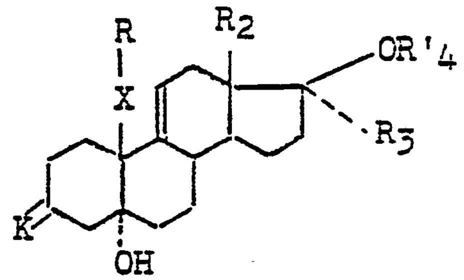

(III)

que l'on traite par un acide pour obtenir les produits de formule (IV):

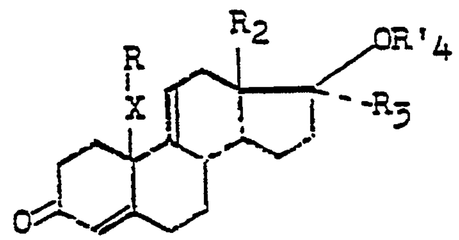

(IV)

que, si nécessaire, l'on traite par un réactif de clivage du groupement R'4 lorsque celui-ci représente un groupement protecteur du radical hydroxyle différent d'un radical acyle, pour obtenir un produit de formule ($I_A$):

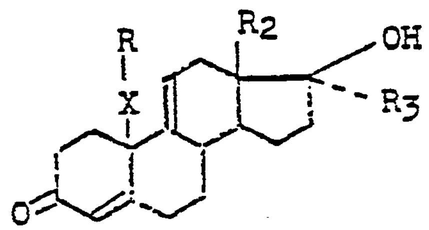

($I_A$)

et que si désiré l'on traite un produit de formule (IV) par un orthoformiate d'alkyle, pouis par un réactif de déshydrogénation et enfin, si nécessaire, par un réactif de clivage du groupement R'4 lorsque celui-ci représente un groupement protecteur du radical hydroxyle différent d'un radical acyle, pour obtenir un produit de formule ($I_B$):

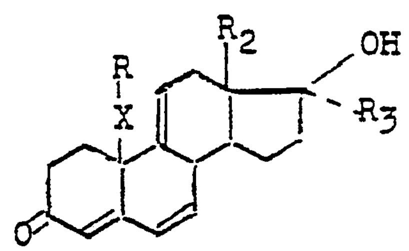

($I_B$)

et que si désiré l'on traite un produit de formule (IV) par un réactif susceptible d'introduire un radical méthylène, en position 6, pour obtenir un produit de formule (V):

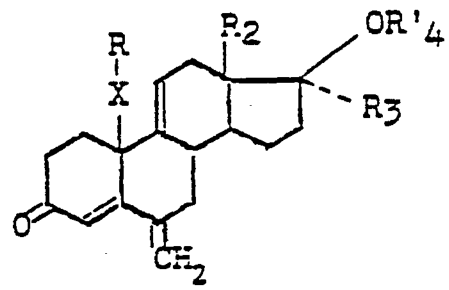

(V)

que, si nécessaire, l'on traite par un réactif de clivage du groupement R'$_4$ lorsque celui-ci représente un groupement protecteur du radical hydroxyle différent d'un radical acyle, pour obtenir un produit de formule ($I_C$):

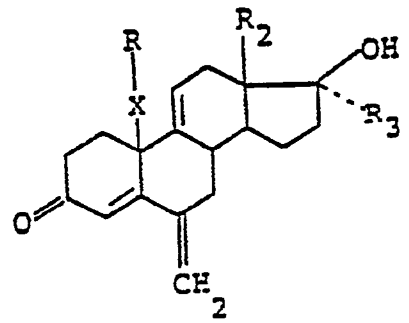

($I_C$)

ou produit de formule (V) que:
– soit l'on traite par un réactif d'hydrogénation et éventuellement d'épimérisation, puis par un réactif de clivage du groupement R'$_4$ lorsque celui-ci représente un groupement protecteur du radical hydroxyle différent d'un radical acyle, pour obtenir un produit de formule ($I_D$):

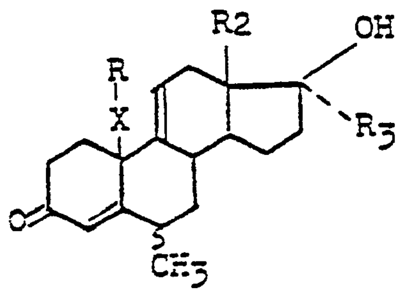

($I_D$)

– soit l'on traite par un agent d'isomérisation pour obtenir après traitement par un réactif de clivage du groupement R'$_4$ lorsque celui-ci représente un groupement protecteur du radical hydroxyle différent d'un radical acyle, pour obtenir un produit de formule ($I_E$):

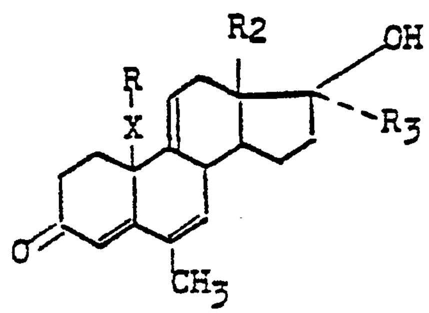

($I_E$)

et que si désiré l'on traite les produits de formules ($I_A$), ($I_B$), ($I_C$), ($I_D$) et ($I_E$) précédentes par un réactif de déshydrogénation ou par un microorganisme susceptible de déshydrogéner, pour obtenir les produits correspondants comportant une insaturation en position 1(2) et produits de formules ($I_A$), ($I_B$),

($I_C$), ($I_D$) et ($I_E$) et les produits correspondants comportant une insaturation en position 1(2) que si désiré l'on traite par un réactif d'acylation.

2. Procédé selon la revendication 1, caractérisé en ce que l'on traite un produit de formule (II):

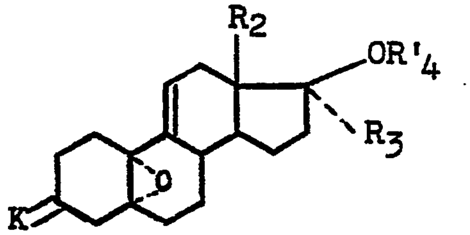

(II)

dans laquelle $R_2$ et $R_3$ ont la signification indiquée précédemment, K représente un groupement protecteur de la fonction cétone et $R'_4$ représente un atome d'hydrogène ou un groupement protecteur du radical hydroxyle,

– soit par un lithien de formule R–X–Li,

– soit par un magnésien de formule R–X'–Mg–Hal, formules dans lesquelles

R et X ont les significations indiquées précédemment,

X' représente un radical méthylène ou une simple liaison et Hal représente un atome d'halogène, pour obtenir un produit de formule (III):

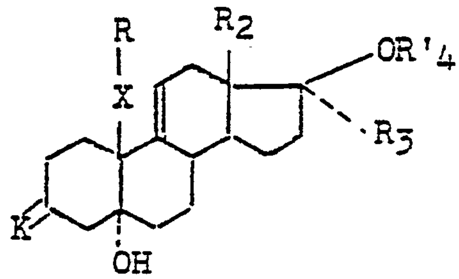

(III)

que l'on traite par un acide pour obtenir les produits de formule (IV):

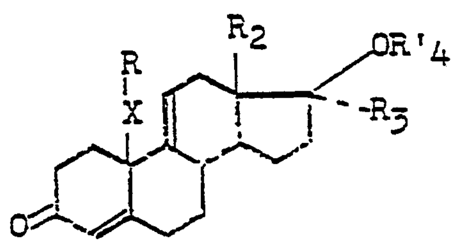

(IV)

que, si nécessaire, l'on traite par un réactif de clivage du groupement $R'_4$ lorsque celui-ci représente un groupement protecteur du radical hydroxyle différent d'un radical acyle, pour obtenir un produit de formule ($I_A$):

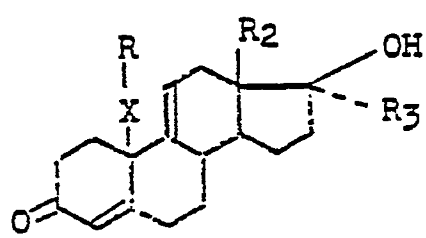

($I_A$)

et que si désiré l'on traite un produit de formule (IV) par un orthoformiate d'alkyle, puis par un réactif de déshydrogénation et enfin, si nécessaire, par un réactif de clivage du groupement $R'_4$ lorsque celui-ci représente un groupement protecteur du radical hydroxyle différent d'un radical acyle, pour obtenir un produit de formule ($I_B$):

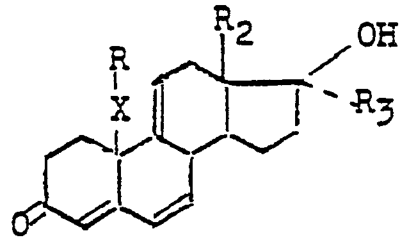

$(I_B)$

et que si désiré l'on traite un produit de formule (IV) par un réactif susceptible d'introduire un radical méthylène, en position 6, pour obtenir un produit de formule (V):

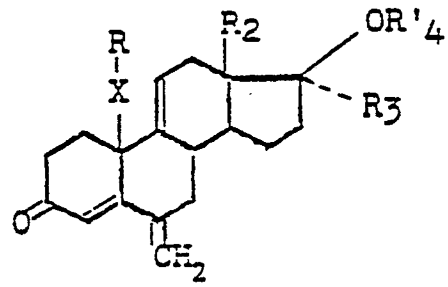

(V)

lequel correspond, lorsque R'4 représente un atome d'hydrogène, à un produit de formule ($I_C$):

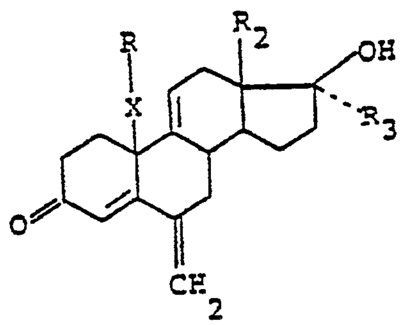

$(I_C)$

ou produit de formule (V) que:

– soit l'on traite par un réactif d'hydrogénation et éventuellement d'épimérisation, puis par un réactif de clivage du groupement R'4 lorsque celui-ci représente un groupement protecteur du radical hydroxyle différent d'un radical acyle, pour obtenir un produit de formule ($I_D$):

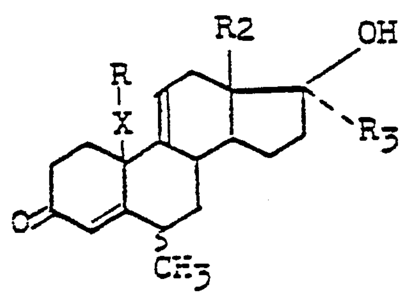

$(I_D)$

– soit l'on traite par un agent d'isomérisation pour obtenir après traitement par un réactif de clivage du groupement R'4 lorsque celui-ci représente un groupement protecteur du radical hydroxyle un produit de formule ($I_E$):

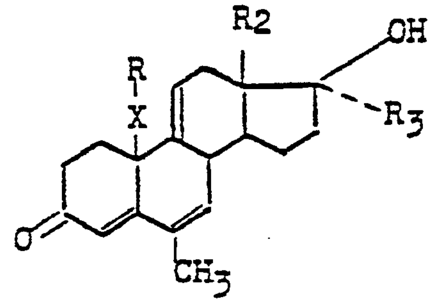

$(I_E)$

et que si désiré l'on traite les produits de formules ($I_A$), ($I_B$), ($I_C$), ($I_D$) et ($I_E$) précédentes par un réactif de déshydrogénation ou par un microorganisme susceptible de déshydrogéner, pour obtenir les produits correspondants comportant une insaturation en position 1(2) et produits de formules ($I_A$), ($I_B$), ($I_C$), ($I_D$) et ($I_E$) et les produits correspondants comportant une insaturation en position 1(2) que si désiré l'on traite par un réactif d'acylation.

3. Procédé de préparation des produits de formule générale (I) telle que définie ci-dessus caractérisé en ce que l'on traite un produit de formule (VI):

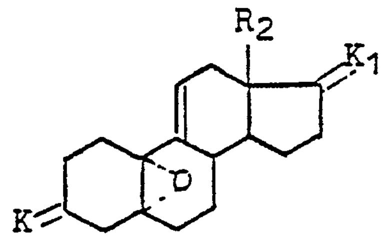

(VI)

dans laquelle $R_2$ a la signification indiquée à la revendication 1, K représente un groupement protecteur de la fonction cétone et $K_1$ représente une fonction cétone éventuellement protégée:

– <u>soit</u> par un lithien de formule R–X–Li,

– <u>soit</u> par un magnésien de formule R–X'–Mg–Hal, formules dans lesquelles

R et X ont les significations indiquées à la revendication 1,

X' représente un radical méthylène ou une simple liaison et Hal représente un atome d'halogène, pour obtenir un produit de formule (VII):

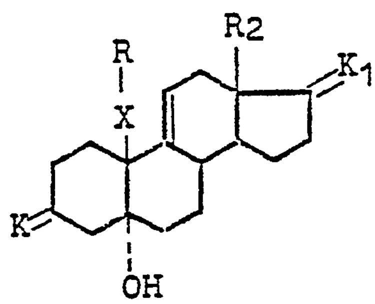

(VII)

produit que l'on traite:

– <u>soit</u> par un réactif de clivage du radical $K_1$ lorsque celui-ci représente un groupement protecteur du radical céto en position 17, puis par un magnésien de formule $R_3$–Mg–$Hal_1$ ou par un lithien de formule $R_3$–Li, formules dans lesquelles $R_3$ a la signification indiquée à la revendication 1, et $Hal_1$ représente un atome d'halogène, pour obtenir un produit de formule ($III_A$):

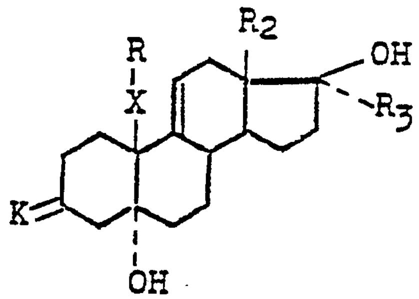

(III$_A$)

que l'on traite par un acide pour obtenir un produit de formule ($I_A$) telle que définie à la revendication 5, produit que l'on soumet éventuellement à une réaction de protection du radical hydroxyle en position 17β et traite le produit de formules ($I_A$) ou (IV) ainsi obtenus selon le procédé décrit à la revendication 5, pour obtenir les produits de formules ($I_B$), ($I_C$), ($I_D$) et ($I_E$) et les produits insaturés en 1(2) correspondants,

– soit l'on traite le produit de formule (VII) par un acide pour obtenir un produit de formule (VIII):

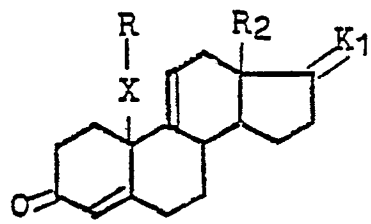

(VIII)

produit de formule (VIII) que l'on traite, ou bien par un orthoformiate d'alkyle, puis par un réactif de déshydrogénation, pour obtenir un produit de formule (IX):

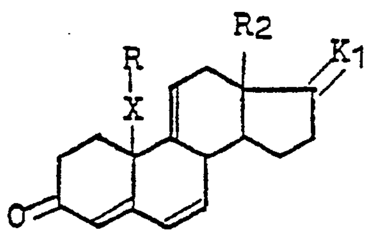

(IX)

ou bien par un réactif susceptible d'introduire un radical méthylène, en position 6, pour obtenir un produit de formule (X):

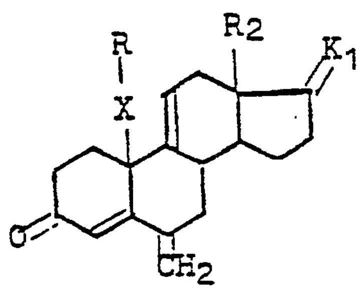

(X)

et produit de formule X que l'on traite ou par un réactif d'hydrogénation et éventuellement d'épimérisation, pour obtenir un produit de formule (XI):

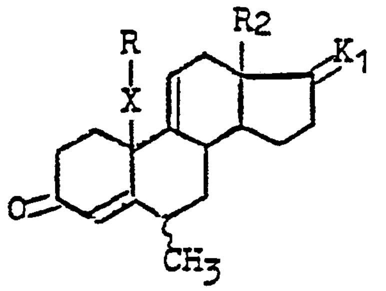

(XI)

ou par un agent d'isomérisation, pour obtenir un produit de formule (XII):

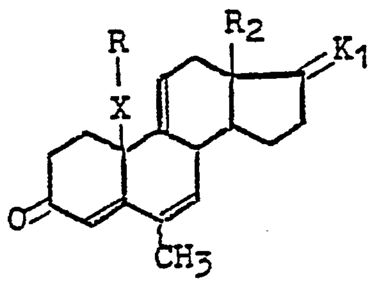

(XII)

et produits de formules (VIII), (IX), (X), (XI) et (XII) que l'on traite, éventuellement, par un réactif de clivage du groupement $K_1$ lorsque celui-ci représente un groupement protecteur de la fonction cétone et par un agent de blocage de la fonction cétone en position 3, puis par un magnésien de formule $R_3$–Mg–$Hal_1$ dans laquelle $R_3$ a la signification indiquée à la revendication 1 et $Hal_1$ représente un atome d'halogène pour obtenir respectivement, après déblocage de la fonction cétone en position 3, les produits de formules ($I_A$), ($I_B$), ($I_C$), ($I_D$) et ($I_E$) tels que définis à la revendication 1, que l'on peut transformer selon le procédé de la revendication 1, en produits insaturés en position 1(2) correspondants et que l'on peut acyler.

4. Procédé selon la revendication 2 ou 3, caractérisé en ce que l'on utilise au départ un lithien de formule R–X–Li ou un magnésien de formule R–X'–Mg–Hal, formule dans laquelle le radical aryle carbocyclique ou hétérocyclique éventuellement substitué que peut représenter R signifie ou bien un radical phényle éventuellement substitué par un radical alkyle ou alkoxy ayant de 1 à 4 atomes de carbone, par un atome d'halogène ou par un radical amino ou dialkylamino, ou bien un radical pyridyle.

5. Procédé selon la revendication 2 ou 3, caractérisé en ce que l'on utilise au départ un lithien de formule R–X–Li ou un magnésien de formule R–X'–Mg–Hal, dans laquelle le radical vinyle ou éthynyle éventuellement substitué que peut représenter R signifie ou bien un radical vinyle ou méthylvinyle de formule:

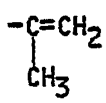

ou bien un radical éthynyle éventuellement substitué par un radical alkyle ayant de 1 à 4 atomes de carbone, par un radical phényle, par un radical carboxy éventuellement estérifié, par un radical hydroxyméthyle ou par un radical amino éventuellement protégé ou alkylé.

**Patentansprüche für den Vertragsstaat: AT**

1. Verfahren zur Herstellung der Produkte der allgemeinen Formel (I)

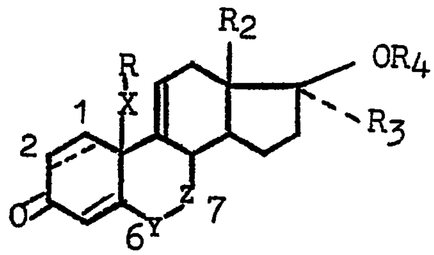

(I)

worin X und R derart sind, daß

– entweder X einen Methylenrest bedeutet und R bedeutet: entweder einen carbocyclischen oder heterocyclischen Arylrest, ausgewählt unter den Phenyl- oder Naphthyl-, Furyl-, Thienyl-, Pyrolyl-, Oxazolyl-, Thiazolyl-, Imidazolyl-, Pyrazolyl-, Thiadiazolyl-, Triazolyl-, Oxadiazolyl- oder Tetrazolyl-, Pyridinyl-, Pyrazinyl- oder Pyrimidinylresten, wobei ein jeder dieser Reste gegebenenfalls durch einen oder mehrere Substituenten substituiert ist, ausgewählt unter den Alkylresten mit 1 bis 4 Kohlenstoffatomen, Alkoxyresten mit 1 bis 4 Kohlenstoffatomen, Alkylthioresten mit 1 bis 4 Kohlenstoffatomen, Alkenylgruppen, ausgewählt unter Vinyl oder Allyl, Alkinylgruppen, ausgewählt unter Ethinyl, Propargyl oder Prop-1-inyl, den Halogenatomen, Trifluormethyl, Amino, Methylamino oder Dimethylamino, geschütztem Amino, ausgewählt unter den Tritylamino- oder Bistrimethylsilylaminoresten, Hydroxyl, Mercapto, Carboxyl, Methoxycarbonyl, Ethoxycarbonyl oder tert.-Butoxycarbonyl, in ein Salz überführtem Carboxyl, Carbamoyl, Nitro, Aminoalkyl, Dimethylaminoethyl, Carboxymethyl oder Hydroxymethyl,

oder einen Vinyl- oder Ethinylrest, gegebenenfalls substituiert durch einen oder mehrere Substituenten, ausgewählt unter den Alkylresten mit 1 bis 4 Kohlenstoffatomen, den Alkoxyresten mit 1 bis 4 Kohlenstoffatomen, den Alkylthioresten mit 1 bis 4 Kohlenstoffatomen, Alkenyl ausgewählt unter Vinyl oder Allyl, Alkinyl, ausgewählt unter Ethinyl, Propargyl oder Prop-1-inyl, den Halogenatomen, Trifluormethyl, Amino, Methylamino oder Dimethylamino, geschütztem Amino, ausgewählt unter den Tritylamino- oder Bistrimethylsilylaminoresten, Hydroxyl, Mercapto, Carboxyl, Methoxycarbonyl, Ethoxycarbonyl oder tert.-Butoxycarbonyl, in ein Salz überführtem Carboxyl; Carbamoyl, Nitro, Aminoalkyl, Dimethylaminoethyl, Carboxymethyl oder Hydroxymethyl und den carbocyclischen oder heterocyclischen Arylresten, ausgewählt unter den Phenyl- oder Naphthyl-, Furyl-, Thienyl-, Pyrolyl, Oxazolyl-, Thiazolyl-, Imidazolyl-, Pyrazolyl-, Thiadiazolyl-, Triazolyl-, Oxadiazolyl- oder Tetrazolyl-, Pyridinyl-, Pyrazinyl- oder Pyrimidinylresten,

– oder X ein Schwefelatom oder eine einfache Bindung wiedergibt und R einen carbocyclischen oder heterocyclischen Arylrest darstellt, ausgewählt unter den Phenyl- oder Naphthyl-, Furyl-, Thienyl-, Pyrolyl-, Oxazolyl-, Thiazolyl-, Imidazolyl-, Pyrazolyl-, Thiadiazolyl-, Triazolyl-, Oxadiazolyl- oder Tetrazolyl-, Pyridinyl-, Pyrazinyl- oder Pyrimidinylresten, wobei ein jeder dieser Reste gegebenenfalls substituiert ist durch einen oder mehrere Substituenten, ausgewählt unter den Alkylresten mit 1 bis 4 Kohlenstoffatomen, Alkoxyresten mit 1 bis 4 Kohlenstoffatomen, Alkylthioresten mit 1 bis 4 Kohlenstoffatomen, Alkenylresten, ausgewählt unter Vinyl oder Allyl, Alkinylresten, ausgewählt unter Ethinyl, Propargyl oder Prop-1-inyl, den Halogenatomen, Trifluormethyl, Amino, Methylamino oder Dimethylamino, geschütztem Amino, ausgewählt unter den Tritylamino- oder Bistrimethylsilylaminoresten, Hydroxyl, Mercapto, Carboxyl, Methoxycarbonyl, Ethoxycarbonyl oder tert.- Butoxycarbonyl, in ein Salz überführtem Carboxyl; Carbamoyl, Nitro, Aminoalkyl, Monoalkylaminoalkyl, Dimethylaminoethyl, Carboxymethyl oder Hydroxymethyl;

$R_2$ einen Methyl- oder Ethylrest wiedergibt;

$R_3$ für einen Alkyl-, Alkenyl- oder Alkinylrest mit höchstens 8 Kohlenstoffatomen oder einen Hydroxypropinylrest oder Chlorethinylrest steht,

$R_4$ ein Wasserstoffatom oder einen Acylrest wiedergibt, ausgewählt unter den Acetyl-, Propionyl- oder Benzoylresten,

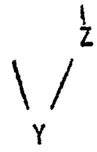

bedeutet: entweder eine Gruppe

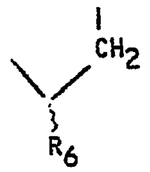

worin $R_6$ für ein Wasserstoffatom oder eine Methylgruppe in α- und/oder β-Stellung steht, oder eine Gruppe

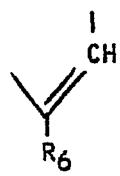

worin $R_6$ für ein Wasserstoffatom oder einen Methylrest steht, oder eine Gruppe

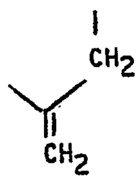

und die gestrichelte Linie die etwaige Anwesenheit einer zweiten Kohlenstoff-Kohlenstoff-Bindung anzeigt, dadurch gekennzeichnet, daß man ein Produkt der Formel (II)

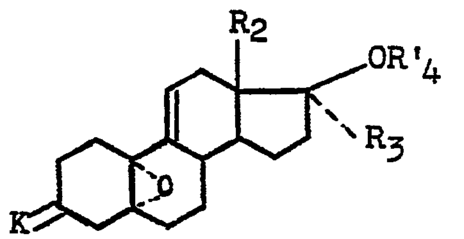

(II)

worin $R_2$ und $R_3$ die vorstehend angegebene Bedeutung besitzen, K eine Schutzgruppe für die Ketonfunktion wiedergibt und $R'_4$ ein Wasserstoffatom oder eine Schutzgruppe für den Hydroxylrest bedeutet,

– <u>entweder</u> mit einer Lithiumverbindung der Formel R–X–Li

– <u>oder</u> mit einer Magnesiumverbindung der Formel R–X'–Mg–Hal, worin R und X jeweils die vorstehend gegebene Bedeutung besitzen, X' eine Methylengruppe oder eine einfache Bindung wiedergibt und Hal ein Halogenatom bedeutet, behandelt, um zu einem Produkt der Formel (III)

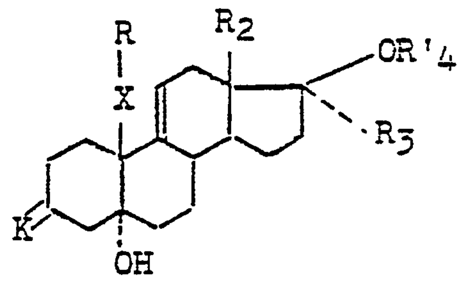

(III)

zu gelangen, das man mit einer Säure behandelt, um die Produkte der Formel IV

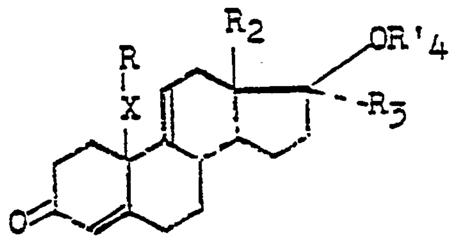

(IV)

zu erhalten, die man erforderlichenfalls mit einem Reagens zur Abspaltung der Gruppe R'$_4$ behandelt, wenn diese eine Schutzgruppe für die Hydroxylgruppe wiedergibt, die von einer Acylgruppe verschieden ist, um zu einem Produkt der Formel $I_A$

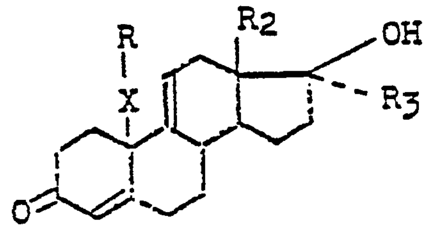

($I_A$)

zu gelangen, und gewünschtenfalls ein Produkt der Formel IV mit einem Alkylorthoformiat, anschließend mit einem Dehydrierungsreagens und schließlich erforderlichenfalls mit einem Reagens zur Abspaltung der Gruppe R'$_4$ behandelt, wenn diese eine Schutzgruppe für die Hydroxylgruppe wiedergibt, die von einer Acylgruppe verschieden ist, um zu einem Produkt der Formel $I_B$

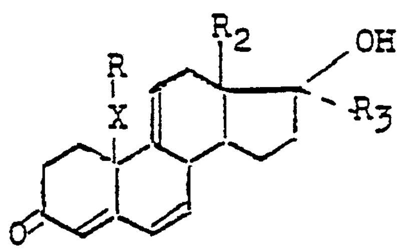

($I_B$)

zu gelangen, und gewünschtenfalls das Produkt der Formel IV mit einem Reagens behandelt, das befähigt ist, eine Methylengruppe in 6-Stellung einzuführen, um zu einem Produkt der Formel V

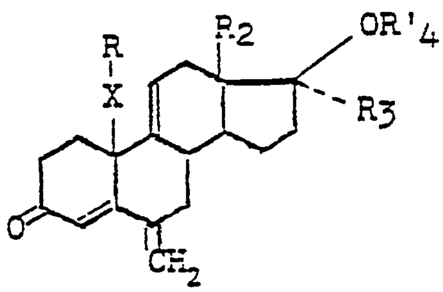

(V)

zu gelangen, das man erforderlichenfalls mit einem Reagens zur Abspaltung der Gruppe R'$_4$ behandelt, wenn diese eine Schutzgruppe für die Hydroxylgruppe wiedergibt, die von einer Acylgruppe verschieden ist, um zu einem Produkt der Formel $I_C$

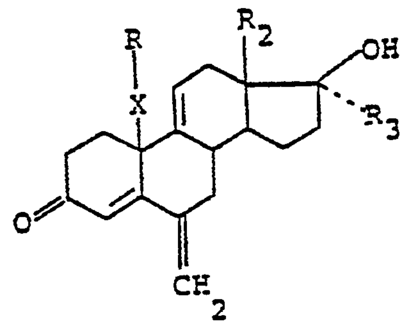

($I_C$)

oder einem Produkt der Formel V zu gelangen, das man
– entweder mit einem Hydrierungsreagens und gegebenenfalls Epimerisierungsreagens, danach mit einem Reagens zur Abspaltung der Gruppe R'₄ behandelt, wenn diese eine Schutzgruppe für die Hydroxylgruppe wiedergibt, die von einer Acylgruppe verschieden ist, um zu einem Produkt der Formel $I_D$

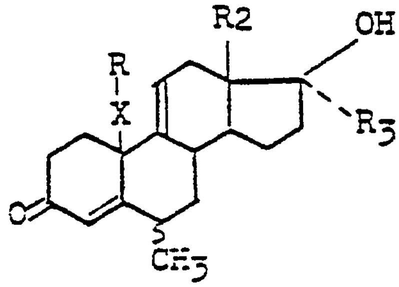

($I_D$)

zu gelangen,
– oder mit einem Isomerisierungsreagens behandelt, um nach Behandlung mit einem Reagens zur Abspaltung der Gruppe $R'_4$, wenn diese eine Schutzgruppe für den Hydroxylrest wiedergibt, ein Produkt der Formel $I_E$

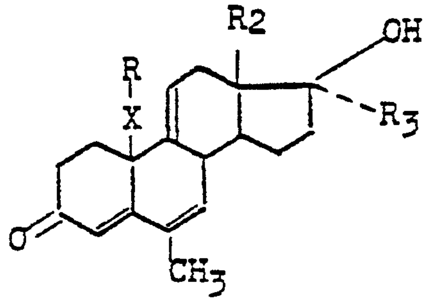

($I_E$)

zu erhalten, und gewünschtenfalls die Produkte der vorstehenden Formeln ($I_A$), ($I_B$), ($I_C$), ($I_D$) und ($I_E$) mit einem Dehydrierungsreagens oder mit einem zur Dehydrierung befähigten Mikroorganismus behandelt, um die entsprechenden Produkte mit einer Unsättigung in 1(2)-Stellung zu erhalten, und die Produkte der Formeln ($I_A$), ($I_B$), ($I_C$) und ($I_D$) sowie ($I_E$) und die entsprechenden Produkte mit einer Unsättigung in 1(2)-Stellung gewünschtenfalls mit einem Acylierungsreagens behandelt.

2. Verfahren gemäß Anspruch 1, dadurch gekennzeichnet, daß man ein Produkt der Formel (II)

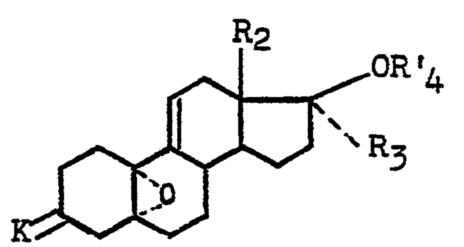

(II)

worin $R_2$ und $R_3$ die vorstehend angegebene Bedeutung besitzen, K eine Schutzgruppe für die Ketonfunktion wiedergibt und $R'_4$ ein Wasserstoffatom oder eine Schutzgruppe für den Hydroxylrest bedeutet,

– entweder mit einer Lithiumverbindung der Formel R–X–Li
– oder mit einer Magnesiumverbindung der Formel R–X'–Mg–Hal, worin R und X jeweils die vorstehend gegebene Bedeutung besitzen, X' eine Methylengruppe oder eine einfache Bindung wiedergibt und Hal ein Halogenatom bedeutet, behandelt, um zu einem Produkt der Formel III

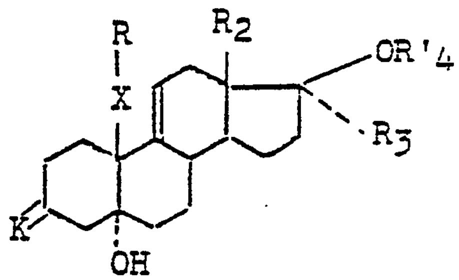

(III)

zu gelangen, das man mit einer Säure behandelt, um die Produkte der Formel IV

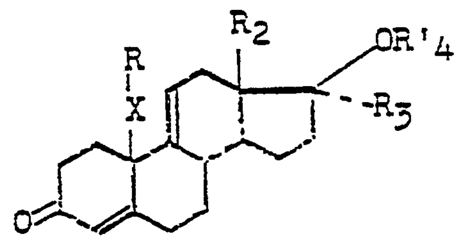

(IV)

zu erhalten, die man erforderlichenfalls mit einem Reagens zur Abspaltung der Gruppe $R'_4$ behandelt, wenn diese eine Schutzgruppe für die Hydroxylgruppe wiedergibt, die von einer Acylgruppe verschieden ist, um zu einem Produkt der Formel $I_A$

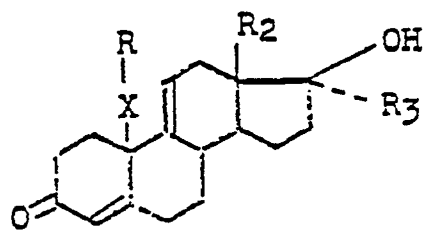

($I_A$)

zu gelangen, und gewünschtenfalls ein Produkt der Formel IV mit einem Alkylorthoformiat, anschließend mit einem Dehydrierungsreagens und schließlich erforderlichenfalls mit einem Reagens zur Abspaltung der Gruppe $R'_4$ behandelt, wenn diese eine Schutzgruppe für die Hydroxylgruppe wiedergibt, die von einem Acylrest verschieden ist, um zu einem Produkt der Formel $I_B$

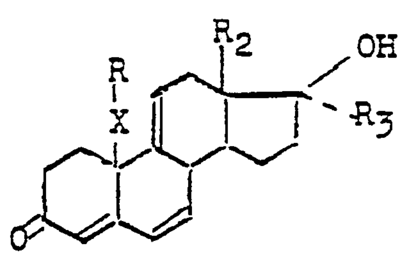

($I_B$)

zu gelangen, und gewünschtenfalls das Produkt der Formel IV mit einem Reagens behandelt, das befähigt ist, eine Methylengruppe in 6-Stellung einzuführen, um zu einem Produkt der Formel V

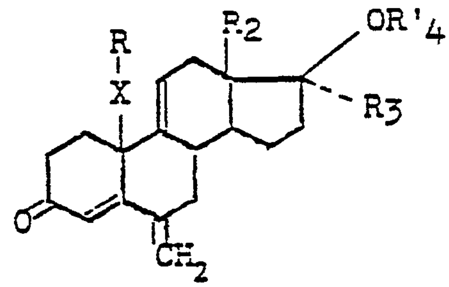

(V)

zu gelangen, das, falls $R'_4$ ein Wasserstoffatom bedeutet, einem Produkt der Formel ($I_C$) entspricht

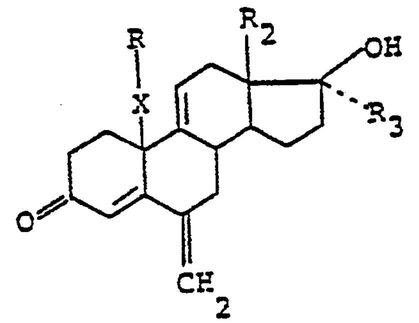

($I_C$)

oder einem Produkt der Formel V zu gelangen, das man
– entweder mit einem Hydrierungsreagens und gegebenenfalls Epimerisierungsreagens, danach mit einem Reagens zur Abspaltung der Gruppe $R'_4$ behandelt, wenn diese eine Schutzgruppe für die Hydroxylgruppe wiedergibt, die von einem Acylrest verschieden ist, um zu einem Produkt der Formel $I_D$

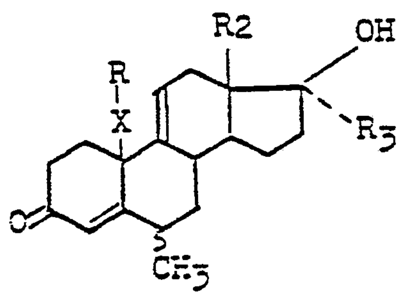

($I_D$)

zu gelangen,
– oder mit einem Isomerisierungsreagens behandelt, um nach Behandlung mit einem Reagens zur Abspaltung der Gruppe $R'_4$, wenn diese eine Schutzgruppe für den Hydroxylrest wiedergibt, ein Produkt der Formel $I_E$

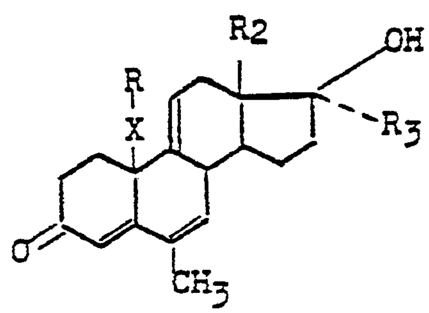

($I_E$)

zu erhalten, und gewünschtenfalls die Produkte der vorstehenden Formeln ($I_A$), ($I_B$), ($I_C$), ($I_D$) und ($I_E$) mit einem Dehydrierungsreagens oder mit einem zur Dehydrierung befähigten Mikroorganismus behandelt, um die entsprechenden Produkte mit einer Unsättigung in 1(2)-Stellung zu erhalten, und die

Produkte der Formeln ($I_A$), ($I_B$), ($I_C$) und ($I_D$) sowie ($I_E$) und die entsprechenden Produkte mit einer Unsättigung in 1(2)-Stellung gewünschtenfalls mit einem Acylierungsreagens behandelt.

3. Verfahren zur Herstellung der Produkte der allgemeinen Formel (I), wie vorstehend definiert, dadurch gekennzeichnet, daß man ein Produkt der Formel (VI)

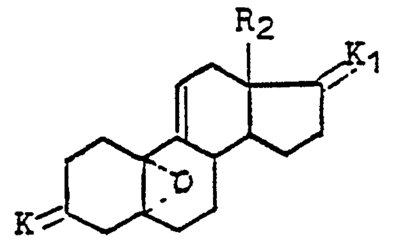
(VI)

worin $R_2$ wie in Anspruch 1 definiert ist, K eine Schutzgruppe für die Ketonfunktion wiedergibt und $K_1$ eine gegebenenfalls geschützte Ketonfunktion bedeutet,

– entweder mit einer Lithiumverbindung der Formel R–X–Li

– oder mit einer Magnesiumverbindung der Formel R–X'–Mg–Hal, worin jeweils R und X die in Anspruch 1 gegebenen Bedeutungen besitzen, X' einen Methylenrest oder eine Einfachbindung wiedergibt und Hal ein Halogenatom bedeutet, behandelt, um zu einem Produkt der Formel (VII)

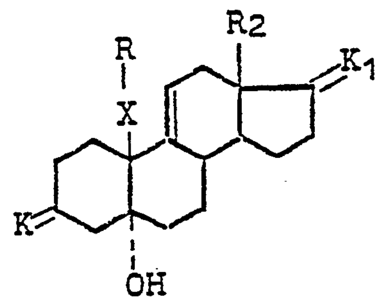
(VII)

zu gelangen, das man behandelt

– entweder mit einem Reagens zur Abspaltung des Restes $K_1$, wenn dieser eine Schutzgruppe für den Ketorest in 17-Stellung wiedergibt, hiernach mit einer Magnesiumverbindung $R_3$–Mg–$Hal_1$ oder mti einer Lithiumverbindung der Formel $R_3$–Li, worin jeweils $R_3$ wie in Anspruch 1 definiert ist und $Hal_1$ ein Halogenatom bedeutet, um zu einem Produkt der Formel ($III_A$)

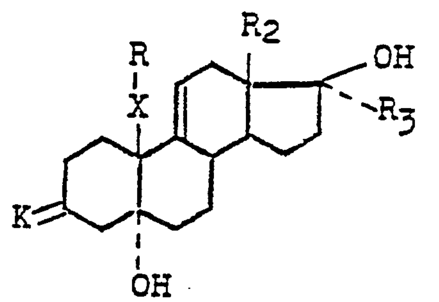
($III_A$)

zu gelangen, das man mit einer Säure behandelt, um ein Produkt der Formel ($I_A$), wie in Anspruch 1 definiert, zu erhalten, welches man gegebenenfalls einer Reaktion zum Schutz der Hydroxylgruppe in 17β-Stellung unterzieht, und das so erhaltene Produkt der Formeln ($I_A$) oder (IV) nach dem in Anspruch 1 beschriebenen Verfahren behandelt, um zu den Produkten der Formeln ($I_B$), ($I_C$), ($I_D$) und ($I_E$) und den entsprechenden, in 1(2)-Stellung ungesättigten Produkten zu gelangen;

– oder das Produkt der Formel (VII) mit einer Säure behandelt, um zu einem Produkt der Formel (VIII)

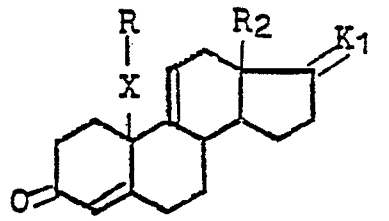

(VIII)

zu gelangen, welches Produkt der Formel (VIII) man
entweder mit einem Alkylorthoformiat, hiernach mit einem Dehydrierungsreagens behandelt, um zu einem Produkt der Formel (IX)

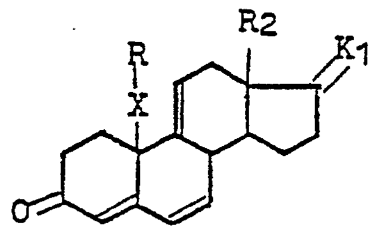

(IX)

zu gelangen,
oder mit einem Reagens behandelt, das befähigt ist, einen Methylrenrest in 6-Stellung einzuführen, um zu einem Produkt der Formel (X)

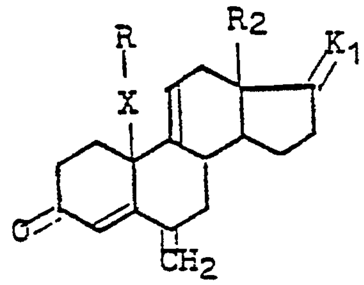

(X)

zu gelangen, und das Produkt der Formel (X) entweder mit einem Hydrierungsreagens und gegebenenfalls einem Epimerisierungsreagens behandelt, um zu einem Produkt der Formel (XI)

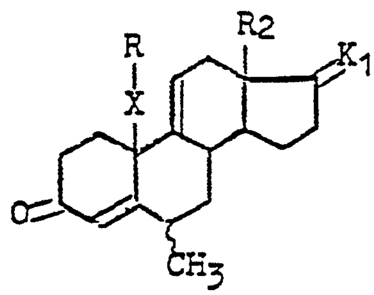

(XI)

zu gelangen, oder mit einem Isomerisierungsmittel behandelt, um zu einem Produkt der Formel (XII)

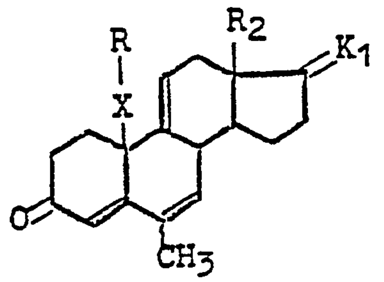

(XII)

zu gelangen, und die Produkte der Formeln (VIII), (IX), (X), (XI) und (XII) gegebenenfalls mit einem Reagens zur Abspaltung der Gruppe $K_1$ behandelt, wenn diese eine Schutzgruppe für die Ketonfunktion wiedergibt, und mit einem Mittel zur Blockierung der Ketonfunktion in 3-Stellung und hiernach mit einer Magnesiumverbindung der Formel $R_3$–Mg–$Hal_1$ behandelt, worin $R_3$ die in Anspruch 1 gegebene Bedeutung besitzt und $Hal_1$ ein Halogenatom bedeutet, um jeweils nach Abspaltung der Schutzgruppe der Ketonfunktion in 3-Stellung zu den Produkten der Formeln ($I_A$), ($I_B$), ($I_C$), ($I_D$) und ($I_E$), wie in Anspruch 1 definiert, zu gelangen, die man nach dem in Anspruch 1 beschriebenen Verfahren in die entsprechenden, in 1(2)-Stellung ungesättigten Produkte überführen und acylieren kann.

4. Verfahren gemäß Anspruch 2 oder 3, dadurch gekennzeichnet, daß man von einer Lithiumverbindung der Formel R–X–Li oder einer Magnesiumverbindung der Formel R–X'–Mg–Hal ausgeht, worin der gegebenenfalls substituierte, carbocyclische oder heterocyclische Arylrest, der von R wiedergegeben werden kann, entweder einen Phenylrest, gegebenenfalls substituiert durch einen Alkyl- oder Alkoxyrest mit 1 bis 4 Kohlenstoffatomen, durch ein Halogenatom oder durch einen Amino- oder Dialkylaminorest, oder einen Pyridylrest bezeichnet.

5. Verfahren gemäß Anspruch 2 oder 3, dadurch gekennzeichnet, daß man von einer Lithiumverbindung der Formel R–X–Li oder einer Magnesiumverbindung der Formel R–X'–Mg–Hal ausgeht, worin der gegebenenfalls substituierte Vinyl- oder Ethinylrest, der von R wiedergegeben werden kann, entweder einen Vinyl- oder Methylvinylrest der Formel

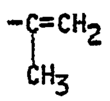

oder einen Ethinylrest, gegebenenfalls substituiert durch einen Alkylrest mit 1 bis 4 Kohlenstoffatomen, durch einen Phenylrest, durch einen gegebenenfalls veresterten Carboxyrest, durch einen Hydroxymethylrest oder durch einen gegebenenfalls geschützten oder alkylierten Aminorest bezeichnet.

**Patentansprüche für die Vertragsstaaten: BE, CH, DE, FR, GB, IT, LI, LU, NL, SE**

1. Produkte der allgemeinen Formel I

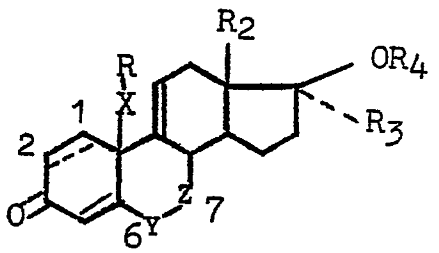

(I)

worin X und R derart sind, daß

– entweder X einen Methylenrest bedeutet und R bedeutet: entweder einen carbocyclischen oder heterocyclischen Arylrest, ausgewählt unter den Phenyl- oder Naphthyl-, Furyl-, Thienyl-, Pyrolyl-, Oxazolyl-, Thiazolyl-, Imidazolyl-, Pyrazolyl-, Thiadiazolyl-, Triazolyl-, Oxadiazolyl- oder Tetrazolyl-, Pyridinyl-, Pyrazinyl- oder Pyrimidinylresten, wobei ein jeder dieser Reste gegebenenfalls durch einen oder mehrere Substituenten substituiert ist, ausgewählt unter den Alkylresten mit 1 bis 4 Kohlenstoffatomen, Alkoxyresten mit 1 bis 4 Kohlenstoffatomen, Alkylthioresten mit 1 bis 4 Kohlenstoffatomen, Alkenylgruppen, ausgwählt unter Vinyl oder Allyl, Alkinylgruppen, ausgewählt unter Ethinyl, Propargyl oder Prop-1-inyl, den Halogenatomen, Trifluormethyl, Amino, Methylamino oder Dimethylamino, geschütztem Amino, ausgewählt unter den Tritylamino- oder Bistrimethylsilylaminoresten, Hydroxyl, Mercapto, Carboxyl, Methoxycarbonyl, Ethoxycarbonyl oder tert.-Butoxycarbonyl, in ein Salz überführtem Carboxyl, Carbamoyl, Nitro, Aminoalkyl, Dimethylaminoethyl, Carboxymethyl oder Hydroxymethyl,
oder einen Vinyl- oder Ethinylrest, gegebenenfalls substituiert durch einen oder mehrere Substituenten, ausgewählt unter den Alkylresten mit 1 bis 4 Kohlenstoffatomen, den Alkoxyresten mit 1 bis 4 Kohlenstoffatomen, den Alkylthioresten mit 1 bis 4 Kohlenstoffatomen, Alkenyl ausgewählt unter Vinyl oder Allyl, Alkinyl, ausgewählt unter Ethinyl, Propargyl oder Prop-1-inyl, den Halogenatomen, Trifluormethyl, Amino, Methylamino oder Dimethylamino, geschütztem Amino, ausgewählt unter den Tritylamino- oder Bistrimethylsilylaminoresten, Hydroxyl, Mercapto, Carboxyl, Methoxycarbonyl, Ethoxycarbonyl oder tert.-Butoxycarbonyl, in ein Salz überführtem Carboxyl; Carbamoyl, Nitro, Aminoalkyl, Dimethylaminoethyl, Carboxymethyl oder Hydroxymethyl, und den carbocyclischen oder heterocyclischen Arylresten, ausgewählt unter den Phenyl- oder Naphthyl-, Furyl-, Thienyl-, Pyrolyl, Oxazolyl-, Thiazolyl-, Imidazolyl-, Pyrazolyl-, Thiadiazolyl-, Triazolyl-, Oxadiazolyl- oder Tetrazolyl-, Pyridinyl-, Pyrazinyl- oder Pyrimidinylresten,

– oder X ein Schwefelatom oder eine einfache Bindung wiedergibt und R einen carbocyclischen oder heterocyclischen Arylrest darstellt, ausgewählt unter den Phenyl- oder Naphthyl-, Furyl-, Thienyl-, Pyrolyl-, Oxazolyl-, Thiazolyl-, Imidazolyl-, Pyrazolyl-, Thiadiazolyl-, Triazolyl-, Oxadiazolyl- oder Tetrazolyl-, Pyridinyl-, Pyrazinyl- oder Pyrimidinylresten, wobei ein jeder dieser Reste gegebenenfalls substituiert ist durch einen oder mehrere Substituenten, ausgewählt unter den Alkylresten mit 1 bis 4 Kohlenstoffatomen, Alkoxyresten mit 1 bis 4 Kohlenstoffatomen, Alkylthioresten mit 1 bis 4 Kohlenstoffatomen, Alkenylresten, ausgewählt unter Vinyl oder Allyl, Alkinylresten, ausgewählt unter Ethinyl, Propargyl oder Prop-1-inyl, den Halogenatomen, Trifluormethyl, Amino, Methylamino oder Dimethylamino, geschütztem Amino, ausgewählt unter den Tritylamino- oder Bistrimethylsilylaminoresten, Hydroxyl, Mercapto, Carboxyl, Methoxycarbonyl, Ethoxycarbonyl oder tert.-Butoxycarbonyl, in ein Salz überführtem Carboxyl; Carbamoyl, Nitro, Aminoalkyl, Monoalkylaminoalkyl, Dimethylaminoethyl, Carboxymethyl oder Hydroxymethyl;

$R_2$ einen Methyl- oder Ethylrest wiedergibt;

$R_3$ für einen Alkyl-, Alkenyl- oder Alkinylrest mit höchstens 8 Kohlenstoffatomen oder einen Hydroxypropinylrest oder Chlorethinylrest steht,

$R_4$ ein Wasserstoffatom oder einen Acylrest wiedergibt, ausgewählt unter den Acetyl-, Propionyl- oder Benzoylresten,

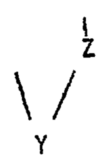

bdeutet oder eine Gruppe

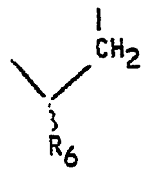

worin $R_6$ für ein Wasserstoffatom oder eine Methylgruppe in α- und/oder β-Stellung steht, oder eine Gruppe

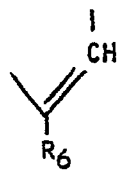

worin $R_6$ für ein Wasserstoffatom oder eine Methylrest steht, oder eine Gruppe

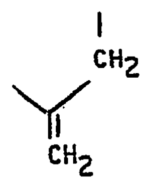

und die gestrichelte Linie die etwaige Anwesenheit einer zweiten Kohlenstoff-Kohlenstoff-Bindung anzeigt.

2. Produkte der allgemeinen Formel I, wie in Anspruch 1 definiert, dadurch gekennzeichnet, daß der gegebenenfalls substituierte, carbocyclische oder heterocyclische Arylrest, den R wiedergeben kann, <u>entweder</u> einen Phenylrest, der gegebenenfalls durch einen Alkyl- oder Alkoxyrest mit 1 bis 4 Kohlenstoffatomen, durch ein Halogenatom oder durch eine Amino- oder Dialkylaminogruppe substituiert ist, <u>oder</u> einen Pyridylrest bedeutet.

3. Produkte der allgemeinen Formel I, wie in Anspruch 1 definiert, dadurch gekennzeichnet, daß der gegebenenfalls substituierte Vinyl- oder Ethinylrest, den R wiedergeben kann, <u>entweder</u> einen Vinyl- oder Methylvinylrest der Formel

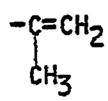

<u>oder</u> einen Ethinylrest bezeichnet, der gegebenenfalls substituiert ist durch einen Alkylrest mit 1 bis 4 Kohlenstoffatomen, durch einen Phenylrest, durch einen gegebenenfalls veresterten Carboxyrest, durch einen Hydroxymethylrest oder durch eine Amino-, Tritylamino-, Bistrimethylsilylamino-, Methylamino- oder Dimethylaminogruppe.

4. Produkte der allgemeinen Formel I mit den folgenden Bezeichnungen:
10β-Benzyl-17β-hydroxy-17α-(prop-1-inyl)-östra-4,9(11)-dien-3-on;
17β-Hydroxy-10β-[(4-methylphenyl)-methyl]-17α-(prop-1-inyl)-östra-4,9(11)-dien-3-on;
10β-(2-Methylprop-2-en-1-yl)-17α-(prop-1-inyl)-17β-hydroxy-östra-4,9(11)-dien-3-on;
10β-[(4-Methoxyphenyl)-thio]-17α-(prop-1-inyl)-17β-hydroxy-östra-4,9(11)-dien-3-on;
10β-[(2-Fluorphenyl)-methyl]-17α-(prop-1-inyl)-17β-hydroxy-östra-4,9(11)-dien-3-on.

5. Verfahren zur Herstellung der Produkte der Formel I, wie in Anspruch 1 definiert, dadurch gekennzeichnet, daß man ein Produkt der Formel II:

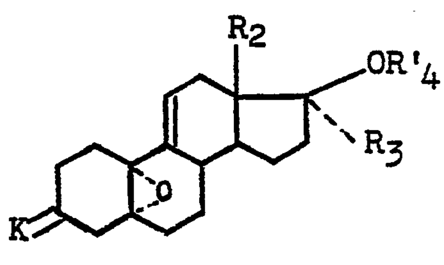

(II)

worin $R_2$ und $R_3$ wie in Anspruch 1 definiert sind, K eine Schutzgruppe für die Ketonfunktion wiedergibt und $R'_4$ ein Wasserstoffatom oder eine Schutzgruppe für den Hydroxylrest bedeutet,

– <u>entweder</u> mit einer Lithiumverbindung der Formel R–X–Li

– <u>oder</u> mit einer Magnesiumverbindung der Formel R–X′–Mg–Hal, worin R und X jeweils die in Anspruch 1 angegebene Bedeutung besitzen, X′ eine Methylgengruppe oder eine einfache Bindung wiedergibt und Hal eine Halogenatom bedeutet, behandelt, um zu einem Produkt der Formel III

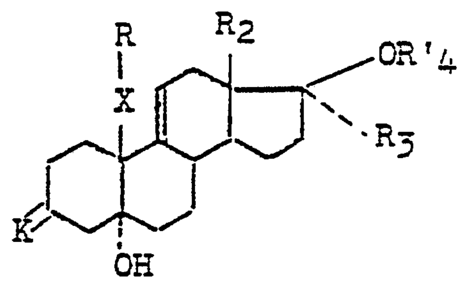

(III)

zu gelangen, das man mit einer Säure behandelt, um die Produkte der Formel IV

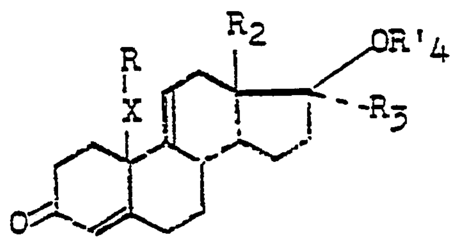

(IV)

zu erhalten, die man erforderlichenfalls mit einem Reagens zur Abspaltung der Gruppe $R'_4$ behandelt, wenn diese eine Schutzgruppe für die Hydroxylgruppe wiedergibt, die von einer Acylgruppe verschieden ist, um zu einem Produkt der Formel $I_A$

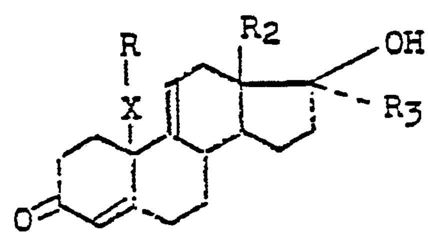

($I_A$)

zu gelangen, und gewünschtenfalls ein Produkt der Formel IV mit einem Alkylorthoformiat, anschließend mit einem Dehydrierungsreagens und schließlich erforderlichenfalls mit einem Reagens zur Abspaltung der Gruppe $R'_4$ behandelt, wenn diese eine Schutzgruppe für die Hydroxylgruppe wiedergibt, die von einem Acylrest verschieden ist, um zu einem Produkt der Formel $I_B$

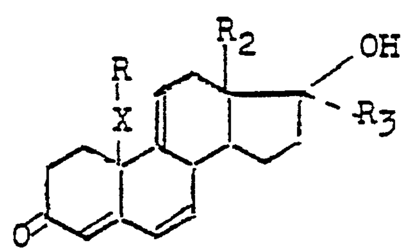

($I_B$)

zu gelangen, und gewünschtenfalls das Produkt der Formel IV mit einem Reagens behandelt, das befähigt ist, eine Methylengruppe in 6-Stellung einzuführen, um zu einem Produkt der Formel V

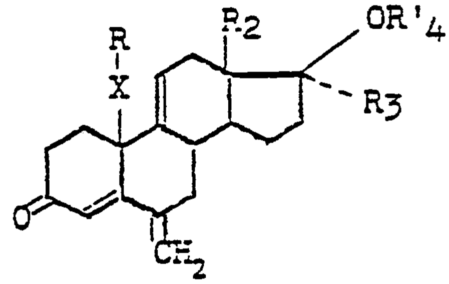

(V)

zu gelangen, das man erforderlichenfalls mit einem Reagens zur Abspaltung der Gruppe R'$_4$ behandelt, wenn diese eine Schutzgruppe für den Hydroxylrest darstellt, die von einer Acylgruppe verschieden ist, um zu einem Produkt der Formel I$_C$

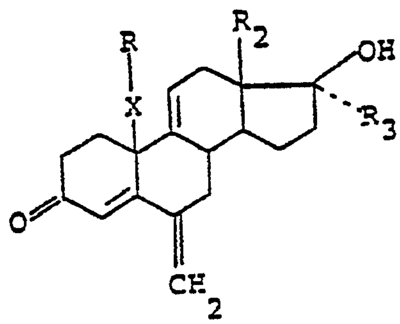

(I$_C$)

oder einem Produkt der Formel V zu gelangen, das man
– entweder mit einem Hydrierungsreagens und gegebenenfalls Epimerisierungsreagens, danach mit einem Reagens zur Abspaltung der Gruppe R'$_4$ behandelt, wenn diese eine Schutzgruppe für die Hydroxylgruppe wiedergibt, die von einem Acylrest verschieden ist, um zu einem Produkt der Formel I$_D$

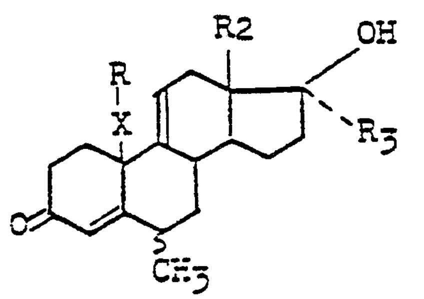

(I$_D$)

zu gelangen,
– oder mit einem Isomerisierungsreagens behandelt, um nach Behandlung mit einem Reagens zur Abspaltung der Gruppe R'$_4$, wenn diese eine Schutzgruppe für den Hydroxylrest wiedergibt, ein Produkt der Formel I$_E$

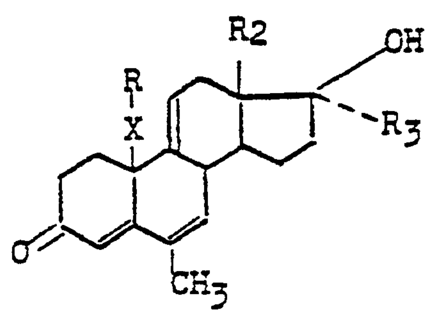

(I$_E$)

zu erhalten, und gewünschtenfalls die Produkte der vorstehenden Formeln (I$_A$), (I$_B$), (I$_C$), (I$_D$) und (I$_E$) mit einem Dehydrierungsreagens oder mit einem zur Dehydrierung befähigten Mikroorganismus behandelt, um die entsprechenden Produkte mit einer Unsättigung in 1(2)-Stellung zu erhalten, und die Produkte der Formeln ($I_A$), ($I_B$), ($I_C$) und ($I_D$) sowie ($I_E$) und die entsprechenden Produkte mit einer Unsättigung in 1(2)-Stellung gewünschtenfalls mit einem Acylierungsreagens behandelt.

6. Verfahren zur Herstellung der Produkte der allgemeinen Formel (I), wie vorstehend definiert, dadurch gekennzeichnet, daß man ein Produkt der Formel (VI)

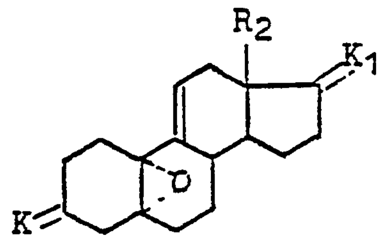

(VI)

worin $R_2$ wie in Anspruch 1 definiert ist, K eine Schutzgruppe für die Ketonfunktion wiedergibt und $K_1$ eine gegebenenfalls geschützte Ketonfunktion bedeutet,

– <u>entweder</u> mit einer Lithiumverbindung der Formel R–X–Li

– <u>oder</u> mit einer Magnesiumverbindung der Formel R–X'–Mg–Hal, worin jeweils R und X wie in Anspruch 1 definiert sind, X' einen Methylenrest oder eine Einfachbindung wiedergibt und Hal ein Halogenatom bedeutet, behandelt, um zu einem Produkt der Formel (VII)

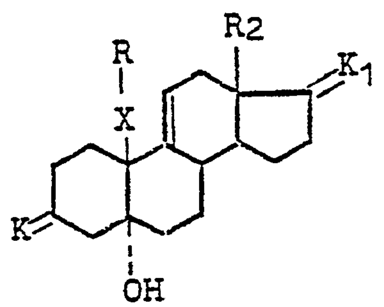

(VII)

zu gelangen, das man behandelt

– <u>entweder</u> mit einem Reagens zur Abspaltung des Restes $K_1$, wenn dieser eine Schutzgruppe für den Ketorest in 17-Stellung wiedergibt, hiernach mit einer Magnesiumverbindung $R_3$–Mg–$Hal_1$ oder mit einer Lithiumverbindung der Formel $R_3$–Li, worin jeweils $R_3$ wie in Anspruch 1 definiert ist und $Hal_1$ ein Halogenatom bedeutet, um zu einem Produkt der Formel ($III_A$)

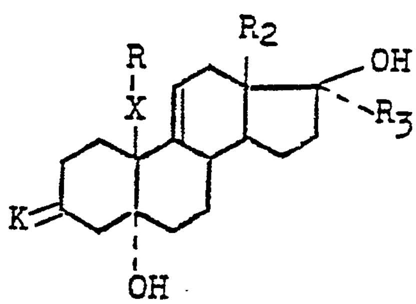

($III_A$)

zu gelangen, das man mit einer Säure behandelt, um ein Produkt der Formel ($I_A$), wie in Anspruch 5 definiert, zu erhalten, welches man gegebenenfalls einer Reaktion zum Schutz der Hydroxylgruppe in 17β-Stellung unterzieht, und das so erhaltene Produkt der Formeln ($I_A$) oder (IV) nach dem in Anspruch 1 beschriebenen Verfahren behandelt, um zu den Produkten der Formeln ($I_B$), ($I_C$), ($I_D$) und ($I_E$) und den entsprechenden, in 1(2)-Stellung ungesättigten Produkten zu gelangen;

– <u>oder</u> das Produkt der Formel (VII) mit einer Säure behandelt, um zu einem Produkt der Formel (VIII)

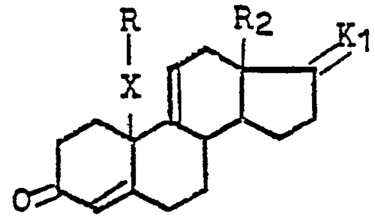

(VIII)

zu gelangen, welches Produkt der Formel (VIII) man
entweder mit einem Alkylorthoformiat, hiernach mit einem Dehydrierungsreagens behandelt, um zu einem Produkt der Formel (IX)

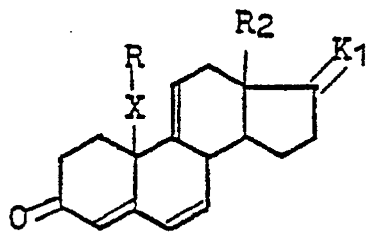

(IX)

zu gelangen,
oder mit einem Reagens behandelt, das befähigt ist, einen Methylenrest in 6-Stellung einzuführen, um zu einem Produkt der Formel (X)

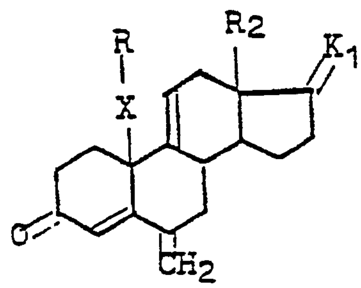

(X)

zu gelangen, und das Produkt der Formel (X) entweder mit einem Hydrierungsreagens und gegebenenfalls einem Epimerisierungsreagens behandelt, um zu einem Produkt der Formel (XI)

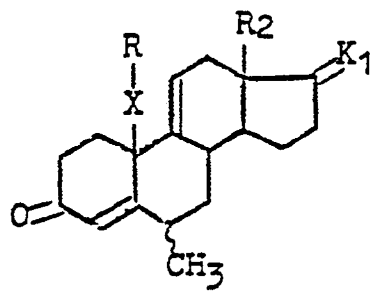

(XI)

zu gelangen, oder mit einem Isomerisierungsmittel behandelt, um zu einem Produkt der Formel (XII)

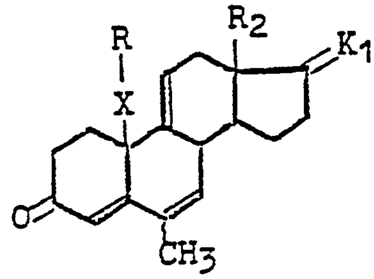

(XII)

zu gelangen, und die Produkte der Formeln (VIII), (IX), (X), (XI) und (XII) gegebenenfalls mit einem Reagens zur Abspaltung der Gruppe $K_1$ behandelt, wenn diese eine Schutzgruppe für die Ketonfunktion wiedergibt, und mit einem Mittel zur Blockierung der Ketonfunktion in 3-Stellung und hiernach mit einer Magnesiumverbindung der Formel $R_3$–Mg–$Hal_1$ behandelt, worin $R_3$ die in Anspruch 1 gegebene Bedeutung besitzt und $Hal_1$ ein Halogenatom bedeutet, um jeweils nach Abspaltung der Schutzgruppe der Ketonfunktion in 3-Stellung zu den Produkten der Formeln ($I_A$), ($I_B$), ($I_C$), ($I_D$) und ($I_E$), wie in Anspruch 1 definiert, zu gelangen, die man nach dem in Anspruch 1 beschriebenen Verfahren in die entsprechenden, in 1(2)-Stellung ungesättigten Produkte überführen und acylieren kann.

7. Als Arzneimittel die Produkte der allgemeinen Formel (I) gemäß einem der Ansprüche 1 bis 3.

8. Als Arzneimittel die Produkte der allgemeinen Formel (I) gemäß Anspruch 4.

9. Pharmazeutische Zusammensetzungen, dadurch gekennzeichnet, daß sie als Wirkstoff zumindest ein Arzneimittel gemäß einem der Ansprüche 7 oder 8 enthalten.

10. Als neue, industrielle Produkte die Produkte der allgemeinen Formel (A)

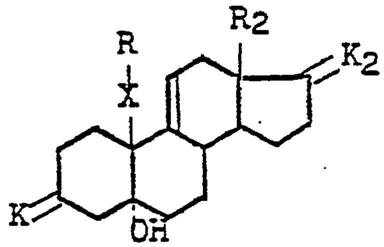

(A)

worin X, R und $R_2$ wie in Anspruch 1 definiert sind, K eine Schutzgruppe für die Ketonfunktion, ausgewählt unter den Ketal-, Thioketal-, Oxim- und Alkoximgruppen, wiedergibt und $K_2$ bedeutet:

– entweder eine Ketonfunktion, die gegebenenfalls durch eine Ketal-, Thioketal-, Oxim- oder Alkoximgruppe geschützt ist,

– oder einen Rest

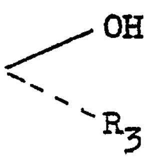

,

worin $R_3$ wie in Anspruch 1 definiert ist und worin die Hydroxylgruppe gegebenenfalls geschützt ist durch eine Gruppe, ausgewählt unter den Tetrahydropyranyl-, tert.-Butyl-, Acetyl-, Chloracetyl- oder Trifluoracetylgruppen.

**Claims for the contracting States: BE, CH, DE, FR, GB, IT, LI, LU, NL, SE**

1. The products of general formula (I):

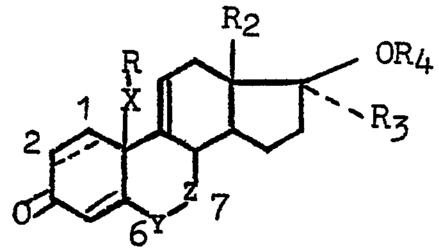

(I)

in which X and R are such that:

– either X represents a methylene radical and R represents either a carbocyclic or heterocyclic aryl radical chosen from the following radicals: phenyl or naphthyl, furyl, thienyl, pyrrolyl, oxazolyl, thiazolyl, imidazolyl, pyrazolyl, thiadiazolyl, triazolyl, oxadiazolyl or tetrazolyl, pyridinyl, pyrazinyl or pyrimidinyl, each of these radicals being optionally substituted by one or more substituents chosen from alkyl radicals having from 1 to 4 carbon atoms, alkoxy radicals having from 1 to 4 carbon atoms, alkylthio radicals having from 1 to 4 carbon atoms, alkenyl radicals chosen from vinyl or allyl, alkenyl radicals chosen from ethynyl, propargyl or prop-1-ynyl, halogens, trifluoromethyl, amino, methylamino or dimethylamino, protected amino chosen from tritylamino or bis-trimethylsilylamino radicals, hydroxyl, mercapto, carboxyl, methoxycarbonyl, ethoxycarbonyl or tert-butoxy carbonyl, salified carboxyl, carbamoyl, nitro, aminoalkyl, dimethylaminoethyl, carboxymethyl or hydroxymethyl, or a vinyl or ethynyl radical optionally substituted by one or more substituents chosen from alkyl radicals having from 1 to 4 carbon atoms, alkoxy radicals having from 1 to 4 carbon atoms, alkylthio radicals having from 1 to 4 carbon atoms, alkenyl chosen from vinyl or allyl, alkynyl chosen from ethynyl, propargyl or prop-1-ynyl, halogens, trifluoromethyl, amino, methylamino or dimethylamino, protected amino chosen from tritylamino or bis-trimethylsilylamino radicals, hydroxyl, mercapto, carboxyl, methoxycarbonyl, ethoxycarbonyl or tert-butoxy carbonyl, salified carboxyl, carbamoyl, nitro, aminoalkyl, dimethylaminoethyl, carboxymethyl or hydroxymethyl and carbocyclic or heterocyclic aryl radicals chosen from the following radicals: phenyl or naphthyl, furyl, thienyl, pyrrolyl, oxazolyl, thiazolyl, imidazolyl, pyrazolyl, thiadiazolyl, triazolyl, oxadiazolyl or tetrazolyl, pyridinyl, pyrazinyl or pyrimidinyl

– or X represents a sulphur atom or a single bond and R represents a carbocyclic or heterocyclic aryl radical chosen from the following radicals: phenyl or naphthyl, furyl, thienyl, pyrrolyl, oxazolyl, thiazolyl, imidazolyl, pyrazolyl, thiadiazolyl, triazolyl, oxadiazolyl or tetrazolyl, pyridinyl, pyrazinyl or pyrimidinyl, each of these radicals being optionally substituted by one or more substituents chosen from the following radicals: alkyl having from 1 to 4 carbon atoms, alkoxy having from 1 to 4 carbon atoms, alkylthio having from 1 to 4 carbon atoms, alkenyl chosen from vinyl or allyl, alkynyl chosen from ethynyl, propargyl or prop-1-ynyl, halogens, trifluoromethyl, amino, methylamino or dimethylamino, protected amino chosen from tritylamino or bis-trimethylsilylamino radicals, hydroxyl, mercapto, carboxyl, methoxycarbonyl, ethoxycarbonyl or tert-butoxy carbonyl, salified carboxyl, carbamoyl, nitro, aminoalkyl, monoalkylaminoalkyl, dimethylaminoethyl, carboxymethyl or hydroxymethyl

$R_2$ represents a methyl or ethyl radical,

$R_3$ represents an alkyl, alkenyl or alkynyl radical having at the most 8 carbon atoms or a hydroxypropynyl or chloroethynyl radical,

$R_4$ represents a hydrogen atom or an acyl radical, chosen from acetyl, propionyl or benzoyl radicals

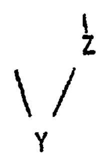

represents: a group

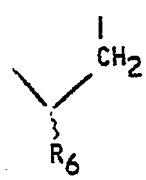

in which $R_6$ represents a hydrogen atom or a methyl radical in alpha and/or beta position, or a group

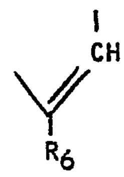

in which $R_6$ represents a hydrogen atom or a methyl radical, or a group

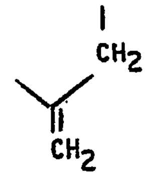

the dotted line in position 1(2) indicates the optional presence of a second carbon-carbon bond.

2. The products of general formula (I) as defined in claim 1, characterized in that the optionally substituted carbocyclic or heterocyclic aryl radical which can be represented by R signifies either a phenyl radical optionally substituted by an alkyl or alkoxy radical having from 1 to 4 carbon atoms, by a halogen atom or by an amino or dialkylamino radical, or a pyridyl radical.

3. The products of general formula (I) as defined in claim 1, characterized in that the optionally substituted vinyl or ethynyl radical which can be represented by R signifies either a vinyl or methylvinyl radical of formula:

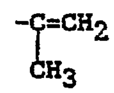

or an ethynyl radical optionally substituted by an alkyl radical having from 1 to 4 carbon atoms, by a phenyl radical, by an optionally esterified carboxy radical, by a hydroxymethyl radical or by one of the following radicals: amino, tritylamino, bis-trimethylsilylamino, methylamino or dimethylamino.

4. The products of general formula (I) of which the names follow:

- 10beta-benzyl 17beta-hydroxy 17alpha-(prop-1-ynyl) estra 4,9(11)-dien-3-one;
- 17beta-hydroxy 10beta-[(4-methylphenyl)methyl] 17alpha-(prop-1-ynyl) estra 4,9(11)-dien-3-one;
- 10beta-(2-methylprop-2-en-1-yl) 17alpha-(prop-1-ynyl) 17beta-hydroxy estra 4,9(11)-dien-3-one;
- 10beta-[(4-methoxyphenyl)thio] 17alpha-(prop-1-ynyl) 17beta-hydroxy estra 4,9(11)-dien-3-one;
- 10beta-[(2-fluorophenyl)methyl] 17alpha-(prop-1-ynyl) 17beta-hydroxy estra 4,9(11)-dien-3-one.

5. Preparation process for products of formula (I) as defined in claim 1, characterized in that a product of formula (II):

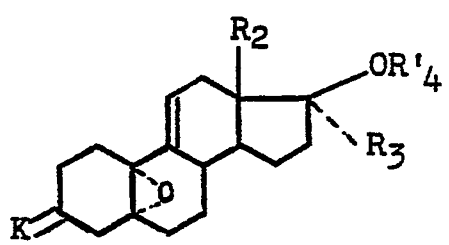

(II)

in which $R_2$ and $R_3$ have the significance indicated in claim 1, K represents a protector group of the ketone function and $R_4$ represents a hydrogen atom or a protector group of the hydroxyl radical, is treated:

- either with a lithium derivative of formula R–X–Li,
- or with a magnesium derivative of formula R–X′–Mg–Hal, in which formulae R and X have the significance indicated in claim 1, X′ represents a methylene radical or a simple bond and Hal represents a halogen atom, so as to obtain a product of formula (III):

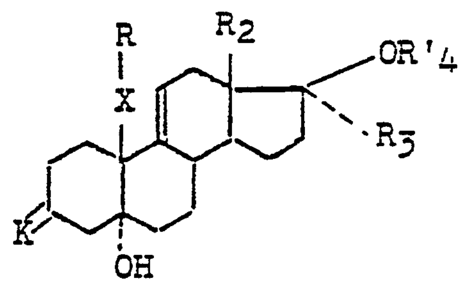

(III)

which is treated with an acid so as to obtain the products of formula (IV):

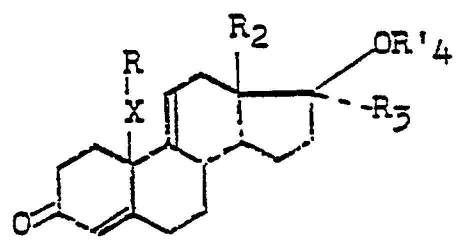

(IV)

which, if necessary, are treated with a cleavage reagent of the R'$_4$ group when the latter represents a protector group of the hydroxyl radical different from an acyl radical, so as to obtain a product of formula ($I_A$):

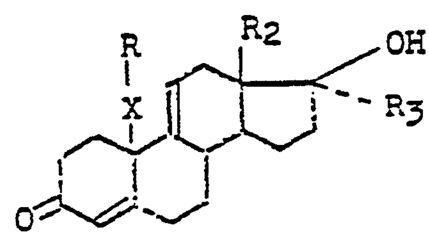

($I_A$)

and in that, if desired, a product of formula (IV) is treated with an alkyl orthoformate, then with a dehydrogenation reagent and finally, if necessary, with a cleavage reagent of the R'$_4$ group when the latter represents a protector group of the hydroxyl radical different from an acyl radical, so as to obtain a product of formula ($I_B$):

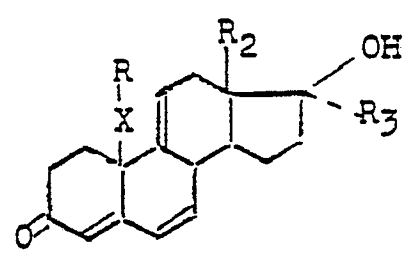

($I_B$)

and in that, if desired, a product of formula (IV) is treated with a reagent capable of introducing a methylene radical, in position 6, so as to obtain a product of formula (V):

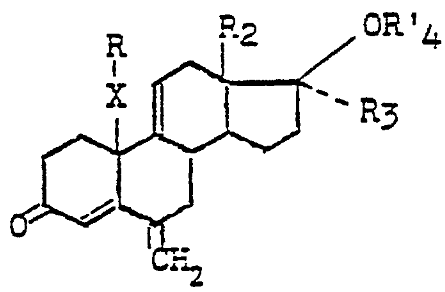

(V)

which, if necessary, is treated with a cleavage reagent of the R'4 group when the latter represents a protector group of the hydroxyl radical different from an acyl radical, so as to obtain a product of formula ($I_C$):

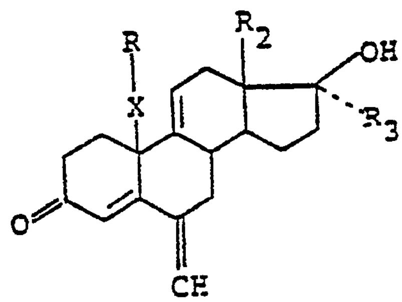

($I_C$)

or which product of formula (V) is:

– <u>either</u> treated with a hydrogenation reagent and optionally an epimerization reagent, then by a cleavage reagent of the R'4 group when the latter represents a protector group of the hydroxyl radical different from an acyl radical, so as to obtain a product of formula ($I_D$):

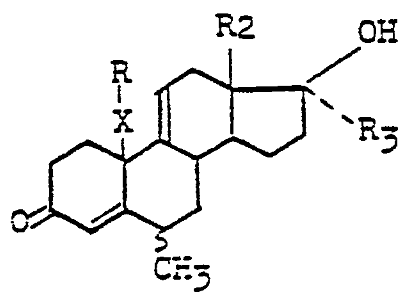

($I_D$)

– <u>or</u> treated with an isomerization agent so as to obtain, after treatment with a cleavage reagent of the R'4 group, when the latter represents a protector group of the hydroxyl radical, a product of formula ($I_E$):

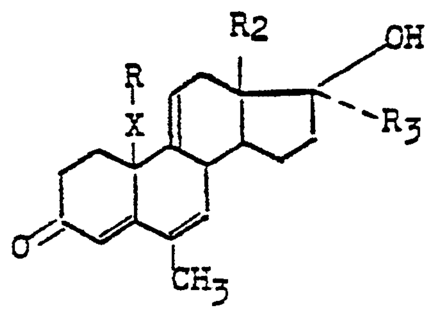

($I_E$)

and in that, if desired, the preceding products of formulae ($I_A$), ($I_B$), ($I_C$), ($I_D$) and ($I_E$) are treated with a dehydrogenation reagent or a micro-organism which can dehydrogenate, so as to obtain the corresponding products containing an unsaturation in position 1(2) and products of formula ($I_A$), ($I_B$), ($I_C$), ($I_D$) and ($I_E$) and the corresponding products containing an unsaturation in position 1(2) which if desired are treated with an acylation reagent.

6. Preparation process for products of general formula (I) as defined above, characterized in that a product of formula (VI):

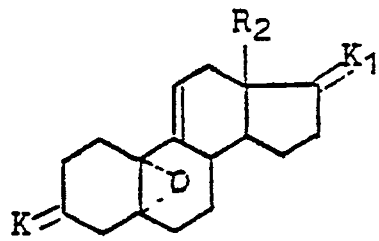

(VI)

in which $R_2$ has the significance indicated in claim 1, K represents a protector group of the ketone function and $K_1$ represents an optionally protected ketone function, is treated:

– either with a lithium derivative of formula R–X–Li,

– or with a magnesium derivative of formula R–X'–Mg–Hal, in which formulae R and X have the significances indicated in claim 1, X' represents a methylene radical or a simple bond and Hal represents a halogen atom, so as to obtain a product of formula (VII):

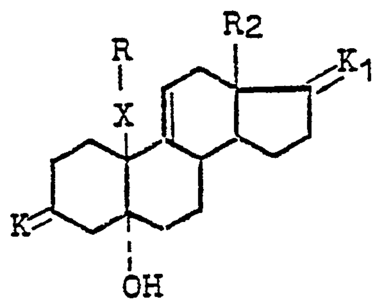

(VII)

which product is treated:

– either with a cleavage reagent of the $K_1$ radical when the latter represents a protector group of the keto radical in position 17, then with a magnesium derivative of formula $R_3$–Mg–$Hal_1$ or with a lithium derivative of formula $R_3$–Li, in which formulae $R_3$ has the significance indicated in claim 1, and $Hal_1$ represents a halogen atom, so as to obtain a product of formula ($III_A$):

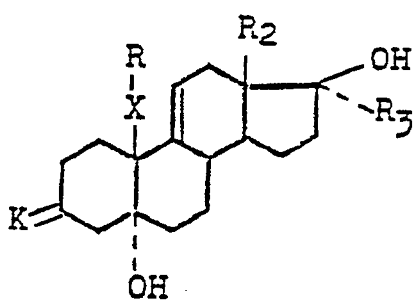

($III_A$)

which is treated with an acid so as to obtain a product of formula ($I_A$) as defined in claim 5, which product is optionally submitted to a protection reaction of the hydroxyl radical in position 17beta, and the product of formula ($I_A$) or (IV) thus obtained is treated according to the process described in claim 5, so as to obtain the products of formulae ($I_B$), ($I_C$), ($I_D$) and ($I_E$) and the corresponding unsaturated products in position 1(2),

– or the product of formula (VII) is treated with an acid so as to obtain a product of formula (VIII):

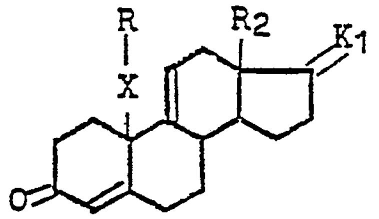

(VIII)

which product of formula (VIII) is treated

either with an alkyl orthoformate, then with a dehydrogenation reagent, so as to obtain a product of formula (IX):

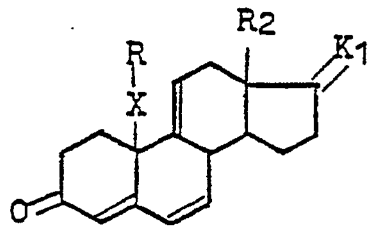

(IX)

or by a reagent capable of introducing a methylene radical, in position 6, so as to obtain a product of formula (X):

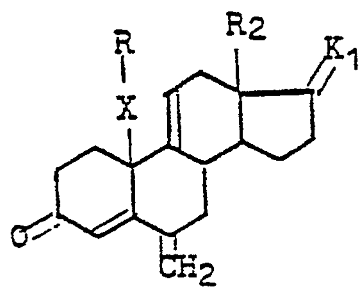

(X)

and which product of formula (X) is treated either by a hydrogenation reagent and optionally an epimerization agent, so as to obtain a product of formula (IX):

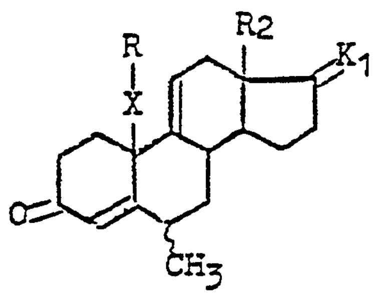

(XI)

or by an isomerization agent, so as to obtain a product of formula (XII):

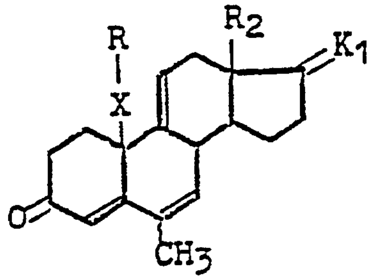

(XII)

and which products of formulae (VIII), (IX), (X), (XI) and (XII) are optionally treated with a cleavage reagent of the $K_1$ group when the latter represents a protector group of the ketone function, and with a blocking agent of the ketone function in position 3, then with a magnesium derivative of formula $R_3$–Mg–$Hal_1$ in which $R_3$ has the significance indicated in claim 1 and $Hal_1$ represents a halogen atom, so as to obtain respectively, after deblocking of the ketone function in position 3, the products of formulae ($I_A$), ($I_B$), ($I_C$), ($I_D$) and ($I_E$) as defined in claim 5, which can be converted, according to the process of claim 5, into corresponding unsaturated products in position 1(2) and which can be acylated.

7. As medicaments, the products of general formula (I) as defined in one of claims 1 to 3.

8. As medicaments, the products of general formula (I) as defined in claim 4.

9. The pharmaceutical compositions containing, as active principle, at least one medicament defined in any one of claims 7 or 8.

10. As new industrial products, the products of general formula (A):

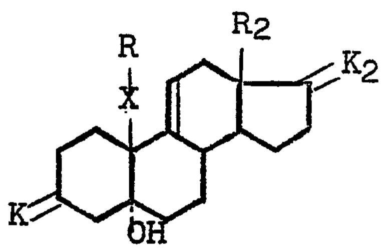

in which X, R and $R_2$ have the significance indicated in claim 1, K represents a protector group of the ketone function chosen from ketal, thioketal, oxime and alkyloxime groups and $K_2$ represents:

– either a ketone function optionally protected by a ketal, thioketal, oxime or alkyloxime group,

– or a radical

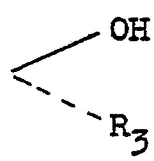 ,

in which $R_3$ has the significance indicated in claim 1 and in which the hydroxyl is optionally protected by a group chosen from the following groups: tetrahydropyrannyl, tert-butyl, acetyl, chloroacetyl or trifluoroacetyl.

**Claims for the contracting State: AT**

1. Process for the preparation of products of general formula (I):

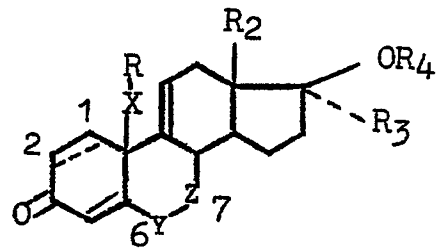

(I)

in which X and R are such that:

– either X represents a methylene radical and R represents either a carbocyclic or heterocyclic aryl radical chosen from the following radicals: phenyl or naphthyl, furyl, thienyl, pyrrolyl, oxazolyl, thiazolyl, imidazolyl, pyrazolyl, thiadiazolyl, triazolyl, oxadiazolyl or tetrazolyl, pyridinyl, pyrazinyl or pyrimidinyl, each of these radicals being optionally substituted by one more substituents chosen from alkyl radicals having from 1 to 4 carbon atoms, alkoxy radicals having from 1 to 4 carbon atoms, alkylthio radicals having from 1 to 4 carbon atoms, alkenyl radicals chosen from vinyl or allyl, alkenyl radicals chosen from ethynyl, propargyl or prop-1-ynyl, halogens, trifluoromethyl, amino, methylamino or dimethylamino, protected amino chosen from tritylamino or bis-trimethylsilylamino radicals, hydroxyl, mercapto, carboxyl, methoxycarbonyl, ethoxycarbonyl or tert-butoxy carbonyl, salified carboxyl, carbamoyl, nitro, aminoalkyl, dimethylaminoethyl, carboxymethyl or hydroxymethyl, or a vinyl or ethynyl radical optionally substituted by one or more substituents chosen from alkyl radicals having from 1 to 4 carbon atoms, alkoxy radicals having from 1 to 4 carbon atoms, alkylthio radicals having from 1 to 4 carbon atoms, alkenyl chosen from vinyl or allyl, alkynyl chosen from ethynyl, propargyl or prop-1-ynyl, halogens, trifluoromethyl, amino, methylamino or dimethylamino, protected amino chosen from tritylamino or bis-trimethylsilylamino radicals, hydroxyl, mercapto, carboxyl, methoxycarbonyl, ethoxycarbonyl or tert-butoxy carbonyl, salified carboxyl, carbamoyl, nitro, aminoalkyl, dimethylaminoethyl, carboxymethyl or hydroxymethyl and carbocyclic or heterocyclic aryl radicals chosen from the following radicals: phenyl or naphthyl, furyl, thienyl, pyrrolyl, oxazolyl, thiazolyl, imidazolyl, pyrazolyl, thiadiazolyl, triazolyl, oxadiazolyl or tetrazolyl, pyridinyl, pyrazinyl or pyrimidinyl

– or X represents a sulphur atom or a single bond and R represents a carbocyclic or heterocyclic aryl radical chosen from the following radicals: phenyl or naphthyl, furyl, thienyl, pyrrolyl, oxazolyl, thiazolyl, imidazolyl, pyrazolyl, thiadiazolyl, triazolyl, oxadiazolyl or tetrazolyl, pyridinyl, pyrazinyl or pyrimidinyl, each of these radicals being optionally substituted by one or more substituents chosen from the following radicals: alkyl having from 1 to 4 carbon atoms, alkoxy having from 1 to 4 carbon atoms, alkylthio having from 1 to 4 carbon atoms, alkenyl chosen from vinyl or allyl, alkynyl chosen from ethynyl, propargyl or prop-1-ynyl, halogens, trifluoromethyl, amino, methylamino or dimethylamino, protected amino chosen from tritylamino or bis-trimethylsilylamino radicals, hydroxyl, mercapto, carboxyl, methoxycarbonyl, ethoxycarbonyl or tert-butoxy carbonyl, salified carboxyl, carbamoyl, nitro, aminoalkyl, monoalkylaminoalkyl, dimethylaminoethyl, carboxymethyl or hydroxymethyl

$R_2$ represents a methyl or ethyl radical,

$R_3$ represents an alkyl, alkenyl or alkynyl radical having at the most 8 carbon atoms or a hydroxypropynyl or chloroethynyl radical,

$R_4$ represents a hydrogen atom or an acyl radical, chosen from acetyl, propionyl or benzoyl radicals

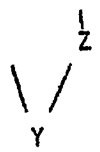

represents: a group

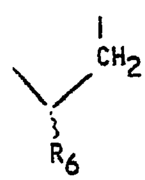

in which $R_6$ represents a hydrogen atom or a methyl radical in alpha and/or beta position, or a group

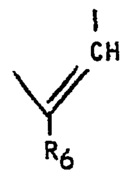

in which $R_6$ represents a hydrogen atom or a methyl radical,
or a group

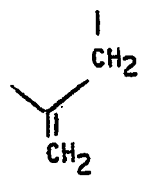

the dotted line in position 1(2) indicates the optional presence of a second carbon-carbon bond, characterized in that a product of formula (II)

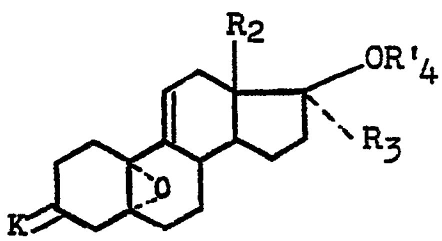

(II)

in which $R_2$ and $R_3$ have the significance indicated in claim 1, K represents a protector group of the ketone function and $R_4$ represents a hydrogen atom or a protector group of the hydroxyl radical, is treated:
- either with a lithium derivative of formula R–X–Li,
- or with a magnesium derivative of formula R–X′–Mg–Hal, in which formulae R and X have the significances indicated previously, X′ represents a methylene radical or a simple bond and Hal represents a halogen atom, so as to obtain a product of formula (III):

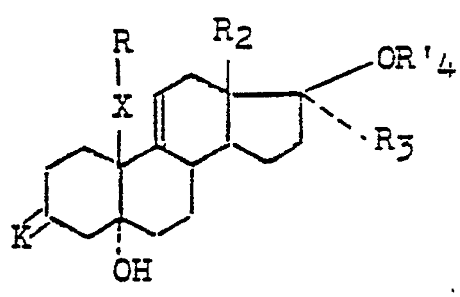

(III)

which is treated with an acid so as to obtain the products of formula (IV):

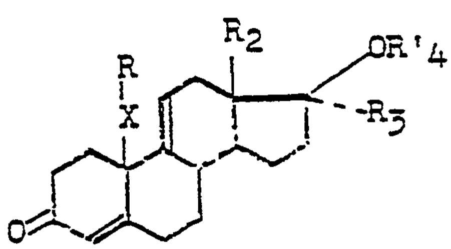

(IV)

which, if necessary, are treated with a cleavage reagent of the $R'_4$ group when the latter represents

a protector group of the hydroxyl radical different from an acyl radical, so as to obtain a product of formula ($I_A$):

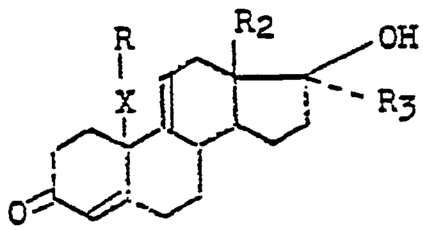

($I_A$)

and in that, if desired, a product of formula (IV) is treated with an alkyl orthoformate, then with a dehydrogenation reagent and finally, if necessary, with a cleavage reagent of the $R'_4$ group when the latter represents a protector group of the hydroxyl radical different from an acyl radical, so as to obtain a product of formula ($I_B$):

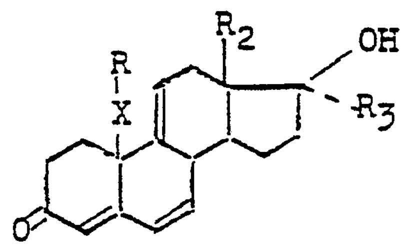

($I_B$)

and in that, if desired, a product of formula (IV) is treated with a reagent capable of introducing a methylene radical, in position 6, so as to obtain a product of formula (V):

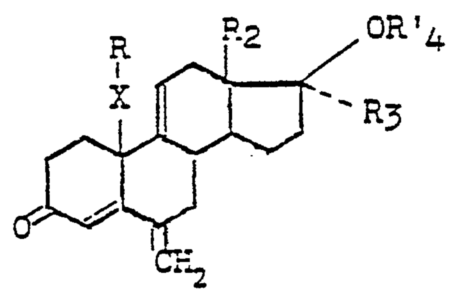

(V)

which, if necessary, is treated with a cleavage reagent of the $R'_4$ group when the latter represents a protector group of the hydroxyl radical different from an acyl radical, so as to obtain a product of formula ($I_C$):

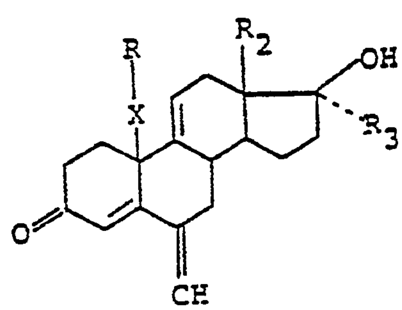

($I_C$)

or which product of formula (V) is:

– <u>either</u> treated with a hydrogenation reagent and optionally an epimerization reagent, then by a cleavage reagent of the $R'_4$ group when the latter represents a protector group of the hydroxyl radical different from an acyl radical, so as to obtain a product of formula ($I_D$):

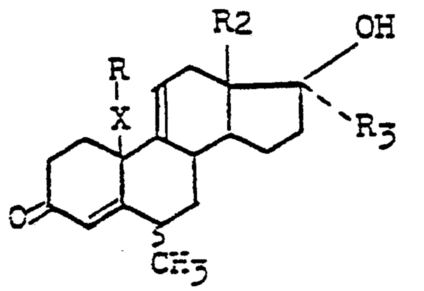

($I_D$)

– or treated with an isomerization agent so as to obtain, after treatment with a cleavage reagent of the $R'_4$ group when the latter represents a protector group of the hydroxyl radical, a product of formula ($I_E$)

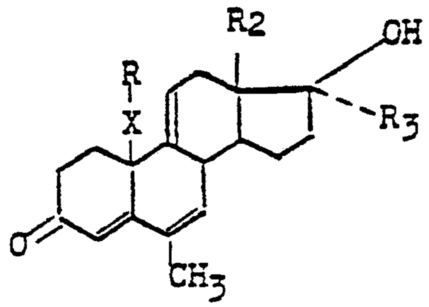

($I_E$)

and in that, if desired, the preceding products of formulae ($I_A$), ($I_B$), ($I_C$), ($I_D$) and ($I_E$) are treated with a dehydrogenation reagent or a micro-organism which can dehydrogenate, so as to obtain the corresponding products containing an unsaturation in position 1(2) and products of formula ($I_A$), ($I_B$), ($I_C$), ($I_D$) and ($I_E$) and the corresponding products containing an unsaturation in position 1(2) which if desired are treated with an acylation reagent.

2. Process according to claim 1, characterized in that a product of formula (II):

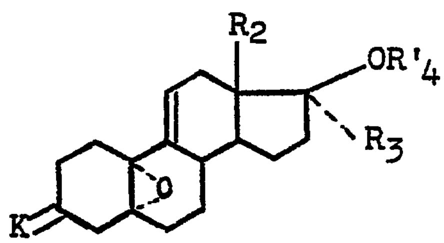

(II)

in which $R_2$ and $R_3$ have the significance indicated in claim 1, K represents a protector group of the ketone function and $R'_4$ represents a hydrogen atom or a protector group of the hydroxyl radical, is treated:

– either with a lithium derivative of formula R–X–Li,

– or with a magnesium derivative of formula R–X'–Mg–Hal, in which formulae R and X have the significance indicated in claim 1, X' represents a methylene radical or a simple bond and Hal represents a halogen atom, so as to obtain a product of formula (III):

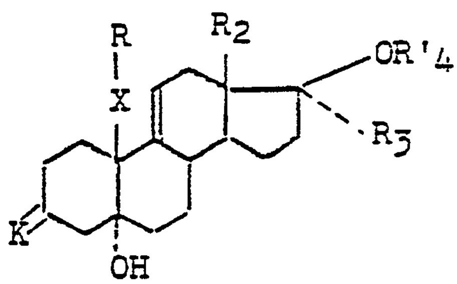

(III)

which is treated with an acid so as to obtain the products of formula (IV):

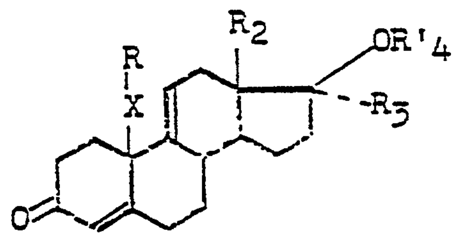

(IV)

which, if necessary, are treated with a cleavage reagent of the R'4 group when the latter represents a protector group of the hydroxyl radical different from an acyl radical, so as to obtain a product of formula ($I_A$):

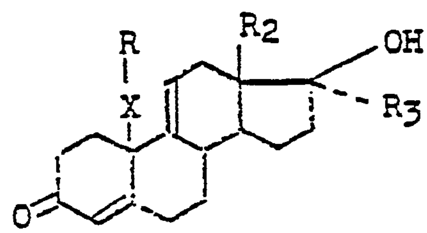

($I_A$)

and in that, if desired, a product of formula (IV) is treated with an alkyl orthoformate, then with a dehydrogenation reagent and finally, if necessary, with a cleavage reagent of the R'4 group when the latter represents a protector group of the hydroxyl radical different from an acyl radical, so as to obtain a product of formula ($I_B$):

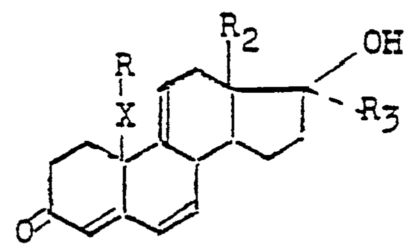

($I_B$)

and in that, if desired, a product of formula (IV) is treated with a reagent capable of introducing a methylene radical, in position 6, so as to obtain a product of formula (V):

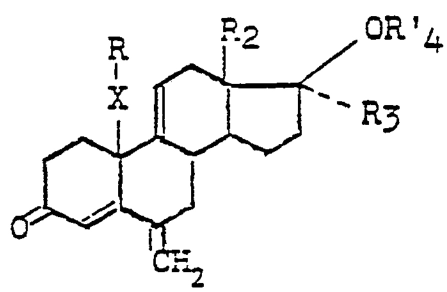

(V)

which corresponds, when R'4 represents a hydrogen atom, to a product of formula ($I_C$):

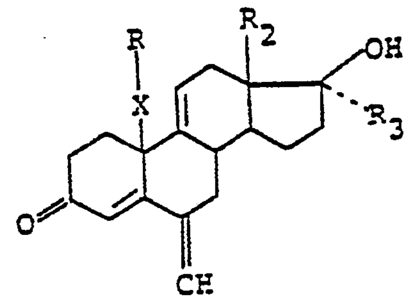

$(I_C)$

or which product of formula (V) is:

– <u>either</u> treated with a hydrogenation reagent and optionally an epimerization reagent, then by a cleavage reagent of the $R'_4$ group when the latter represents a protector group of the hydroxyl radical different from an acyl radical, so as to obtain a product of formula $(I_D)$:

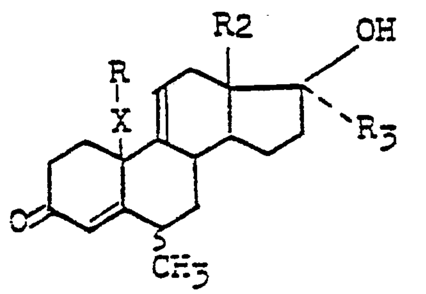

$(I_D)$

– <u>or</u> treated with an isomerization agent so as to obtain, after treatment with a cleavage reagent of the $R'_4$ group when the latter represents a protector group of the hydroxyl radical, a product of formula $(I_E)$

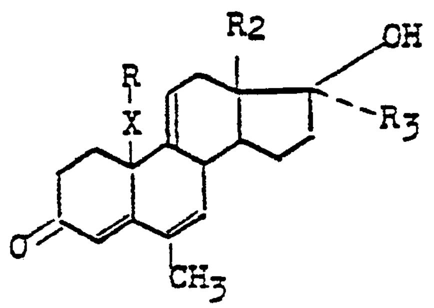

$(I_E)$

and in that, if desired, the preceding products of formulae $(I_A)$, $(I_B)$, $(I_C)$, $(I_D)$ and $(I_E)$ are treated with a dehydrogenation reagent or a micro-organism which can dehydrogenate, so as to obtain the corresponding products containing an unsaturation in position 1(2) and products of formulae $(I_A)$, $(I_B)$, $(I_C)$, $(I_D)$ and $(I_E)$ and the corresponding products containing an unsaturation in position 1(2) which if desired are treated with an acylation reagent.

3. Preparation process for products of general formula (I) as defined above, characterized in that a product of formula (VI):

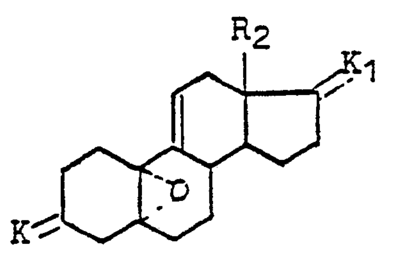

(VI)

in which $R_2$ has the significance indicated in claim 1, K represents a protector group of the ketone function and $K_1$ represents an optionally protected ketone function, is treated:

– either with a lithium derivative of formula R–X–Li,
– or with a magnesium derivative of formula R–X'–Mg–Hal, in which formulae R and X have the significance indicated in claim 1, X' represents a methylene radical or a simple bond and Hal represents a halogen atom, so as to obtain a product of formula (VII):

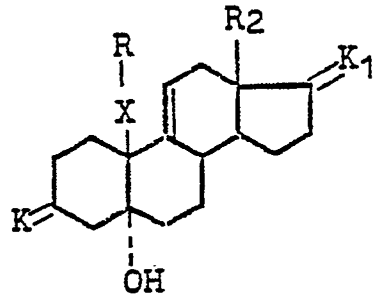
(VII)

which product is treated:
– either with a cleavage reagent of the $K_1$ radical when the latter represents a protector group of the keto radical in position 17, then with a magnesium derivative of formula $R_3$–Mg–$Hal_1$ or with a lithium derivative of formula $R_3$–Li, in which formulae $R_3$ has the significance indicated in claim 1, and $Hal_1$ represents a halogen atom, so as to obtain a product of formula ($III_A$):

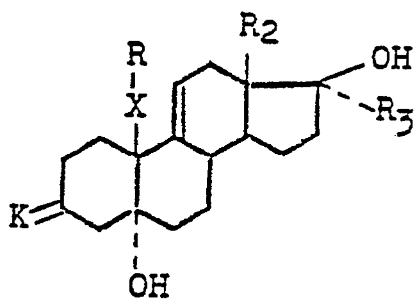
($III_A$)

which is treated with an acid so as to obtain a product of formula ($I_A$) as defined in claim 5, which product is optionally submitted to a protection reaction of the hydroxyl radical in position 17beta, and the product of formula ($I_A$) or (IV) thus obtained is treated according to the process described in claim 5, so as to obtain the products of formulae ($I_B$), ($I_C$), ($I_D$) and ($I_E$) and the corresponding unsaturated products in position 1(2),
– or the product of formula (VII) is treated with an acid so as to obtain a product of formula (VIII):

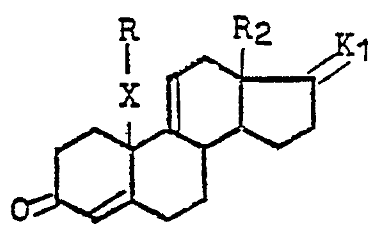
(VIII)

which product of formula (VIII) is treated
either with an alkyl orthoformate, then with a dehydrogenation reagent, so as to obtain a product of formula (IX):

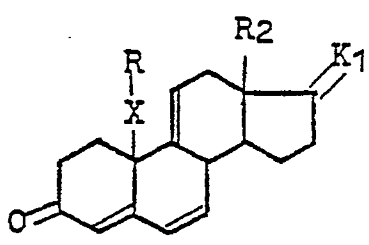
(IX)

or by a reagent capable of introducing a methylene radical, in position 6, so as to obtain a product of formula (X):

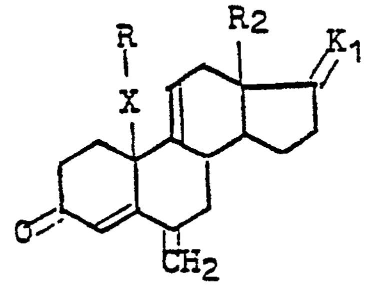

(X)

and which product of formula (X) is treated either by a hydrogenation reagent and optionally an epimerization agent, so as to obtain a product of formula (XI):

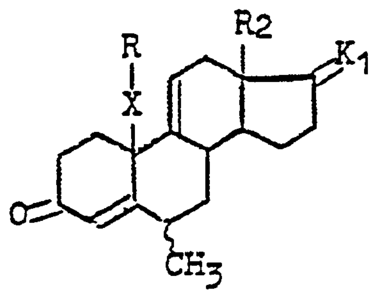

(XI)

or by an isomerization agent, so as to obtain a product of formula (XII):

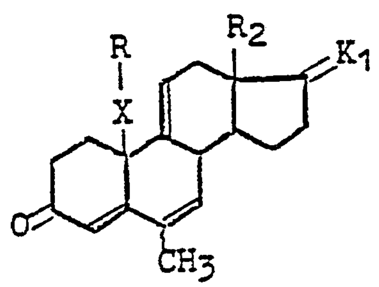

(XII)

and which products of formulae (VIII), (IX), (X), (XI) and (XII) are optionally treated with a cleavage reagent of the $K_1$ group when the latter represents a protector group of the ketone function, and with a blocking agent of the ketone function in position 3, then with a magnesium derivative of formula $R_3$–Mg–$Hal_1$ in which $R_3$ has the significance indicated in claim 1 and $Hal_1$ represents a halogen atom, so as to obtain respectively, after deblocking of the ketone function in position 3, the products of formulae ($I_A$), ($I_B$), ($I_C$), ($I_D$) and ($I_E$) as defined in claim 1, which can be converted, according to the process of claim 1, into corresponding unsaturated products in position 1(2) and which can be acylated.

4. Process according to claim 2 or 3, characterized in that at the start a lithium derivative of formula R–X–Li or a magnesium derivative of formula R–X'–Mg–Hal is used, in which formula the optionally substituted carbocyclic or heterocyclic aryl radical which can be represented by R signifies either a phenyl radical optionally substituted by an alkyl or alkoxy radical having from 1 to 4 carbon atoms, by a halogen atom or by an amino or dialkylamino radical, or a pyridyl radical.

5. Process according to claim 2 or 3, characterized in that at the start a lithium derivative of formula R–X–Li or a magnesium derivative of formula R–X'–Mg–Hal is used, in which the optionally substituted vinyl or ethynyl radical which can be represented by R signifies either a vinyl or methylvinyl radical of formula:

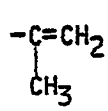

or an ethynyl radical optionally substituted by an alkyl radical having from 1 to 4 carbon atoms, by a phenyl radical, by an optionally esterified carboxy radical, by a hydroxymethyl radical or by an optionally protected or alkylated amino radical.